# EUROPEAN PATENT SPECIFICATION

(11) **EP 1 121 436 B1**
(45) Date of publication and mention of the grant of the patent: **27.06.2007**
(21) Application number: 99955852.1
(22) Date of filing: 11.10.1999
(51) Int. Cl.: C12N 15/12, C12N 5/10, C07K 14/705, C07K 16/28, A61K 31/70, A61K 38/17, A61K 39/395, A01K 67/027, G01N 33/50, A61P 25/00

(54) **NON-DESENSITIZING AMPA-RECEPTORS**
NICHT-DESENSIBILISIERENDE AMPA-REZEPTOREN
RECEPTEURS DU TYPE AMPA, NON DESENSIBILISANTS

(30) Priority: 13.10.1998 DE 19847064
(43) Date of publication of application: 08.08.2001
(73) Proprietor: Max-Planck-Gesellschaft zur Förderung der Wissenschaften e.V., Berlin (DE); YISSUM RESEARCH DEVELOPMENT COMPANY OF THE HEBREW UNIVERSITY OF JERUSALEM, 91042 Jerusalem (IL)
(72) Inventor: ROSENMUND, Christian, D-37073 Göttingen (DE); RUSSO, Sebastian, D-37073 Göttingen (DE); NEUMAN, Menahem, 71700 Modi'in (IL); STERN-BACH, Yael, 93714 Jerusalem (IL)
(74) Representative: Vossius & Partner
(86) International application number: PCT/EP1999/007604
(87) International publication number: WO 2000/022118

(56) References cited:
- EP-A- 0 574 257
- STERN-BACJ Y. ET AL.: "A point mutation in the glutamate binding site blocks desensitization of AMPA receptors" NEURON, vol. 21, October 1998 (1998-10), pages 907-918, XP000891606
- STERN-BACH Y. ET AL.: "Agonist selectivity of glutamate receptors is specified by two domains structurally related to bacteria amino acid-binding proteins" NEURON, vol. 13, 1994, pages 1345-1357, XP000891623 cited in the application
- PARTIN K. M. ET AL.: "AMPA receptor flip/flop mutants affecting deactivation, desensitization, and modulation by cyclothiazide, aniracetam, and thiocyanate" J. NEUROSCI., vol. 16, no. 21, 1 November 1996 (1996-11-01), pages 6634-6647, XP002134206 cited in the application
- UCHINO S. ET AL.: "Mutations in a putative agonist binding region of the AMPA-selective glutamate receptor channel" FEBS LETTERS, vol. 308, no. 3, 24 August 1992 (1992-08-24), pages 253-257, XP002134207

## Description

The present invention relates to a nucleic acid molecule encoding a (poly)peptide which has an amino acid sequence of a glutamate receptor of the AMPA-type and functions as a non-desensitizing AMPA-receptor or as a non-desensitizing subunit thereof, wherein the leucine corresponding to position 497 of the wildtype rat AMPA-receptor GluR1_{flip} or the leucine at the position which corresponds in other glutamate receptors of the AMPA-type by comparison of homology to position 497 of the wildtype rat AMPA-receptor GluR1_{flip} is replaced by an aromatic amino acid. The invention further relates to (poly)peptides encoded by said nucleic acid molecules, vectors and hosts comprising said nucleic acid molecules, as well as to methods for producing (poly)peptides encoded by said nucleic acid molecules. The present invention also provides for antibodies specifically directed to (poly)peptides encoded by said nucleic acid molecules. Additionally, the invention relates to a method for the blocking of desensitization of a glutamate receptor of the AMPA-type, comprising the step of replacing a leucine which corresponds by comparison of homology to position 497 of the rat AMPA-receptor GluR1 by an aromatic amino acid and methods for identifying and/or characterizing molecules which are capable of interaction with glutamate receptors of the AMPA type. The invention also relates to the one of the aforementioned nucleic acid molecules, (poly)peptides, hosts, vectors and/or antibodies as biosensors, for the characterization of glutamate receptor channel properties and/or for the preparation of pharmaceutical compositions. Furthermore, the invention provides for pharmaceutical compositions, and diagnostics comprising and/or employing the compounds of the invention.

Several documents are cited throughout the text of this specification. Each of the documents cited herein (including any manufacturers specifications, instructions, etc.) are hereby incorporated by reference; however, there is no admission that any document cited is indeed prior art for patentability of the present invention.

Fast glutamatergic neurotransmission is a major contributor to cell-to-cell communication in the central nervous system. Approximately 90% of all synapses in the brain are glutamatergic.

Glutamate receptors are found throughout the mammalian brain, where they constitute the major excitatory transmitter system. The longest-known and best-studied glutamate receptors are ligand-gated ion channels, also called ionotropic glutamate receptors, which are permeable to cations. They have traditionally been classified into three broad subtypes based upon pharmacological and electrophysiological data: α-amino-3-hydroxy-5-methyl-4-isoaxole propionate (AMPA) receptors, kainate (KA) receptors, and N-methyl-D-aspartate (NMDA) receptors. Furthermore, a family of G protein-coupled glutamate receptors, which are also called metabotropic glutamate *trans*-1-aminiocyclopentane-1,3-dicarboxylate (tACPD) receptors, was identified (Sugiyama, Nature 325 (1987), 531-533).

At excitatory synapses, presynaptically released glutamate diffuses across the synaptic cleft, and binds to postsynaptically localized ionotropic glutamate receptors from the α-amino-3-hydroxy-5-methyl-4-isoazole propionate (AMPA), NMDA, and kainate receptor subtypes. The binding of glutamate somehow leads to a conformational change in the channel, thereby opening the pore and allowing the influx of cations into the postsynaptic cell. Longer exposures to glutamate result in desensitization of the channel, i.e. it resides in a long-lasting ligand-bound, yet shut state. Desensitization of AMPA and kainate receptors occurs within a few milliseconds, a value found to be on the time scale of the postsynaptic response. This remarkable fast rate, together with a slow recovery from desensitization, is thought to be one of the mechanisms modifying the processing of synaptic information (especially at synapses with multiple release zones), and may serve as a negative feedback mechanism to prevent excitotoxic processes caused by excessive activation or brain damage that leads to prolonged glutamate exposure at the synaptic cleft. Recent molecular studies have provided increasing detailed models of the agonist binding site and of the channel pore (Reviewed by Green, Neuron 20 (1998), 427-444 and Pass, Trends Neurosci. 21 (1998), 117-125), however, little is known about structures underlying gating and desensitization.
There are four AMPA-selective subunits, GluR1-4 (or GluRA-RD), that can form functional distinct channels in homo- or hetero-oligomeric assemblies. The kainate receptors assemble from two pools: GluR5-7 and KA1-2 (reviewed by Seeburg, Trends Neurosci. 16 (1993), 359-365; Hollmann and Heinemann, Annu. Rev. Neurosci. 17 (1994), 31-108; Nakanishi and Masu, Annu. Rev. Biophys. Biomol. Struct. 23 (1994), 319-348). Recent topological studies divide the single subunit protein into several domains: (i) four hydrophobic domains, M1-M4, of which M1, M3 and M4 are thought to form transmembrane domains, while M2 forms a reentrant loop that lines the channel pore; (ii) a short cytoplasmic C-terminal domain, and (iii) two extracellular domains, composed of the N-terminus and the segment between M3-M4. Using a set of functional chimeric proteins made from the AMPA receptor GluR3 and the kainate receptor GluR6, it has been demonstrated that agonist binding specificity of these receptors is determined by two discontinuous segments, which were termed S1 and S2, respectively. S1 corresponds to a segment of ~150-amino acids preceding M1, and S2 to the segment between M3-M4 (Stern-Bach, Neuron 13 (1994), 1345-1357). Stimulated by the homology of S1-S2 to a set of bacterial periplasmic amino acid-binding proteins (PBPs), further specific residues within these segments, that bind agonists, and several structural models for the glutamate binding site have been constructed. According to these models, S1 and S2 form a bilobated structure that binds the agonist molecule in-between. S1 and the C-terminal half of S2 form the larger lobe 1, and the N-terminal half of S2 forms the smaller lobe 2. Recent crystallization of a S1-S2 protein construct confirmed the proposed model (Armstrong, Nature 395 (1998), 913-917. AMPA receptor subunits share an approximately 70 % overall sequence homology, with over 90% identical sequences within the binding region S1/S2.
Although kainate receptors do not coassemble with AMPA receptors, they share substantial sequence homology as well as structural and functional similarities to AMPA-receptors. The sequence homology between GluR3 and GluR6 for example is app. 40%, and large portions of intra- and extracellular domains can be exchanged between receptors without loosing receptor function (Kuusinen, EMBO 24 (1995), 6327-6332; Stern-Bach, 1994, loc. cit.). Both AMPA- and kainate receptors show rapid and almost complete desensitization upon exposure of glutamate, yet they vary in their kinetics of recovery from desensitization and their specificity for blockers of desensitization such as cyclothiazide and concavalin A (Partin, 1993, loc. cit.).

In AMPA receptors desensitization is modulated by altern*ative splicing and RNA editing of segments in S2. The alternative spliced versions (known as 'flip' and 'flop') differ in their time course of desensitization and in their sensitivity to the desensitization blocker cyclothiazide (Sommer, Science 249 (1990), 1580-1585; Mosbacher, Science 266 (1994), 1059-1062; Partin, Mol. Pharmacol. 46 (1994), 129-138) and some of the molecular determinants for these differences have been elucidated (Partin, Neuron 14 (1995), 883-843; Partin, J. Neurosci. 16 (1996), 6634-6647). The amino acid preceding the alternative spliced 'flip' and 'flop' modules is subject to RNA editing and the edited channels possess faster recovery rates from desensitization (R/G site; Lomeli, Science 266 (1994), 1709-1713). Recently, it has been also demonstrated that residues at the N-terminus of S2 modulate desensitization of GluR1 (Mano, J.B.C. 271 (1996), 15266-15302) and GluR6 receptors (Swanson, Neuron 19 (1997), 913-926). Currently, it is not exactly clear how these sites participate in the desensitization process, and if, beside S2, structures in other parts of the protein are also involved. In NMDA receptors, for example, it been recently shown that structures flanking S1 control glycine-independent desensitization (Krupp, Neuron 20 (1998), 317-327; Villarroel, Neuron 20 (1998), 329-339).

Although both AMPA and kainate receptors desensitize upon continuous application of glutamate, specific kinetic parameters vary considerably. These include the time course for recovery from desensitization, the differential sensitivity to allosteric modulators and the shape and extent of desensitization produced by other agonists (reviewed in Bettler and Mulle, Neuropharmacology 34 (1995), 123-139).

Said desensitization of AMPA-receptors is thought to shape the synaptic response and to act as a neuroprotective mechanism at central synapses. However, very little is known about relevant structures and mechanisms underlying the gating process and the basic mechanism responsible for desensitization is poorly understood.

Interestingly, the most plausible theories of learning, pattern recognition and memory depend upon changes in the efficiency of chemical synapses. The glutamate receptor, especially the NMDA receptor, has attracted much attention in this context since its properties make it an ideal candidate for a receptor involved directly in the learning process. Additionally, the above described AMPA receptors play critical roles in learning and/or some forms of associative memory in animals (see, e.g., Tsien, Cell 87 (1996), 1327-1338) and there are suggestions that slowing AMPA receptor desensitization may have a cognitive enhancing effect (Ingvar, Exp. Neurology 146 (1997), 553-559). Furthermore, a large body of evidence indicates that glutamate receptors play a role in a number of brain diseases and the damage that occurs after head injury. It also has been known for decades that glutamate is toxic to neurons in culture and in vivo, and many experiments implicate the glutamate receptor as a mediator of these toxic effects of glutamate, (for review see, inter alia, Choi, Neuron 1 (1988), 623-624; Choi & Rothman, Annu. Rev. Neurol. 13 (1990), 171-182; Storey, Ann. Neurol. 32 (1992), 526-534; and Appel, Trends Neurosci. 16 (1993), 3-5). It is furthermore well known that glutamate (as well as aspartate) can be neurotoxic, especially when energy supply is compromised (reviewed, inter alia, in Dingledine, Pharmaco. Rev. 51 (1999), 7-61). These observations have led investigators to suggest that many neurological accidents, including strokes in which there is a loss of oxygen and glucose or epileptic seizures, result in brain damage because of over-stimulation by glutamate. It has also been proposed that degenerative diseases such as Alzheimer's disease, Huntington's disease, Parkinson's disease, and amyotrophic lateral sclerosis (ALS) may involve neuronal cell death caused by excessive activation of the glutamate receptor system. Neurodegeneration associated with a variety of acute and chronic disorders (ischemic stroke, epilepsy, AIDS dementia, Rasmussen encephalitis among others) may, therefore, be caused in part by overactivation of glutamate receptors. Indeed, there is evidence from animal studies for marked neuroprotective effects of NMDA and AMPA receptor antagonists in models of ischemic stroke and epilepsy (Choi, Mt. Sinai J. Med. 65 (1998), 133-138).

The physiologically fast and complete desensitization of the above described wildtype AMPA receptors (time constant of 1 to 13 ms and inhibition of the current of 93 to 99% (Colquhoum, J. Physiol. 458 (1997), 261-28; Trussell, PNAS 85 (1988), 4562-4566; Mosbacher, Science 266 (1994), 1059-1062) is in vivo certainly neuroprotective for post-synaptic cells. However, the same physiological feature of fast desensitization makes experimental measurements of channel activities rather difficult and precludes the wildtype AMPA-receptors from a variety of pharmacological tests which may lead to a better understanding of the physiology of AMPA receptors and/or the detection/characterization of pharmacologically active substances capable of modifying said physiology.

Therefore, the technical problem underlying the present invention was to provide means and methods for the reproducible and reliable characterization of AMPA-receptor interactions with ligands and/or for the further elucidation of biochemical, biophysiological and/or electrophysiological properties of said receptors.

Accordingly, the present invention relates to a nucleic acid molecule encoding a (poly)peptide which has an amino acid sequence of a glutamate receptor of the AMPA-type and/or of a subunit of said receptor and functions as a non-desensitizing AMPA-receptor or as a non-desensitizing subunit thereof, wherein the leucine corresponding to position 497 of the wildtype rat AMPA-receptor GluR1_{flip} or the leucine at the position which corresponds in other glutamate receptors of the AMPA-type by comparison of homology to position 497 of the wildtype rat AMPA-receptor GluR1_{flip} is replaced by an aromatic amino acid.

The term "nucleic acid molecule" in accordance with the present invention comprises coding and, wherever applicable, non-coding sequences (like promotors, enhancers etc.) In accordance with the present invention, the term " nucleic acid molecule" comprises also any feasible derivative of a nucleic acid to which a nucleic acid probe may hybridize. Said nucleic acid probe itself may be a derivative of a nucleic acid molecule capable of hybridizing to said nucleic acid molecule or said derivative thereof. The term " nucleic acid molecule" further comprises peptide nucleic acids (PNAs) containing DNA analogs with amide backbone linkages (Nielsen, Science 254 (1991), 1497-1500). The term "nucleic acid molecule" which encodes a (poly)peptide, in connection with the present invention, is defined either by (a) the specific nucleic acid sequences encoding the (poly)peptide specified in the present invention or (b) by nucleic acid sequences hybridizing under stringent conditions to the complementary strand of the nucleotide sequences of (a) and encoding a (poly)peptide deviating from the nucleic acid of (a) by one or more nucleotide substitutions, deletions, additions or inversions and wherein the nucleotide sequence shows at least 40%, preferably at least 50%, more preferably at least 60% identity with the nucleotide sequence of said encoded (poly)peptide having an amino acid sequence as defined herein above and functions as a non-desensitizing AMPA-receptor or as a non-desensitizing subunit thereof.

The term "(poly)peptide" means, in accordance with the present invention, a peptide, a protein, or a (poly)peptide which encompasses amino acid chains of a given length, wherein the amino acid residues are linked by covalent peptide bonds. However, peptidomimetics of such proteins/(poly)peptides wherein amino acid(s) and/or peptide bond(s) have been replaced by functional analogs are also encompassed by the invention.

The term "non-desensitizing AMPA receptor", in accordance with this invention, denotes a glutamate receptor of the AMPA-type which does not desensitize in response to glutamate and/or its analogue(s) while other receptor channel properties remain intact. Since wildtype AMPA-glutamate receptors desensitize rapidly and almost completely in response to glutamate and/or its analogue(s), the term "non-desensitizing AMPA-receptor" also comprises, in accordance with this invention, AMPA receptors which desensitize slower and with less efficacy when compared to the corresponding wildtype glutamate receptor of the AMPA-type. In accordance with this invention, the term "AMPA-receptor" or "glutamate receptor of the AMPA-type" denotes any of the four AMPA-selective subunits, GluR1 to GluR4. Furthermore, said term denotes assembled structures forming distinct channels in heterooligomeric form and also comprises homooligomeric assemblies. Since AMPA receptor subunits only assemble with other AMPA receptor subunits but not with subunits from kainate - or NMDA receptors (Wenthold, J. Biol. Chem. 267 (1992), 501-507; Brose, J. Biol. Chem. 269 (1994), 16780-16784), the term "non-desensitizing AMPA receptor" also comprises the combination of at least one non-desensitizing AMPA receptor subunit with desensitizing (preferably wildtype) AMPA receptor subunits.

The term "leucine corresponding to position 497 of the wildtype rat AMPA-receptor GluR1_{flip} or the leucine at the position which corresponds in other glutamate receptors of the AMPA-type by comparison of homology to position 497 of the wildtype rat AMPA-receptor GluR1_{flip}" means, according to this invention, a specific leucine residue in a known wildtype sequence lying in a stretch of amino acid residues that form part of a glutamate binding site, wherein said glutamate binding side includes in the rat AMPA-receptor GluR1_{flip} , the leucine that lies between T494 and R499 (as shown in the appended examples). The wildtype rat AMPA receptor sequence of GluR1_{flip} is well known in the art and, inter alia, shown in SEQ ID NO. 1 (see also GenEMBL accession number X17184 and Hollmann, Nature 342 (1989), 643-648. Said leucine position 497 of the rat GluR1_{flip} subunit, corresponds, inter alia, to the position 497 of the human GluR1 or the mouse GluR1 subunit. Said position 497 corresponds, however, in the rat GluR2, human GluR2 or mouse GluR2 to position 504 of the known AMPA-selective subunits. Furthermore, said position 497 of the rat AMPA-receptor GluR1_{flip} corresponds to position 507 of the rat GluR3 subunit, to the position 505 of the rat GluR4 or the human GluR4 subunit, or to the position 513 of the human GluR3 subunit.

It was surprisingly found that the substitution/replacement of the leucine corresponding to position 497 of the wildtype rat AMPA-receptor GluR1_{flip} (or the leucine at the position which corresponds in other glutamate receptors of the AMPA-type by comparison of homology to position 497 of the wildtype rat AMPA-receptor GluR1_{flip}) with an aromatic amino acid results in a non-desensitizing receptor. However, corresponding reverse mutations in other glutamate receptors, for example Y521 L in kainate receptor subunit GluR6, does not lead to a non-desensitizing receptor. Considering the known affinities of different AMPA receptor subunits for agonists, the person skilled in the art can easily employ the teachings of the present invention and deduce which subunit of an AMPA receptor should be modified in order to obtain a nondesensitizing AMPA receptor according to this invention. The different affinitites of AMPA receptors are well known in the art (see, inter alia, Mosbacher, Science 266 (1994), 1059-1062). Therefore, if an AMPA-receptor of higher affinity isdesired, the person skilled in the art might choose GluR1. In contrast, for AMPA-receptors of lower affinity, GluR3 or GluR4 may be employed.

The wildtype amino acid sequences of different glutamate receptors and/or their subunits are well-known in the art (see, inter alia, Hollmann, Ann. Rev. Neurosci. 17 (1994), 31-108) and easily obtainable from electronic databases (for examble, GenBANK or GenEMBL). For further wildtype sequences of AMPA-receptors and/or their subunits, for example from other species, that will be isolated in the future, due to the high homology of AMPA-receptors, the position correponding to L 497 of the rat GluR1 is easily deducible employing the sequence information which is already available. Methods to be employed in order to elucidate further wildtype sequences of glutamate receptors of the AMPA-type and/or their subunits and methods to identify the leucine which corresponds to the leucine on position 497 of the rat GluR1_{flip} subunit comprise, inter alia, standard homology screenings and PCR-mediated screening techniques for related sequences. For the identification of further wildtype sequences of glutamate receptors of the AMPA-type, as well as for the detection of the relevant amino acid residues corresponding to the leucine on position 497 of the rat GluR1_{flip}, computer programs may be utilized.

For example, BLAST2.0, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments. BLAST produces alignments of both nucleotide and amino acid sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cutoff score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.
Analogous computer techniques using BLAST (Altschul, 1997, 1993 and 1990, supra) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as: $\frac{% sequence identity \times % maximum BLAST score}{100}$ and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules.

The term "by comparison of homology" denotes, in accordance with this invention, that amino acid sequences of other glutamate receptors of the AMPA-types, or subunits thereof, are compared with the amino acid sequence of the amino acid sequences of the AMPA-receptor GluR1_{flip} (as depicted, inter alia, in SEQ ID NO: 1). "Homology" is understood to refer in this context to a sequence identity of glutamate receptors of the AMPA-type of at least 60%, particularly of a amino acid sequence identity of 70%, preferably more than 80% and still more preferably more than 90% on the amino acid level. The present invention, however, comprises also (poly)peptides deviating from wildtype amino acid sequences of glutamate receptors of the AMPA-type described herein above, wherein said deviation may be, for example, the result of amino acid and/or nucleotide substitution(s), deletion(s), addition(s), insertion(s), duplication(s), inversion(s) and/or recombination(s) either alone or in combination. Those deviations may naturally occur or be produced via recombinant DNA techniques well known in the art; see, for example, the techniques described in Sambrook (Molecular Cloning; A Laboratory Manual, 2nd Edition, Cold Spring Harbour Laboratory Press, Cold Spring Harbour, NY (1989)) and Ausubel, "Current Protocols in Molecular Biology", Green Publishing Associates; and Wiley Interscience, N.Y. (1989). The allelic variations may be naturally occurring allelic variants as well as synthetically produced or genetically engineered variants. The (poly)peptides, peptides or protein fragments encoded by the various derivatives, allelic variants, homologues or analogues of the above-described nucleic acid molecules encoding non-desensitizing AMPA-recptors and/or subunits thereof may share specific common characteristics, such as molecular weight, immunological reactivity, conformation etc., as well as physical properties, such as electrophoretic mobility, chromatographic behavior, sedimentation coefficients, pH optimum, stability, solubility, spectroscopic properties etc.

In a preferred embodiment, the nucleic acid molecule of the invention is (a) a nucleic acid molecule comprising a nucleic acid molecule encoding the (poly)peptide having the amino acid sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, or SEQ ID NO: 10, wherein the leucine residue corresponding to position 497 of SEQ ID NO: 1, 5 or 9, corresponding to position 504 of SEQ ID NO: 2, 6 or 10, corresponding to position 507 of SEQ ID NO: 3, to position 505 of SEQ ID NO: 4 or 8, or corresponding to position 513 of SEQ ID NO: 7 is replaced by an aromatic amino acid; or (b) a nucleic acid molecule comprising a nucleic acid molecule encoding the (poly)peptide having the DNA sequence of SEQ ID NO: 11, SEQ ID NO. 12, SEQ ID NO: 13, SEQ ID NO: 14, SEQ ID NO: 15, SEQ ID NO: 16, SEQ ID NO: 17, SEQ ID NO: 18, SEQ ID NO: 18, SEQ ID NO: 20, wherein the codon represented by nnn corresponds to a codon coding for an aromatic amino acid; (c) a nucleic acid molecule hybridizing to the complementary strand of a nucleic acid molecule of (a) or (b); or the nucleic acid molecule of the invention is (d) a nucleic acid molecule being degenerate as a result of the genetic code to the nucleotide sequence of a nucleic acid molecule as defined in (c).

The term "codon represented by nnn corresponds to a codon coding for an aromatic amino acid" means, in accordance with the present invention, a codon which, according to the standard genetic code (as illustrated, inter alia, in Stryer (1995), "Biochemistry", Freemann and Compagny, ISBN 0-7167-2009-4) codes for any aromatic amino acid. For example, the codons TAT and TAC code for tyrosine, the codons TTT and TTC code for phenylalanine, the codon TGG codes for tryptophane, the codons CAT and CAC code for histidine.

The term "hybridizes" as used in accordance with the present invention may relate to hybridizations under stringent or non-stringent conditions. If not further specified, the conditions are preferably non-stringent. Said hybridization conditions may be established according to conventional protocols described, for example, in Sambrook, "Molecular Cloning, A Laboratory Manual", Cold Spring Harbor Laboratory, N.Y. (1989), Ausubel, "Current Protocols in Molecular Biology", Green Publishing Associates and Wiley Interscience, N.Y. (1989), or Higgins and Hames (Eds) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as 0.1xSSC, 0.1% SDS at 65°. Non-stringent hybridization conditions for the detection of homologous or not exactly complementary sequences may be set at 6xSSC, 1% SDS at 65°C. As is well known, the length of the probe and the composition of the nucleic acid to be determined constitute further parameters of the hybridization conditions. Hybridizing nucleic acid molecules or molecules falling under alternative (c), supra, also comprise fragments of the molecules identified in (a), or (b) wherein the nucleotide sequence need not be identical to its counterpart in SEQ ID NOs: 11 to 20 said fragments representing nucleic acid sequences which code for non-desensitizing glutamate receptors of the AMPA-type or a functional fragment thereof, such as a (modified) glutamate binding side, and having a length of at least 12 nucleotides, preferably at least 15, more preferably at least 18, more preferably of at least 21 nucleotides, more preferably at least 30 nucleotides, even more preferably at least 40 nucleotides and most preferably at least 60 nucleotides. Furthermore, nucleic acid molecules which hybridize with any of the aforementioned nucleic acid molecules also include complemetary fragments, derivatives and allelic variants of these molecules. Functional fragments of non-desensitizing glutamate receptors of the AMPA-type and/or subunits may be comprised in a fusion and/or chimeric protein.

The term "derivative" means in this context that the nucleotide sequence of these nucleic acid molecules differs from the sequences of the above-described nucleic acid molecules in one or more nucleotide positions, that the nucleotide sequences are homologous (at least 40%, more preferably at least 50%. even more preferably 60%, most preferably at least 70%) at least to said nucleic acid molecules and that they comprise a codon replacing a codon coding for a corresponding amino acid residue to position 497 of the wildtype rat AMPA-receptor GluR1_{flip} or the leucine at the position which corresponds in other glutamate receptors of the AMPA-type by comparison of homology to position 497 of the wildtype rat AMPA-receptor GluR1_{flip}, wherein said replacing codons code for any aromatic amino acid.
Homology is understood to refer in the context of "fragments", "derivatives" or "allelic variants" to a sequence identity of at least 60%, particularly an identity of at least 70%, preferably more than 80% and still more preferably more than 90%.

The nucleic acid molecule of the invention is a nucleic acid molecule encoding a (poly)peptide which comprises an aromatic amino acid at position 497 of SEQ ID NO: 1, 5 or 9, at position 504 of SEQ ID NO: 2, 6 or 10, at position 507 of SEQ ID NO: 3, at position 505 of SEQ ID NO: 4 or 8 or at position 513 of SEQ ID NO: 7, but differs therefrom by at least one mutation selected from the group consisting of amino acid substitutions, insertions, deletions, inversions and/or duplications.

Whereas nucleic acid molecules derived from a variety of species encoding homologous (poly)peptides representing glutamate receptors of the present invention are included in the present invention (for example, glutamate receptor genes have been reported in various species, like in insects, yeasts, fungi or plants (see, inter alia, Lam, Nature 396(1998), 125-126; Chiu, Molecular Biology and Evolution 16 (1999) 826-838)), in an even more preferred embodiment the nucleic acid molecule of the invention is derived from a rat, a mouse or a human.

Particularly preferred are nucleic acid molecule of the invention wherein said aromatic amino acid residue is tyrosine, phenylalanine, tryptophan or histidine.

In a preferred embodiment the nucleic acid molecule of the invention is DNA, RNA or PNA. The DNA may be cDNA, the RNA may be mRNA. Its origin may be natural, synthetic or semisynthetic or it may be a derivative, such as said peptide nucleic acid (Nielsen, Science 254 (1991), 1497-1500). Furthermore, said nucleic acid molecule may be a recombinantly produced chimeric nucleic acid molecule comprising any of the aforementioned nucleic acid molecules either alone or in combination.

In a particularly preferred embodiment, the nucleic acid molecule of the invention encodes a fusion protein. The term "fusion protein" denotes any polypeptide consisting or comprising of at least two (poly)peptides not naturally forming such a polypeptide. On the DNA level, the two or more coding sequences are fused in frame.
Such fusion proteins are, inter alia, exemplified in the append examples and comprise fusion proteins like specific chimeric polypeptides combining the glutamate binding domain of AMPA receptors with different parts of other glutamate receptors, like, kainate receptors (for example, GluR1-GluR6 or GluR3-GluR6 chimeras), NMDA receptors and/or tACPD receptors. Chimeric exchanges between AMPA and kainate receptors represent conservative exchanges, as both receptors share high sequence homology. However, this invention also comprises fusion proteins wherein a part of or a complete AMPA receptor is linked to another protein or a part of another protein which does not function as a glutamate receptor. Examples of said other proteins comprise proteins representing further receptors, channels (voltage or transmitter gated) and/or pumps like, e.g. serotonin receptors, acetylcholine receptors, GABA receptors, glycine receptors, G-protein-coupled receptors and/or parts of these receptors. Additionally, said fusion protein may comprise proteins and/or parts which do not naturally function as receptors, channels and/or pumps. Therefore, the nucleic acid molecule of the present invention may also have the coding sequence fused in frame to, e.g. a sequence encoding a marker which allows, inter alia, the purification, isolation, and/or detection of the (poly)peptide of the present invention. Such a marker may be a label or a tag, like, e.g. GST, cellulose binding domain, green fluorescent protein, maltose binding protein, alkaline phosphatase, lacZ, c-myc, His-tag, FLAG, EpiTag^{™}, V5 tag, T7 tag, Xpress^{™} tag or Strep-tag. In accordance with the invention, two or more tags may be comprised by the fusion protein. Any additional domain present in the fusion protein of the present invention comprising a (poly)peptide as defined herein above according to this invention may be joined directly (i.e. no intervening amino acids) or may be linked via a (flexible) linker, advantageously a polypeptide linker. The above defined fusion protein may further comprise a cleavable linker or a cleavage site, which, for example, is specifically recognized and cleaved by proteinases or chemical agents. Cleavable linker sequences include, but are not limited to, Factor XA or enterokinase (Invitrogen, San Diego, USA).

Preferably, the nucleic acid molecule of the present invention is part of a vector. Therefore, the present invention relates in another embodiment to a vector comprising the nucleic acid molecule of this invention. Such a vector may be, e.g., a plasmid, cosmid, virus, bacteriophage or another vector used e.g. conventionally in genetic engineering, and may comprise further genes such as marker genes which allow for the selection of said vector in a suitable host cell and under suitable conditions.
Furthermore, the vectors may, in addition to the nucleic acid sequences of the invention, comprise expression control elements, allowing proper expression of the coding regions in suitable hosts. Such control elements are known to the artisan and may include a promoter, translation initiation codon, translation and insertion site for introducing an insert into the vector. Preferably, the nucleic acid molecule of the invention is operatively linked to said expression control sequences allowing expression in eukaryotic or prokaryotic cells. Particularly preferred are in this context control sequences which allow for correct expression in neuronal cells and/or cells derived from nervous tissue.

Control elements ensuring expression in eukaryotic and prokaryotic cells are well known to those skilled in the art. As mentioned above, they usually comprise regulatory sequences ensuring initiation of transcription and optionally poly-A signals ensuring termination of transcription and stabilization of the transcript. Additional regulatory elements may include transcriptional as well as translational enhancers, and/or naturally-associated or heterologous promoter regions. Possible regulatory elements permitting expression in for example mammalian host cells comprise the CMV- HSV thymiakine kinase promoter, SV40, RSV-promoter (Rous sarcome virus), human elongation factor 1α-promoter, CMV enhancer, CaM-kinase promoter or SV40-enhancer. For the expression for example in nervous tissue and/or cells derived therefrom, several regulatory sequences are well known in the art, like the minimal promoter sequence of human neurofilament L (Charron, J. Biol. Chem 270 (1995), 25739-25745). For the expression in prokaryotic cells, a multitude of promoters including, for example, the tac-lac-promoter or the trp promoter, has been described. Beside elements which are responsible for the initiation of transcription such regulatory elements may also comprise transcription termination signals, such as SV40-poly-A site or the tk-poly-A site, downstream of the polynucleotide. In this context, suitable expression vectors are known in the art such as Okayama-Berg cDNA expression vector pcDV1 (Pharmacia), pRc/CMV, pcDNA1, pcDNA3 (In-Vitrogene, as used, inter alia in the appended examples), pSPORT1 (GIBCO BRL) or pGEMHE (Promega), or prokaryotic expression vectors, such as lambda gt11. Beside the nucleic acid molecules of the present invention, the vector may further comprise nucleic acid sequences encoding for secretion signals. Such sequences are well known to the person skilled in the art. Furthermore, depending on the expression system used leader sequences capable of directing the protein/(poly)peptide to a cellular compartment may be added to the coding sequence of the nucleic acid molecules of the invention and are well known in the art. The leader sequence(s) is (are) assembled in appropriate phase with translation, initiation and termination sequences, and preferably, a leader sequence capable of directing secretion of translated protein, or a part thereof, into, inter alia, the extracellular membrane. Optionally, the heterologous sequence can encode a fusion protein including an C- or N-terminal identification peptide imparting desired characteristics, e.g., stabilization or simplified purification of expressed recombinant product. Once the vector has been incorporated into the appropriate host, the host is maintained under conditions suitable for high level expression of the nucleotide sequences, and, as desired, the collection and purification of the proteins, antigenic fragments or fusion proteins of the invention may follow. Of course, the vector can also comprise regulatory regions from pathogenic organisms.

Furthermore, said vector may also be, besides an expression vector, a gene transfer and/or gene targeting vector. Gene therapy, which is based on introducing therapeutic genes (for example for vaccination) into cells by ex-vivo or in-vivo techniques is one of the most important applications of gene transfer. Suitable vectors, vector systems and methods for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813, Isner, Lancet 348 (1996), 370-374; Muhlhauser, Circ. Res. 77 (1995), 1077-1086; Wang, Nature Medicine 2 (1996), 714-716; WO 94/29469; WO 97/00957, Schaper, Current Opinion in Biotechnology 7 (1996), 635-640 or Verma, Nature 389 (1997), 239-242 and references cited therein.
The nucleic acid molecules of the invention and vectors as described herein above may be designed for direct introduction or for introduction via liposomes, or viral vectors (e.g. adenoviral, retroviral) into the cell. Additionally, baculoviral systems or systems based on vaccinia virus or Semliki Forest Virus can be used as eukaryotic expression system for the nucleic acid molecules of the invention. In addition to recombinant production, fragments of the protein, the fusion protein or antigenic fragments of the invention may be produced by direct peptide synthesis using solid-phase techniques (cf Stewart et al. (1969) Solid Phase Peptide Synthesis, WH Freeman Co, San Francisco; Merrifield, J. Am. Chem. Soc. 85 (1963), 2149-2154). *In vitro* protein synthesis may be performed using manual techniques or by automation. Automated synthesis may be achieved, for example, using Applied Biosystems 431A Peptide Synthesizer (Perkin Elmer, Foster City CA) in accordance with the instructions provided by the manufacturer. Various fragments may be chemically synthesized separately and combined using chemical methods to produce the full length molecule.

The present invention in addition relates to a host transformed with a vector of the present invention or to a host comprising the nucleic acid molecule of this invention. Said host may be any prokaryotic or eukaryotic cell. Suitable prokaryotic/bacterial cells are those generally used for cloning like E. coli or Bacillus subtilis. Said eukaryotic host may be a mammalian cell, an amphibian cell, an insect cell, a fungal cell, or a plant cell. Suitable fungal cells are yeast cells, preferably those of the genus Saccharomyces and most preferably those of the species S. cerevisiae. In a particularly preferred embodiment said mammalian cell is a neuronal cell and/or a cultured cell like, inter alia, a HEK 293 (human embryonic kidney) cell, a CHO, HeLa, NIH3T3, BHK or a PC12 cell. Said amphibian cell may be an oocyte. Said oocyte may be, inter alia, a frog oocyte, for example Xenopus laevis oocyte.

In a more preferred embodiment , the present invention relates to an host of the invention which is a non-human transgenic organism. Said non-human organism may be a mammal, anamphibian, an insect, a fungus or a plant. Particularly preferred non-human transgenic animals are Drosophila, C. elegans, Xenopus, mice and rats. Transgenic plants comprise, but are not limited to, wheat, tobacco, parsley and Arabidopsis. Transgenic fungi are also well known in the art and comprise, inter alia, yeasts, like S. pombe or S. cerevisae, or Aspergillus species.

In another embodiment, the present invention relates to a method for producing the (poly)peptide encoded by a nucleic acid molecule of the invention comprising culturing/raising the host of the invention and isolating the produced (poly)peptide.
A large number of suitable methods exist in the art to produce proteins/(poly)peptides in appropriate hosts. If the host is a unicellular organism or a mammalian or insect cell, the person skilled in the art can revert to a variety of culture conditions that can be further optimized without an undue burden of work. Conveniently, the produced protein is harvested from the culture medium or from isolated (biological) membranes by established techniques. Furthermore, the produced protein/(poly)peptide may be directly isolated from the host cell. Said host cell may be part of or derived from a part of a host organism, for example said host cell may be part of the CNS of an animal or the harvestable part of a plant. Additionally, the produced (poly)peptide may be isolated from fluids derived from said host, like blood, milk or cerebrospinal fluid.

Additionally the present invention relates to a (poly)peptide that is encoded by the nucleic acid molecule of the invention or produced by the method of the invention. The (poly)peptide of the invention may accordingly be produced by microbiological methods or by transgenic mammals. It is also envisaged that the polypeptide of the invention is recovered from transgenic plants. Alternatively, the polypeptide of the invention may be produced synthetically or semi-synthetically.

In a further embodiment, the present invention relates to an antibody specifically directed to the (poly)peptide and/or fusion protein of the invention, wherein said antibody specifically reacts with an epitope comprising the aromatic amino acid which replaces the leucine at position 497 of the wildtype rat AMPA-receptor GluR1_{flip} or the leucine at the position which corresponds in other glutamate receptors of the AMPA-type by comparison of homology to position 497 of said wildtype rat AMPA receptor GluR1_{flip}. Whether said antibody specifically reacts as defined herein above can easily be tested, inter alia, by comparing the reaction of said antibody with a wildtype AMPA-receptor (or a subunit or a fragment thereof) with the reaction of said antibody with a (poly)peptide of this invention. The antibody of the present invention can be, for example, polyclonal or monoclonal antibodies. Techniques for the production of antibodies are well known in the are and described, e.g. in Harlow and Lane "Antibodies, A Laboratory Manual", CSH Press, Cold Spring Harbor, 1988. These antibodies can be used, for example, for the immunoprecipitation and immunolocalization of the (poly)peptides of the invention as well as for the monitoring of the presence of such (poly)peptides, for example, in recombinant organisms, and for the identification of compounds interacting with the proteins according to the invention (as mentioned herein below). For example, surface plasmon resonance as employed in the BIAcore system can be used to increase the efficiency of phage antibodies which bind to an epitope of the polypeptide of the invention (Schier, Human Antibodies Hybridomas 7 (1996), 97-105; Malmborg, J. Immunol. Methods 183 (1995), 7-13).
The present invention furthermore includes chimeric, single chain and humanized antibodies, as well as antibody fragments, like, inter alia, Fab fragments. Antibody fragments or derivatives further comprise F(ab')₂, Fv or scFv fragments; see, for example, Harlow and Lane, loc.cit.. Various procedures are known in the art and may be used for the production of such antibodies and/or fragments. Thus, the (antibody) derivatives can be produced by peptidomimetics. Further, techniques described for the production of single chain antibodies (see, inter alia, US Patent 4,946,778) can be adapted to produce single chain antibodies to polypeptide(s) of this invention. Also, transgenic animals may be used to express humanized antibodies to polypeptides of this invention. Most preferably, the antibody of this invention is a monoclonal antibody. The general methodology for producing, monoclonal antibodies is well-known and has been described in, for example, Köhler and Milstein, Nature 256 (1975), 494-496 and reviewed in J.G.R. Hurrel, ed., "Monoclonal Hybridoma Antibodies: Techniques and Applications", CRC Press Inc., Boco Raron, FL (1982); as well as that taught by L. T. Mimms et al., Virology 176 (1990), 604-619.

In yet another embodiment, the present invention relates to composition comprising the nucleic acid molecule, the (poly)peptide and/or the antibody of the invention.

The term "composition", as used in accordance with the present invention, comprises at least one nucleic acid molecule, (poly)peptide, an antigenic and preferably immunogenic fragment of said (poly)peptide comprising an epitope comprising the aromatic amino acid which replaces the leucine at position 497 of the wildtype rat AMPA-receptor GluR1_{flip} or the leucine at the position which corresponds in other glutamate receptors of the AMPA-type by comparison of homology to position 497 of said wildtype rat AMPA receptor GluR1_{flip}., a fusion protein, and/or an antibody of this invention and, optionally, further molecules, either alone or in combination, like e.g. molecules which are capable of suppressing glutamate release, capable of blocking, modulating and/or activating glutamate receptors or molecules which have neuroprotective and/or nootropic properties.

The composition may be in solid, liquid or gaseous form and may be, inter alia, in the form of (a) powder(s), (a) tablet(s), (a) solution(s) or (an) aerosol(s).

Furthermore, the present invention relates to a the composition of this invention which is a pharmaceutical composition, optionally further comprising an acceptable carrier and/or diluent and/or excipient. The pharmaceutical composition of the present invention may be particularly useful in preventing and/or treating pathological disorders in humans or animals. Said pathological disorders comprise, but are not limited to, neurological, neurodegenerative and/or neuro-psychiatric disorders. These disorders comprise, inter alia, Alzheimer's disease, Parkinson's disease, Huntington's disease, amyotrophic lateral sclerosis (FALSISALS), ischemia, stroke, epilepsy, AIDS dementia and learning disorders. The pharmaceutical composition may also be used for prophylactic purposes.
Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. However, it is also encisaged that the pharmaceutical compostions are directly applied to the nervous tissue. The dosage regimen will be determined by the attending physician and clinical factors. As is well known in the medical arts, dosages for any one patient depends upon many factors, including the patient's size, body surface area, general health, age, sex, the particular compound to be administered, time and route of administration, and other drugs being administered concurrently. Pharmaceutically active matter may be present, inter alia, in amounts between 1 ng and 100 mg per dose; however, doses below or above this exemplary range are envisioned, especially considering the aforementioned factors. If the regimen is a continuous infusion, it should also be in the range of 1 µg to 10 mg units per kilogram of body weight per minute, respectively. Progress can be monitored by periodic assessment. The compositions of the invention may be administered locally or systemically. Administration will generally be parenterally, e.g., intravenously. The compositions of the invention may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringers dextrose), and the like. Preservatives and other additives may also be present such as, for example, antimicrobials, anti-oxidants, chelating agents, and inert gases and the like. Furthermore, the pharmaceutical composition of the invention may comprise further agents, depending on the intended use of the pharmaceutical composition. Such agents may be drugs acting on the central nervous system as well as on small, unmyelinated sensory nerve terminals (like in the skin), neurons of the peripheral nervous system of the digestive tract. Furthermore said pharmaceutical composition may additionally comprise drugs and compounds which may influence glutamate-uptake or enhanced glutamate release, leading to excessive activation of the glutamate receptor system, like, inter alia, AMPAkines (Ingvar (1997), loc. cit.). Further drugs acting on the central nervous system comprise, but are not limited to, antidepressants (like monoamine oxidase inhibitors, such as phenelzine) anti-seizure drugs (like, e.g., carbomazepine, phenobarbital or valproate), anti-stroke drugs (like, e.g. watersoluble AMPA receptor antagonists as described, inter alia, in Small, Neuroreport 9 (1998), 1287-1290 or in Turski, Proc. Natl. Acad. Sci. USA 95 (1998), 10960-10965) or drugs employed in the alleviation of learning disorders or for cognitive enhancement, like, inter alia, AMPAkines (Ingvar (1997), loc. cit.).

Additionally, in accordance with this invention, the composition of this invention may be a diagnostic composition, optionally further comprising suitable means for detection. The diagnostic composition comprises at least one of the aforementioned compounds of the invention. The diagnostic composition may be used, inter alia, for methods for determining the expression of the nucleic acids and/or polypeptides of the invention by detecting, inter alia, the presence of the corresponding mRNA which comprises isolation of RNA form a cell, contacting the RNA so obtained with a nucleic acid probe as described above under hybridizing conditions, and detecting the presence of mRNAs hybridized to the probe. Furthermore, (poly)peptides of the invention can be detected with methods known in the art , which comprise, inter alia, immunological methods, like, ELISA or Western blotting.

Furthermore, the diagnostic composition of the invention may be useful, inter alia, in detecting the prevalence, the onset or the progress of a disease related to the aberant expression of a polypeptide of the invention. Accordingly, the diagnostic composition of the invention may be used, inter alia, for assessing the prevalence, the onset and/or the disease status of neurological, neurodegenerative and/or neuro-psychiatric disorders, as defined herein above. It is also contemplated that antibodies and compositions comprising such antibodies of the invention may be useful in discriminating (the) stage(s) of a disease.

The diagnostic composition optionally comprises suitable means for detection. The nucleic acid molecule(s), vector(s), host(s), antibody(ies), (poly)peptide(s), fusion protein(s) described above are, for example, suitable for use in immunoassays in which they can be utilized in liquid phase or bound to a solid phase carrier. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention.
Solid phase carriers are known to those in the art and may comprise polystyrene beads, latex beads, magnetic beads, colloid metal particles, glass and/or silicon chips and surfaces, nitrocellulose strips, membranes, sheets, duracytes and the walls of wells of a reaction tray, plastic tubes or other test tubes. Suitable methods of immobilizing nucleic acid molecule(s), vector(s), host(s), antibody(ies), (poly)peptide(s), fusion protein(s) etc. on solid phases include but are not limited to ionic, hydrophobic, covalent interactions and the like. Examples of immunoassays which can utilize said compounds of the invention are competitive and non-competitive immunoassays in either a direct or indirect format. Commonly used detection assays can comprise radioisotopic or non-radioisotopic methods. Examples of such immunoassays are the radioimmunoassay (RIA), the sandwich (immunometric assay) and the Northern or Southern blot assay. Furthermore, these detection methods comprise, inter alia, IRMA (Immune Radioimmunometric Assay), EIA (Enzyme Immuno Assay), ELISA (Enzyme Linked Immuno Assay), FIA (Fluorescent Immuno Assay), and CLIA (Chemioluminescent Immune Assay). Furthermore, the diagnostic compounds of the present invention may be are employed in techniques like FRET (Fluorescence Resonance Energy Transfer) assays.

Appropriate labels and methods for labeling are known to those of ordinary skill in the art. Examples of the types of labels which can be used in the present invention include inter alia, fluorochromes (like fluorescein, rhodamine, Texas Red, etc.), enzymes (like horse radish peroxidase, β-galactosidase, alkaline phosphatase), radioactive isotopes (like ³²P or ¹²⁵I), biotin, digoxygenin, colloidal metals, chemi- or bioluminescent compounds (like dioxetanes, luminol or acridiniums).

A variety of techniques are available for labeling biomolecules, are well known to the person skilled in the art and are considered to be within the scope of the present invention and comprise, inter alia, covalent coupling of enzymes or biotinyl groups, phosphorylations, biotinylations, random priming, nick-translations, tailing (using terminal transferases). Such techniques are, e.g., described in Tijssen, "Practice and theory of enzyme immuno assays", Burden and von Knippenburg (Eds), Volume 15 (1985); "Basic methods in molecular biology", Davis LG, Dibmer MD, Battey Elsevier (1990); Mayer, (Eds) "Immunochemical methods in cell and molecular biology" Academic Press, London (1987); or in the series "Methods in Enzymology", Academic Press, Inc.

Detection methods comprise, but are not limited to, autoradiography, fluorescence microscopy, direct and indirect enzymatic reactions, etc.
Said diagnostic composition may be used for methods for detecting the abundance of a nucleic acid molecule of the invention in a biological and/or medical sample and/or for detecting expression of a nucleic acid molecule (i.e. an expressed (poly)peptide) of the invention. Furthermore, said diagnostic composition may also be used in methods of the present invention, inter alia, for the detection of specific antagonists or agonists for glutamate receptors (see herein below).

In yet another embodiment, the present invention relates to a method for the blocking of desensitization of a glutamate receptor of the AMPA-type, comprising the step of replacing a leucine corresponding to position 497 of the wildtype rat AMPA-receptor GluR1 or corresponding by comparison of homology to position 497 of the rat AMPA-receptor GluR1 by an aromatic amino acid. Said leucine might be replaced, inter alia, by recombinant methods known in the art and exemplified in the appended examples.

Furthermore, the present invention relates to a method for identifying molecules which are capable of interacting with glutamate receptors of the AMPA-type, comprising the steps of (a) contacting a non-desensitizing AMPA-receptor as encoded by a nucleic acid molecule, a vector, a host, or an antibody of this invention with said molecule; and (b) identifying among these molecules the molecules which are capable of interacting with said glutamate receptor of the AMPA-type. In case of the nuclaic acid molecule and7or the vector of this invention, said nucleic acid molecuel and/or vector may be first activated and/or expressed

Additionally, the present invention relates to a method for the characterization of molecules which are capable of interaction with glutamate receptors of the AMPA-type, comprising the steps of (a) contacting a non-desensitizing AMPA-receptor as defined herein above or a vector, a host, or an antibody of this invention with said molecules; and (b) measuring and/or detecting the characteristic effect said molecules evoke.
Said identification and/or characterization of molecules which are capable of interacting with glutamate receptors of the AMPA-type, may be, inter alia, achieved by transfecting an appropriate host with a nucleic acid molecule of invention. Said hosts comprise, but are not limited to, HEK 293 cells or frog oocytes. After expression of a non-desensitizing AMPA-receptor, membrane currents may be deduced in the absence and/or presence of the molecule to be identified and/or characterized. Methods for the deduction of membrane currents are well known in the art and comprise, e.g., patch clamp methods as described in Hamill, Pfluger's Arch. 391 (1981), 85-100 or two-electrode voltage clamp in oocytes, as described in Methfessel, Pflügers Archive 407 (1986) 577-588.

Furthermore, the present invention relates to a method of screening for molecules which are capable of interacting with glutamate receptors of the AMPA-type, comprising the steps of (a) contacting a non-desensitizing AMPA-receptor as encoded by a nucleic acid molecule, a vector, a host of the invention with a candidate molecule; and (b) measuring and/or detecting a response; and (c) comparing said response to a standard response as measured in the absence of said candidate molecule.

Potential candidate molecules or candidate mixtures of molecules may be, inter alia, substances, compounds or compositions which are of chemical or biological origin, which are naturally occurring and/or which are synthetically, recombinantly and/or chemically produced. Thus, candidate molecules may be proteins, protein-fragments, peptides, amino acids and/or derivatives thereof or other compounds, such as ions, which bind to and/or interact with wild-type AMPA-receptors. Such binding and/or interacting candidate compounds may be found employing, inter alia, yeast two-hybrid systems or modified yeast two-hybrid systems as described, for example, in Fields, Nature 340 (1989), 245-246; Gyuris, Cell 75 (1993), 791-801; or Zervos, Cell 72 (1993), 223-232.

Furthermore, potential candidate molecules may be contacted with a cell, such as an oocyte or a HEK 293 cell, which expresses a (poly)peptide of the invention or with a membrane patch comprising a (poly)peptide of the invention and a corresponding response (inter alia, a dose-response response, a current-response, or single current channel response) may be measured in order to elucidate any effect said candidate molecule causes.

Within the scope of the present invention are also methods for identifying, characterizing and for screening of molecules which are capable of interacting with glutamate receptors of the AMPA-type which comprise so-called high-throughput screening methods and similar approaches which are known in the art (Spencer, Biotechnol. Bioeng. 61 (1998), 61-67; Oldenburg, Annu. Rep. Med. Chem. 33 (1998), 301-311) carried out using 96-well, 384-well, 1536-well (and other) commercially available plates. Further methods to be employed in accordance with the present invention comprise, but are not limited to, homogenous fluorescence readouts in high-throughput screenings (as described, inter alia, in Pope, Drug Discovery Today 4 (1999), 350-362). The method of the present invention for identification, characterization and/or screening of molecules capable of interacting with glutamate receptors of the AMPA-type can, inter alia, employ hosts as defined herein which express the (poly)peptide of the present invention. Cell-based assays, instrumentation for said assays and/or measurements are well-known in the art and described, inter alia, in Gonzalez, Drug Discovery Today 4 (1999), 431-439 or Ramm, Drug Discovery Today 4 (1999), 401-410. For example, the coupling of an receptor activity to changes in intracellular Ca²⁺ is a general and powerful method for high throughput drug screening. Quantitative changes in intracellular calcium concentration can be detected by imaging techniques using Ca²⁺ sensitive dyes such as FURA II and their membrane permeable chemical analogs (Tsien, Biochemistry 19 (1980), 2396-2404; Grynkiewicz, Biological Chemistry 260 (1985), 3440-3450). Since homomeric AMPA receptors of the genes 1,3 and 4 are Calcium-permeable (Bumashev, Neuron 8 (1992) 189-198), cells, cell lines and hosts, expressing a (poly)peptide of this invention, i.e. a non-desensitizing version of an AMPA-receptor, such as the rat GluRl-mutant L497Y descibed in the appended examples, will show an increase of intracellular calcium concentration, depending on degree of glutamate receptor stimulation. Thus, the agonist and antagonist potency of a candidate molecule for AMPA-receptors is detectable using the (poly)peptide of the invention and/or employing the aforementioned receptors of the invention expressed in cell cultures.

Additionally, the specification also describes a method for the production of a pharmaceutical composition comprising the steps of the method of the invention for identifying, characterizing and/or screening of molecules which are capable of interacting with glutamatic receptors of the AMPA-type and further comprising a step, wherein a derivative of said identified, characterized and/or screened molecule is generated. Such a derivative may be generated by, inter alia, peptidomimetics.

The description furthermore describes a method for the production of a pharmaceutical composition comprising the steps of the method of the invention for identifying, characterizing, screening and/or derivatizing of molecules which are capable of interacting with glutamatic receptors of the AMPA-type and formulating the molecules identified, characterized, screened and/or derivatized in pharmaceutically acceptable form.

A further described method of the application relates to a method wherein said molecule(s) are neuroprotective and/or (a) nootropic molecule(s).

As described in the specification is a method, wherein said molecule(s) are antagonist(s), partial antagonist(s), partial agonist(s) and/or agonist(s) for glutamate receptors. Known agonists for AMPA-receptors comprise L-glutamate, quisqualate, (RS)-alpha-amino-3-hydroxy-5-methyl-4-isoxazoleepropionic acid (AMPA) or kainate (partial agonist); whereas known antagonsits are, inter alia, kynurate, 6-nitro-7-sulphamoyt-benzo(F)quinoxalinedion (NBQX) or I-glutamic acid diethyl ester (noncompetitive antagonist).

In accordance with the present invention, the term "antagonist" denotes molecules/substances, which are capable of inhibiting and/or reducing an agonisitic effect. The term "antagonist" comprises competitive, non-competitive, functional and chemical antagonists as described, inter alia, in Mutschler, "Arzneimittelwirkungen" (1986), Wissenschaftliche Verlagsgesellschaft mbH, Stuttgart, Germany. The term "partial antagonist" in accordance with the present invention means a molecule/substance that is capable of incompletely blocking the action of agonists through, inter alia, a non-competitive mechanism. As "agonist", in accordance with this invention, molecules/substances are denoted which have an affinity as well as an intrinsic activity. Mostly, said intrinsic activity (α) is defined as being proportional to the quotient of the effect, triggered by said agonist (E_{A}) and the effect which can be maximally obtained in a given biological system (Eₘₐₓ): therefore, the intrinsic activity can be defined as $α ∼ \frac{{E}_{λ}}{{E}_{max}}$

The highest relative intrinsic activity results from E_{A}/Eₘₐₓ=1. Agonists with an intrinsic activity of 1 are full agonists, whereas substances/molecules with an intrinsic activity of >0 and <1 are partial agonists. Partial agonists show a dualistic effect, i.e. they comprise agonistic as well as antagonistic effects.

The person skilled in the art can, therefore, easily employ the compounds and the methods of this invention in order to elucidate the agonistic and/or antagonistic effects and/or characteristics of a compound/molecule/substance to be identified and/or characterized in accordance with any of the above descibed methods.

The invention also relates to the use of a non-desensitizing AMPA-receptor as encoded by the nucleic acid molecule of the invention or the use of a host of the invention as a biosensor for glutamate concentrations. For example, a patch or cell in whole-cell configuration (sniffer) expressing a (poly)peptide, i.e. a nondesensitizing receptor, of the present invention can first be calibrated by briefly exposing it to saturating agonist concentration via external perfusion to determine the maximal current of the sniffer. Subsequently, the patch or cell is placed into a sample, tissue or specimen. In the presence of, for example, L-glutamate in the solution, a current should be produced by binding of glutamate to the receptors that in turn causes the opening of the channels. Since the developing current will follow strict dependency on the glutamate occupancy on the patch, the concentration of glutamate in the sample can be easily determined by comparing the fraction of the maximal current and the current induced by the specimen. These "sniffer patch" methods are well-known in the art and described, inter alia, in Hume, Nature 305 (1983), 632-634. The well defined dose-response curve for glutamate on the nondesensitizing receptor (as illustrated in the appended examples) allows then a reconstruction of the glutamate concentration. Ligand-gated ion channel receptors have been used to determine the identitiy of neurotransmitters in a qualitative manner (Copenhagen, (1989) Nature 341, 536-539; Allen, (1997) Trends Neurosci. 5, 192-197), however, the above mentioned technique furthermore allows the quantitative determination of glutamate concentration of an aqueous sample, whether it is of biological origin or not.

Furthermore, the present invention relates to the use of a non-desensitizing AMPA-receptor as encoded by the nucleic acid molecule of the invention or use of a host as defined herein above for the characterization of glutamate receptor channel properties. For example, by studying the single channel properties of non-desensitizing AMPA-receptors (like, inter alia, the GluR1-GluR6 and GluR3-GluR6 chimeras of the appended examples), it is possible to detect conductance states depending on the number of bound agonists or antagonists. The methods for such characterizations are well known in the art and comprise methods such as patch clamp. Techniques and methods for glutamate receptor property measurements are also described in Jahr, Nature 325 (1987), 522-525 or Swanson, J. Neurosci. 17 (1997), 58-69.

The invention further relates to the use of the nucleic acid molecule, of the vector, the host, the (poly)peptide and/or the antibody of the invention for the preparation of a pharmaceutical composition for preventing and/or treating neurological and/or neurodegenerative disorders.

In a preferred embodiment said neurological and/or neurodegenerative disorders are selected from the group consisting of Alzheimer's disease, Parkinson's disease, Huntington's disease, amyotrophic lateral sclerosis (FALS/SALS), ischemia, stroke, epilepsy, AIDS dementia and learning disorders.

In another embodiment the present invention relates to the use of the nucleic acid molecule, the vector and/or the host cell of the invention in gene therapy. For example, the nucleic acid molecules of the present invention could be expressed in tissues with pathologically low synaptic activity either due to breakdown of tissue (stroke, eplilepsy, postraumatic degeneration, ALS, Alzheimer) or through traumaticcally induced removal of input fibers (spinal cord injury). Furthermore, said nucleic acid molecules could be expressed in patients suffering from learning disorders.

Additionally, the specification describes a kit comprising the nucleic acid molecule, the vector, the host, the (poly)peptid, or the antibody of the invention or the molecule as identified or characterized in a method of the present invention.

Advantageously, the kit described above further comprises, optionally (a) reaction buffer(s), storage solutions and/or remaining reagents or materials required for the conduct of scientific or diagnostic assays or the like. Furthermore, parts of the kit can be packaged individually in vials or bottles or in combination in containers or multicontainer units.

The kit may be advantageously used, inter alia, for carrying out the method of producing a (poly)peptide of the invention, the method(s) of identification and/or characterization of molecules specifically interacting with glutamate receptors as descibed herein above and/or it could be employed in a variety of applications referred herein, e.g., as diagnostic kits, as research tools or therapeutic tools. Additionally, the kit may contain means for detection suitable for scientific, medical and/or diagnostic purposes. The manufacture of the kits follows preferably standard procedures which are known to the person skilled in the art. Furthermore, the kit may be used for the preparation of biosensors for glutamate concentrations.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

The figures show:

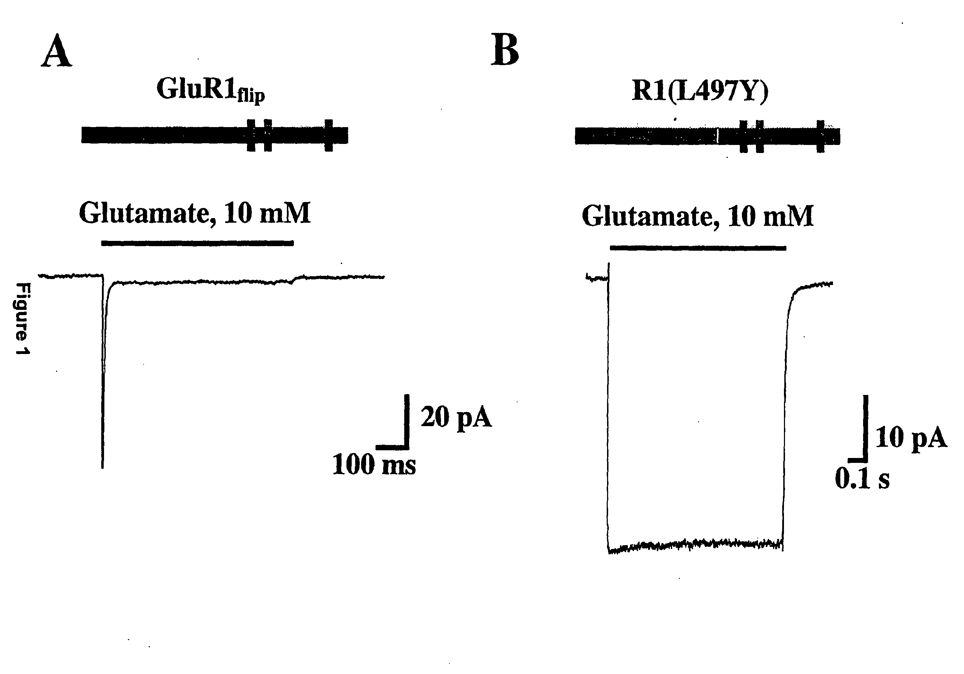

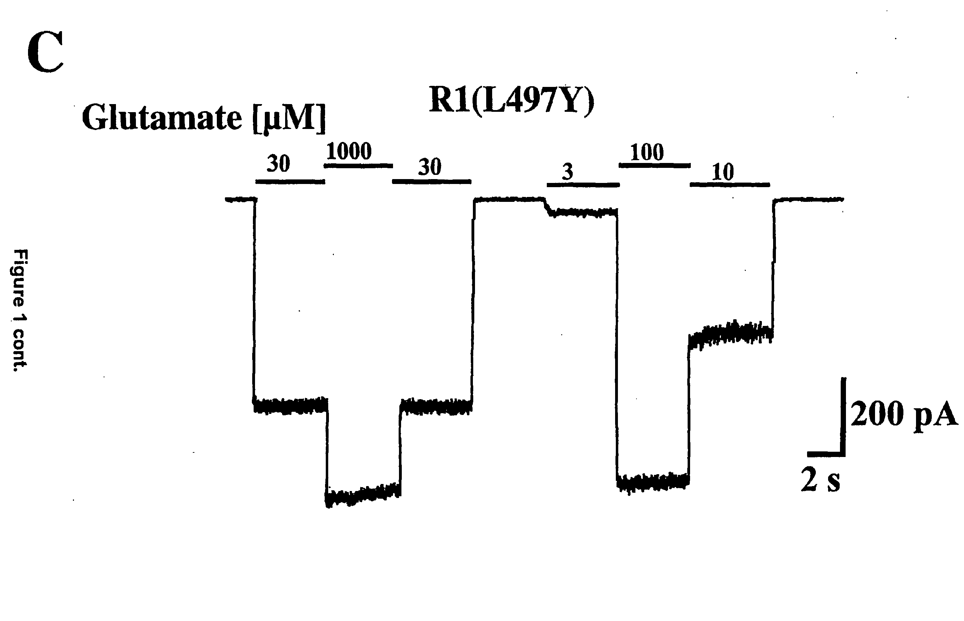

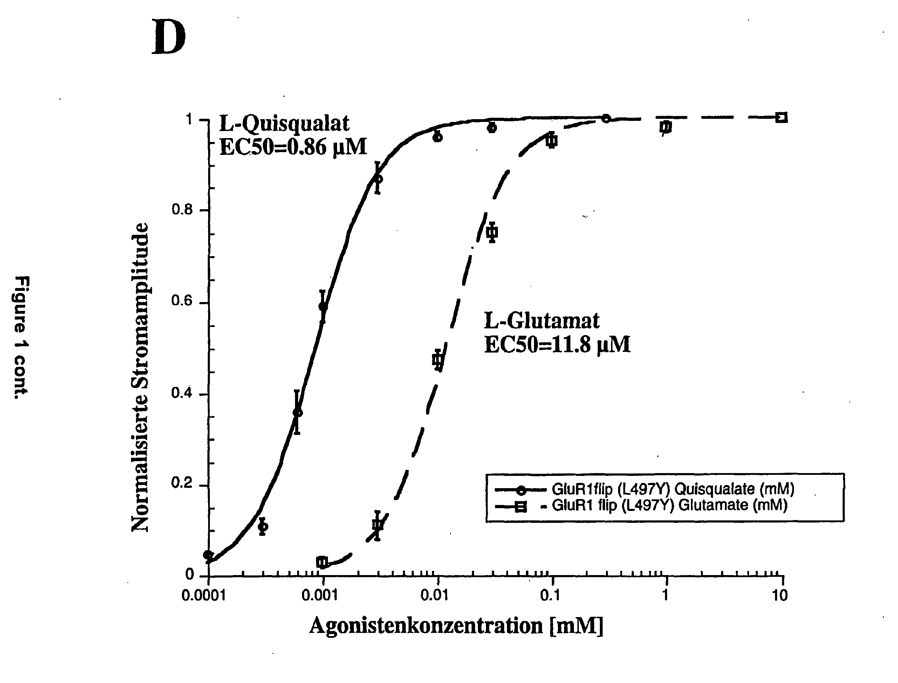

- **Figure 1:**: Desensitization properties of GluRI receptors
(A) Typical response to an application of 10 mM L-glutamate (1s duration, indicated with black bar) to an outside-out membrane patch obtained from a HEK293 cell transiently transfected with the GluR1 wildtype receptor. A rapidly desensitizing inward current is induced, that is only observable by application with a rapid perfusion system. The drawing above the current trace schematizes the primary amino acid structure of a single AMPA receptor subunit, with the putative transmembrane region indicated as thickening of the bar.
(B) Typical response to an application of 10 mM L-glutamate (1s duration, indicated with black bar) to an outside-out membrane patch obtained from a HEK293 cell transiently transfected with the GluR1 receptor with the point mutation Leucine to Tyrosine at position 497 (see SEQ ID NO: 1). During the entire period of agonist application, the agonist induced current is maintained in its activity. The white line within the first segment of the drawing schematizes the position of the point mutation.
(C) The current trace shows whole cell recordings from a cell transfected with GIuR1 L497Y during application of various concentrations of the agonist I-glutamate. The concentrations and the application periods of the solutions containing specific agonist concentrations are indicated as numbers above the black bars (in µMol). The solution exchange under whole cell recordings are app. 20 fold slower, but allow due to the nondesensitizing phenotype an accurate measure for the agonist efficacy and affinity.
(D) The graph shows the dose- response relationship from 11 whole-cell recordings such as shown as an example in Fig. 1C for the agonists L-glutamate and Quisqualate. Error bars indicate standard error and are extremely small, thus allowing accurate determination of affinity and efficacy with few experiments.

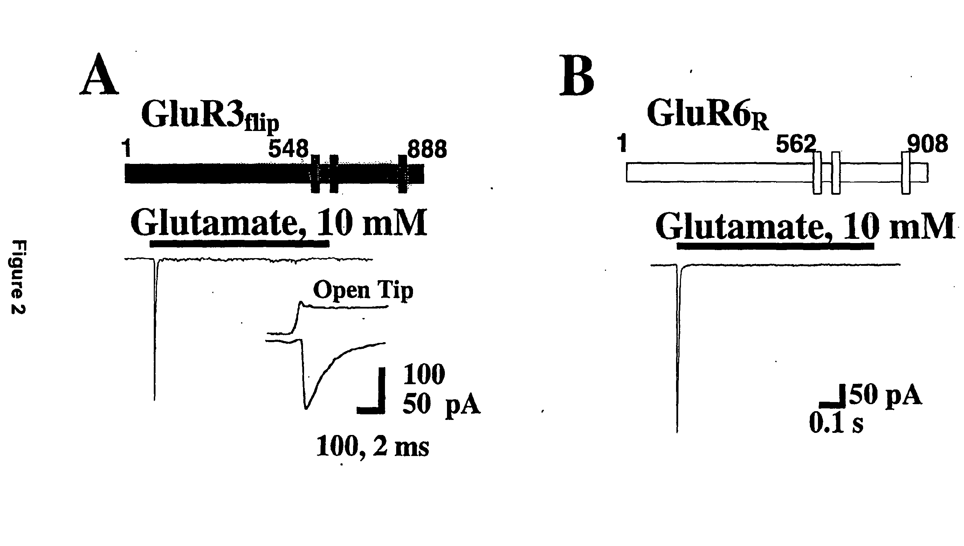

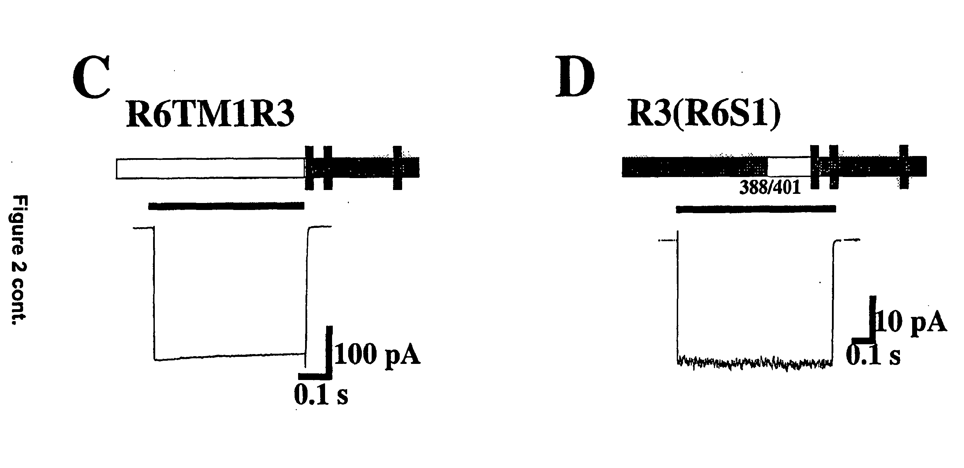

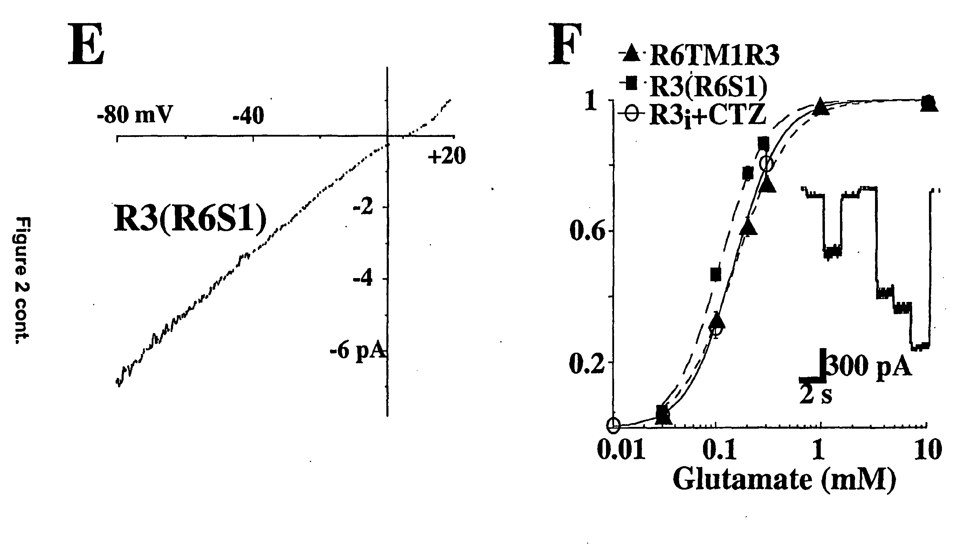

- **Figure 2:**: The role of the N-terminal region in AMPA-type glutamate receptor desensitization.
(A-D) Responses to rapid application of 10 mM glutamate from outside-out patches expressing homomeric receptors GluR3_{flip} (A), GluR6_{R} (B), R6TM1 R3flip (C), and R3(R6S1)_{flip} (D) measured at -60 mV. The subunit type is illustrated above each trace. Black bars correspond to GluR3, white bars to GluR6. The small three vertical bars correspond to the transmembrane domains M1, M3 and M4 respectively. The numbers correspond to the first, beginning of M1 and last amino acid, respectively (panels A-C) and those at the S1 junction (panel D). The amino acid numbering starts from the first methionine of the open reading frame. All responses are averaged from 2-50 episodes. Inset in panel A shows the same response on a 50 fold faster time scale. The solution exchange was estimated by the open tip current at the end of each experiment (as shown above inset).
(E) Current/voltage relationship of R3(R6S1)_{flip} in outside-out patch configuration. Voltage was ramped from -80 to +20 mV at 1 mV/ms. The trace represents an average of 7 episodes in the presence of 10 mM glutamate after leak subtraction. Patch solutions contained no polyamines.
(F) Dose-Response relationship for L-glutamate for GluR3_{flip} (in the presence of 100 µM cyclothiazide, white circles), R6TM1R3_{flip} (black squares) and R3(R6S1)_{flip} (black triangles) recorded in a whole-cell mode. Currents were normalized to the response at 10 mM. EC₅₀ and hill slope values (n) were estimated by fitting the concentration/current relationship with the equation Y=1/(1+(EC₅₀/[Glu]ⁿ)) and were 148 µM /1.95 for GluR3_{flip}, 155 µM/n=1.66 for R6TM1R3_{flip} and 107µM/n=2.02 for R3(R6S1)_{flip}, respectively. Data are from 5-9 cells each (at -60 mV). Error bar represents SE. Inset shows typical responses of a cell transfected with R3(R6S1)_{flip} to a series of glutamate concentrations. The order of concentrations were: control, .03, .1, .03, control .2, .3 and 10 mM).

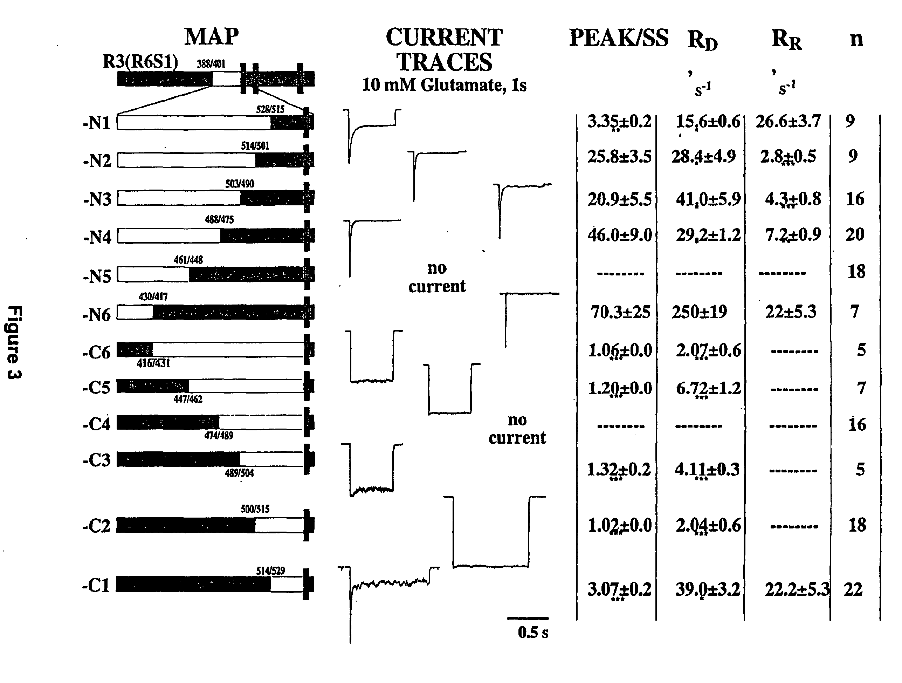

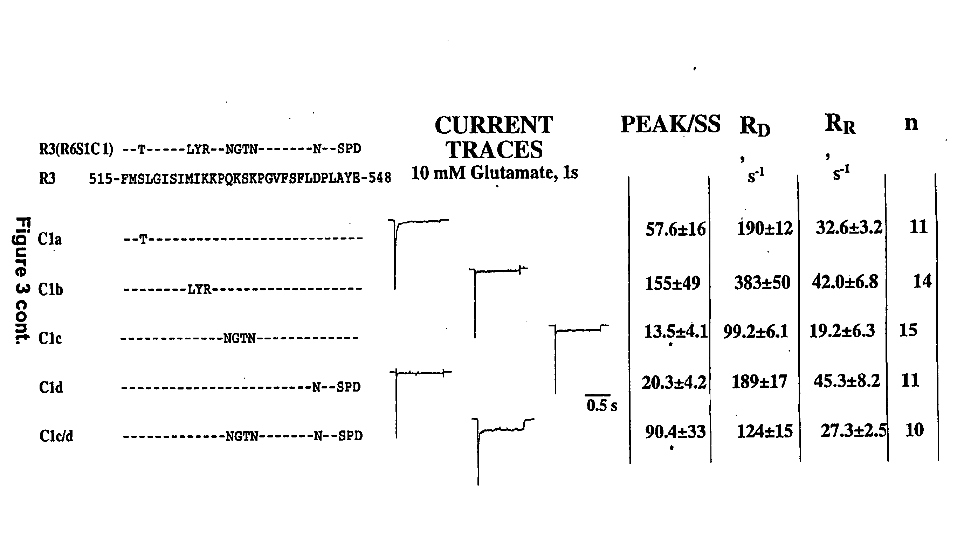

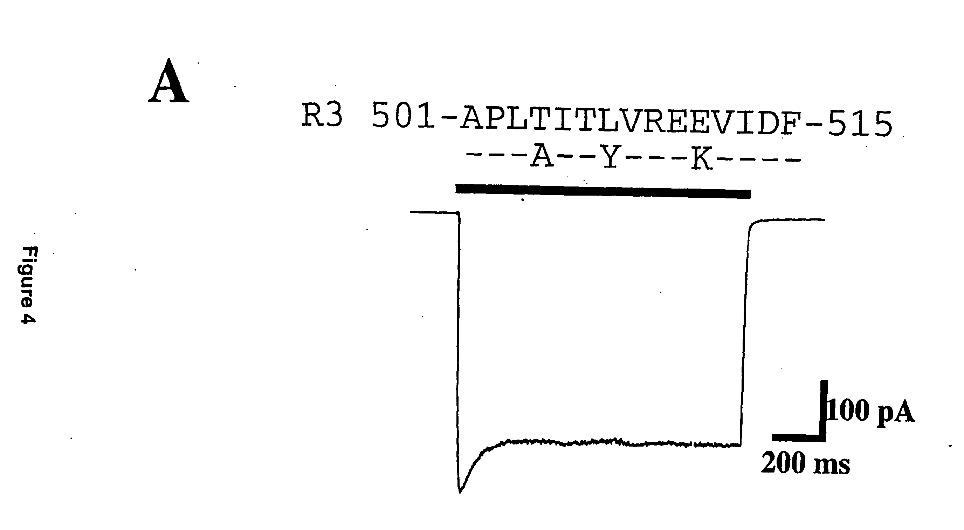

- **Figure 3:**: Desensitization properties of GluR3-'S1' chimeras.
(left column) Map of chimeras and point-mutations. (A) Respective S1 regions are shown in black (GluR3) and white (GluR6). The junction residues, given by their number, are shown above each bar and correspond to the color code. (B) GluR3-'C1' residues, 515-548, are shown in single letter code. Letters in 'C1' mutants indicate the GluR6 amino acid exchanges and their position. Middle column; typical current responses to a 1s-application of 10 mM glutamate. Vertical scale bars were omitted for display purposes. Peak response sizes ranged from 4-660 pA. Right column; peak/Steady-State-(P/S)-, desensitization rate (R_{D})- and resensitization rate (R_{R}) values ± standard error from 5-22 measurements each. Stars under the values indicate significant differences compared to GluR3_{flip} (*p<0.05; **p<0.01; ***p<0.0001)

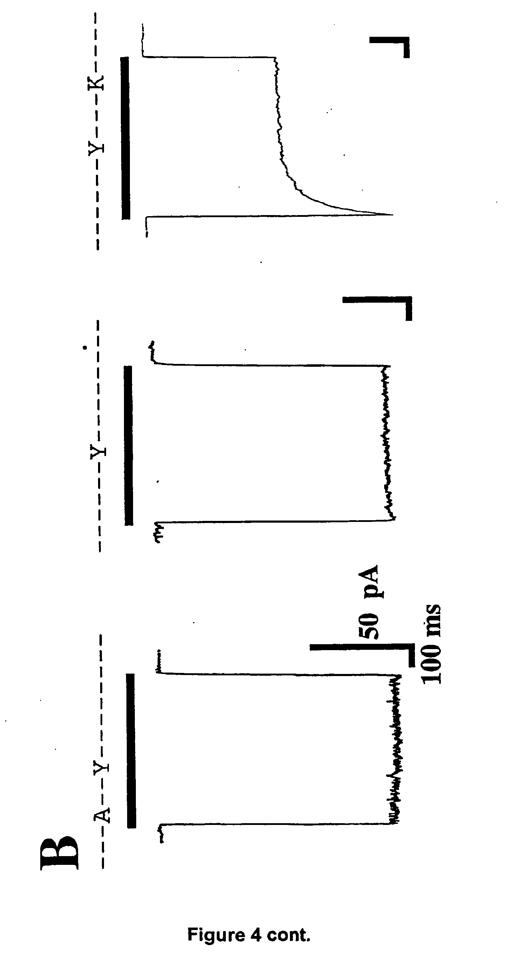

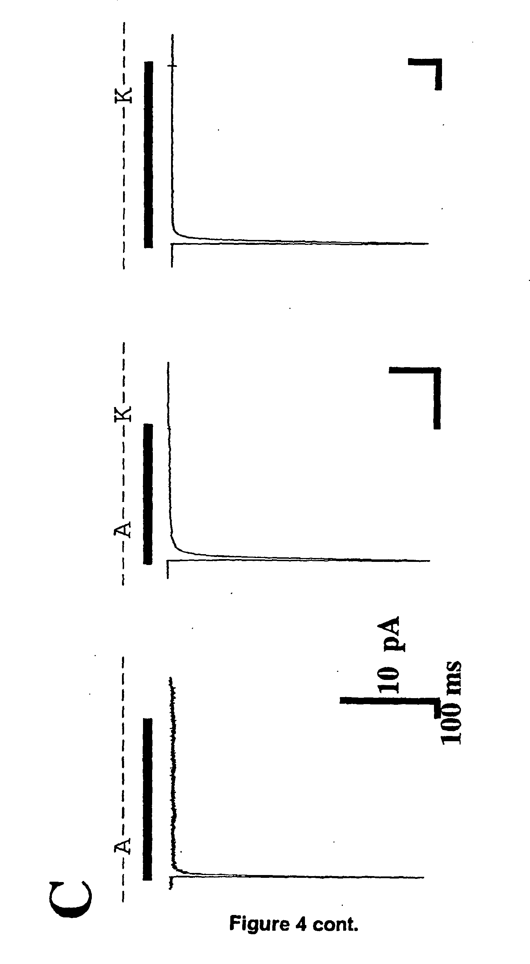

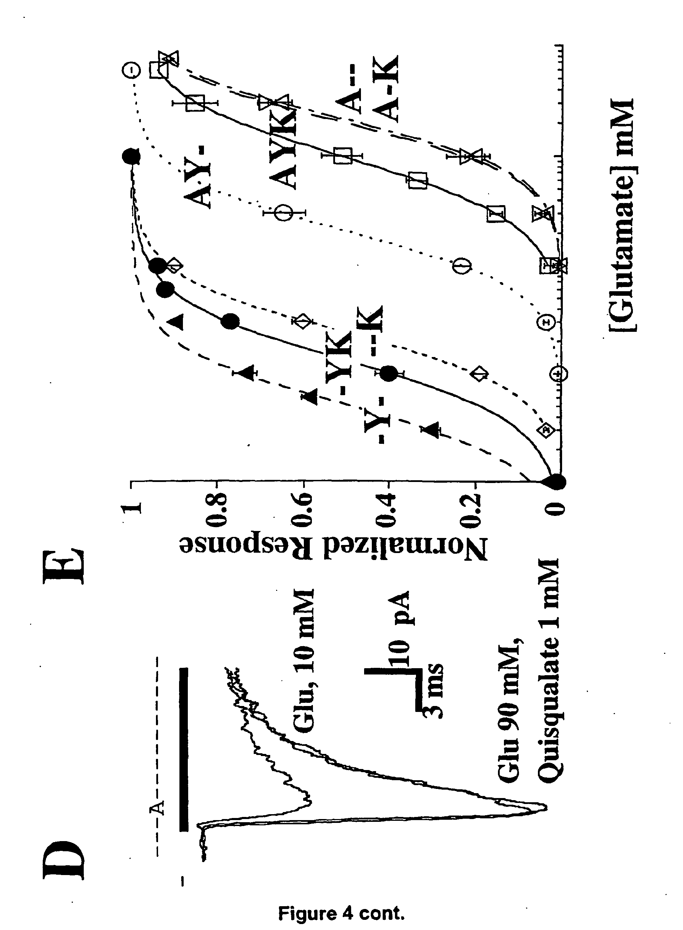

- **Figure 4:**: Mutations of T504A, L507Y, and E511K on GluR3_{flip} differentially control desensitization and agonist binding.
GluR6 residues that replace GluR3 residues are indicated by one letter code above each trace. (A) a response from a patch containing receptors with the triple point mutations T504A , L507Y, and E511K. Specific values obtained from 13 measurements were: P/S=1.51±.07, R_{D}=50.3±8 s⁻¹. (B) representative responses from receptors containing the L507Y mutation alone (middle: P/S=1.01±.01, n=12) and in combination with T504A (left: P/S=1.09±.04. n=8) or E511 K (right: P/S=2.1±.14, R_{D}=8.6±.45 s⁻¹, n=21). (C) representative responses from receptors mutated in T504A (left: P/S=26.0±5.1, R_{D}=90.3±7 s⁻¹, n=11), E511K (right: P/S=43.3±8, R_{D}=90.3±7 s⁻¹, n=11) or combined (middle: P/S=30.7±7.2, R_{D}=186±43 s⁻¹, n=9). All receptors were activated by 10 mM glutamate, except for those containing the T504A mutation where a concentration 90 mM has been used. (D) Superimposed responses from a patch containing R3(T504A) to 1 mM quisqualate, 10 mM and 90 mM glutamate as indicated. L-Quisqualate (1 mM) -induced desensitization was similar to desensitization evoked by glutamate (P/S=24.0±7.1, R_{D}=360±68 s⁻¹, n=8) (E) Dose-Response relationships to glutamate of the mutants shown in panels A-C (indicated by letter code on each trace) were measured as described in Figure 1 F; desensitizing receptors were measured in presence of 100 µM cyclothiazide. EC₅₀ and hill slope values (n) were: L507Y (-Y-)=48 µM/n=1.66; L507Y+E511K (-YK)=131 µM/n=1.52; E511K (--K)=236 µM/n=1.64; T504A+L507Y (AY-)=2.09 mM/n=1.64; T504A+L507Y+E511K (AYK)=9.6 mM/n=1.48; T504A (A-)=19.9 mM/n=1.81; T504A+E511K (A-K)=21.2 mM/n=1.81.
- **Figure 5:**: Specificity of L507 for AMPA receptor desensitization
(A-D) a typical response to application of 10 mM glutamate, at - 60mV, obtained from a patch containing the mutant GluR1(L497Y)_{flip} (A), GluR6(Y521L) (B), and R6TM1R3(Y521L) (C). (D) Glutamate evoked current in the presence of 100 µM cyclothiazide from the same patch as in panel C.
- **Figure 6:**: Aromatic residues in position 507 remove desensitization
(A-C) a typical response to application of 10 mM glutamate, at - 60mV, obtained from a patch containing the GluR3_{flip} mutant L507F (A), L507S (B) and L507T (C). Inset in C shows the same trace on a faster time scale. The time constant of desensitization is 0.72 ms. (D) desensitization rate R_{D} for various substitution at 507, indicated by one letter code for the respective amino acid. Significant deviations from the native receptor R3(L507), left are indicated with an asterisks.

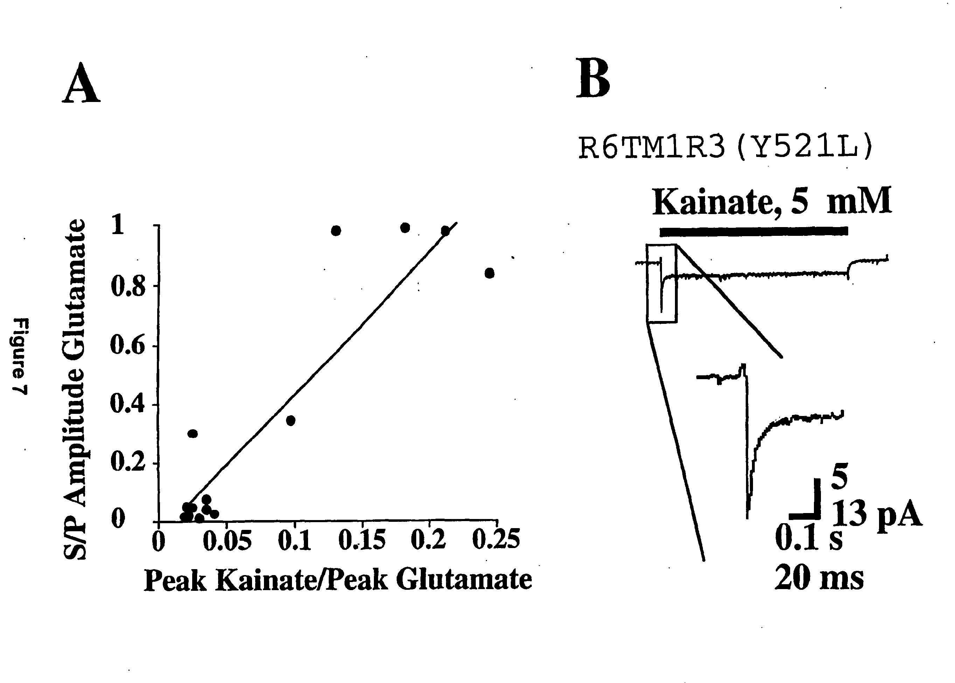

- **Figure 7:**: Kainate elicits fast desensitizing responses at AMPA receptors.
(A) Plot correlates the efficacy of kainate currents (expressed as the ratio of peak kainate current to peak glutamate current) to the Peak/Steady-state ratio of glutamate currents from all receptors examined in figures 1 and 2 (represented as black circles). Correlation coefficient was 0.91. (B) A typical response to application of 5 mM kainate, at -60mV, obtained from the 16 fold slower desensitizing chimera R6TM1R3(Y521L). Data are from the same patch as shown in figure 4C-D. Insert exemplifies kainate current desensitization.

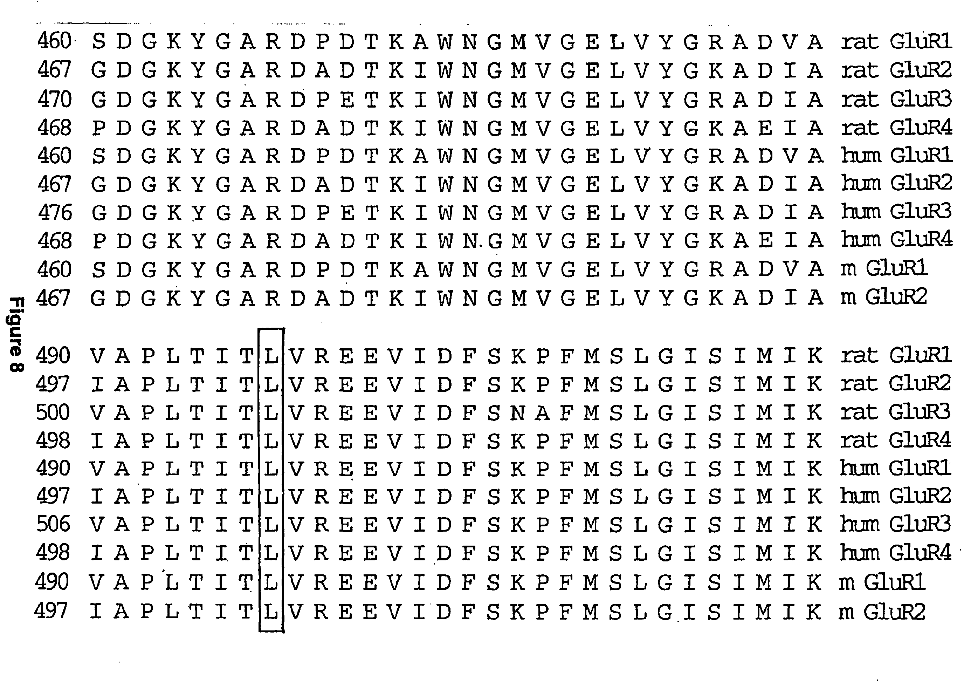

- **Figure 8:**: Alignment of partial amino acid sequences of different AMPA receptors (subunits) from rat, human and mouse
The relevant region of GluR1-4 from rat, GluR1-4 from human and GluR1 and 2 from mouse within the extracellular receptor binding region S1 (Stern-Bach, 1994 cit. loci) are shown. The alignment has been carried out employing the Clustal program, method 250. The relevant leucine (see box) is enwrapped by two residues (e.g. T494 and R499 in the case of the rat GluR1) which are essential for glutamate binding in all AMPA-receptors (Uchino, FEBS Lett. 308 (1992), 253-257).

The invention will now be described by reference to the following biological examples which are merely illustrative and are not to be construed as a limitation of scope of the invention.

### Example 1: Vector construction and use of nondesensitizing AMPA-receptor GluR1L497Y and its expression in mammalian or Xenopus oocytes expression for identification and/or characterization of AMPA-receptor ligand activities

Production of Mammalian/ Xenopus Oocytes Expression vector pC3G: pC3G was made by replacing the polylinker region of pCDNA3 (InVitrogen; Catalog No V790-20) with the polylinker of pGEM-HE (Liman et al., 1992, Neuron 9:861-871). GluR1 (flip) subcloned in the pGEM-HE vector (Promega) has been mutated using the method developed by Stratagene (QuickChange mutagenesis, catalog No. 200518) with the above oligonucleotides. The correct plasmid has been selected by the presence of the silent Nrul restriction site, and verified by DNA sequencing. The mutated cDNA insert has been cut out from pGEM-HE, by EcoRI and Nhel restriction enzymes, and subcloned in pC3G using the same restriction sites. The same vector can be used to either transiently transfect mammalian cell lines or to produce mRNA for injection into oocytes.
The glutamate receptor was transfected in cell lines HEK293 (Clements, Neuron 7 (1991), 605-613) using the calcium phosphate method as described in Chen and Okayama, Biotechniques 6 (1988), 632-638. The detection of transfected cells was facilitated by cotransfection of an EGFP marker gene (eg. pGreen latern^{™}-1, Gibco#10642-015)). Transfected cells were visualized using a inverted microscope equipped with fluorescence and the detection of green fluorescent cells was performed using 480nm excitation and 520 nM emission filter set. Membrane currents from these cells were performed using standard patch clamp whole-cell or outside-out patch recording techniques (Hamill et al., Pflugers Archiv - European J. of Physiol. 391 (1981), 85-100. As shown in Figure 1A, typical responses from membrane patches expressing the native wildtype AMPA-receptor GluR1 show a rapid desensitizing current by exposure 1s duration (black bar) of saturating concentrations of glutamate (10 mM). The peak current can only be observed when using a fast application system (Clements, 1991, loc. cit.), as under these conditions the drug application proceeds faster than the apparent receptor desensitization. In contrast, responses from rat GluR1 with the point mutation Leucine to Tyrosine at the position 497 shows a nondesensitizing response (Fig 1B). The gray bar scheme above the responses indicates schematically the position of the mutation in the primary receptor sequence (left N-terminus, right C-terminus, thick bars represent putative transmembrane regions, the first segment is extracellular (Hollmann and Heinemann, 1994). In whole cell recordings, agonist and antagonist activity measurements can be easily performed using the GluR1L497Y receptor as exemplified in Fig 1C. Glutamate is applied to the extracellular space at the concentration indicated above the black bars by exchanging the external solution to the appropiate agonist containing solutions. Since the receptor exhibit nondesensitizing responses, the solution exchange profile is neglegible for the activity measurement. Fig 1 D shows the measured dose-response profile for the receptor GluR1L497Y using whole-cell measurements from 10 cells and two different specific AMPA-receptor agonists I-glutamate and L-Quisqualate.

### Example 2: Construction of Chimeras and Mutants for further analysis

Chimeras N1-N6 and C1-C6 were made as previously described (Stern-Bach, loc. cit.). Chimeras were constructed by polymerase chain reactions according to the strategy of gene splicing by overlap extension (Horton, Gene 77 (1989), 61-68). Making use of the redundacy of the genetic code, primers for each chimer or mutant were designed so as to introduce diagnostic restriction enzyme cleavage sites, which allowed for rapid screening for mutant genotype. Chimeric and mutant cDNAs were confirmed by double-stranded sequencing with cDNA-specific oligonucleotide primers and were subsequently inserted in mammalian exprssion vector pcDNA 3G. Point mutations were synthesized using the PCR-based method described by the 'QuickChange' mutagenesis (Stratagene). All mutants were first subcloned by an appropriate digest in GluR3_{flip}-pGEMHE (pGMHE from Promega), and subsequently moved into pCDNA3 (InVitrogen) for expression in mammalian cells. All mutations were verified by double-strand DNA sequencing. The original 'flop' module of chimeras R6TM1R3 and R3(R6S1) (Stern-Bach, loc. cit.) has been exchanged by the corresponding 'flip' module using a Sall/Xbal digest of GluR3_{flip}. R6TM1R3 is a chimera containing the backbone structure of rat AMPA-receptor GluR3 in which the N-terminal portion has been replaced by the N-terminal portion of the rat kainate receptor GluR6 (see schematic drawing fig. 2C. The receptor R3(R6S1) is the AMPA-receptor GluR3 with the first 154 amino acids preceeding the first transmembrane have been replaced by the related structure in the rat kainate-receptor GluR6 (see scheme Fig. 2D). Amino acid numbering starts from the first methionine of the open reading frame.

### Example 3: Exchanging the Binding Domain S1 of GluR3 with S1 of GluR6 Results in a Fully Active, But Completely Non-Desensitizing Receptor.

AMPA- and kainate-type glutamate receptor-channels have characteristic desensitization and resensitization kinetics. These were measured in HEK293 cells transiently transfected with pcDNA3 vectors containing GluR3_{flip} (an AMPA receptor) or GluR6 (a kainate receptor) cDNA. For all kinetic measurements outside-out patch recordings in combination with a rapid solution exchange system (Clements and Westbrook, Neuron 7 (1991), 605-613; Colquhoun, J. Physiol-London 458 (1992), 261-287) were employed in order to obtain solution exchanges faster than the rate of desensitization measured for these glutamate receptors. Patches were exposed to 0.5-2 s long pulses of saturating glutamate concentrations (10 mM).
in general, outside-out patches were obtained from the human embryonic cell line HEK293 (ATCC CRL 1573, USA) expressing homomeric channels composed of rat GluR3_{flip}, GluR6_{R} or chimeras, 12-96 hours after transfection, using the Ca₂(PO₄)₃-method (Chen and Okayama, Biotechniques 6 (1988), 632-638). Transfected cells were detected as described (Margolskee, Biotechniques 15 (1993), 906-911). All kinetic measurements were obtained from outside-out patches to maximize solution exchange rates. After excision of the patch, the patch was moved into a stream of a rapid perfusion system (Clements and Westbrook, loc. cit.; Colquhoun, loc. cit.). Solution exchange (20-80% to peak) was judged by open tip control by diluting the control solution with 2% water ranging from 0.3-0.6 ms. Experiments were performed at room temperature (20-25° C). Repeated agonist application was done at 0.2-0.02Hz. Recovery from desensitization were measured by paired-pulse application of agonist. Pipettes were filled with a solution containing 150 mM CsF or CsCl, 20 mM HEPES, 10 mM NaCl, 10 mM EGTA, adjusted to 305 mOsm, pH 7.3. Holding potential was usually - 60 mV. Currents were amplified using an Axopatch amplifier 200 B (Axon Instr., USA), filtered at 1-10 kHz and digitized at 2-20 kHz using pClamp 6.0 (Axon Instr., USA) acquisition system. The extracellular medium contained 170 mM NaCl, 10 mM HEPES, 2-4 mM CaCl₂, 2-4 mM MgCl₂, adjusted to 330 mOsm, pH 7.25. Agonist solutions were made by mixing external medium with isotonic (330 mOsm, pH 7.3) agonist stock solutions by replacing NaCl with the agonist. Analysis was performed using Axograph 3.5 software, and exponential were fitted using the squared error method. Multiple measurements from one patch were averaged and the results were treated as one experiment. Significance of results were determined by analysis of variance followed by Dunns posthoc comparison and are indicated when p<0.05.
As shown in Figure 2A-B, applying the agonist at a holding potential of -60 mV, evoked a rapidly evolving and strongly desensitizing inward current for both GluR3 and GluR6 homomeric channels. The amount of desensitization expressed as the ratio of peak to steady-state amplitude (P/S) was 46.2±3.9 for GluR3_{flip} (n=13) and 236±53 for GluR6 (n=14). For the rate of desensitization (R_{D}; the inverse of the desensitization time constant) 240±15.4 and 225±20 s⁻¹, respectively, was measured. Desensitization was blocked by cyclothiazide (100µM) and Concanavalin A (1µg/µl) when added to the agonist solution of GluR3_{flip} or GluR6 respectively; resulting in P/S values close to 1 (not shown). The rate of recovery from desensitization (R_{R}) was measured in paired-pulse protocols and was ~50 times faster for GluR3_{flip} than for GluR6 (29.9±7.1 s⁻¹ and 0.57±0.06 s⁻¹, n=10 respectively). The kinetic characteristics of GluR3_{flip} and GluR6 are consistent with published values and comparable to native channels (Trussell, Proc. Natl. Acad. Sci. USA (1988), 4562-4566; Sommer, loc. cit.; Heckmann, Biophysical Journal 71 (1996), 1743-1750; Traynelis and Wahl, J. Physiol. 503 (1997), 513-531).
In order to identify specific protein domains modulating receptor desensitization, responses to glutamate from chimeric GluR3-GluR6 receptors were analyzed (Stern-Bach, (1994) loc. cit.). In contrast to both parent receptors, one N-terminal chimera, termed R6TM1R3 (Figure 2C), in which the entire extracellular N-terminal region of GluR3_{flip} was substituted by the corresponding region of GluR6, showed complete removal of desensitization (Figure 2C; P/S=1.02±.01, n=30).
Several studies have indicated that AMPA receptor desensitization is modulated by the 'flip/flop' region located in S2 (Sommer, 1990 loc. cit.; Mosbacher, 1994 loc. cit.; Partin, 1994 loc. cit.; Partin, 1995 loc. cit.). Analysis of the 'flop' version of chimera R6TM1R3 also showed complete removal of desensitization (P/S=1.07±.04; n=9, not shown), suggesting that the removal of desensitization does not require specific splice variants in the 'flip/flop' cassette.
Based on the homology to bacterial proteins and functional studies, the N-terminal region can be separated in two: the LIVBP-like domain and the agonist binding domain S1. These two regions were examined separately by measuring the kinetic properties of chimera R3(R6S1), in which the GluR6 exchange was limited to S1, and chimera R6KBPR3, in which the GluR6 exchange was limited to the LIVBP-like region. Chimera R3(R6S1) exhibited a fully non-desensitizing response (Figure 2d; P/S=1.01±.01, n=6), whereas chimera R6KBPR3 resulted in a receptor indistinguishable from GluR3 (P/S=56.2±22; R_{D}=232±44 s⁻¹; R_{R}=19.3±5.2 s⁻¹, n=6; not shown). The LIVBP-like region was recently reported to affect glycine-independent NMDA receptor desensitization (Krupp, 1998 loc. cit.; Villarroel, 1998 loc. cit.). To further test its possible role in desensitization, the kinetic properties of the reverse chimera R3KBPR6 and chimera NR1KBPR6, in which the LIVBP-like domain was taken from the NMDA receptor subunit NR1a (Stern-Bach, 1994 loc. cit.), were also checked. These two chimeras desensitized in a manner similar to GluR6 (R3KBPR6: P/S=94.3±23, R_{D}=341±45 s⁻¹, R_{R}=0.24±0.07 s⁻¹, n=5; NR1KBPR6: P/S=83.1±33, R_{D}=411±73 s⁻¹, R_{R}=0.31±0.09 s⁻¹, n=4). Thus, abolishing desensitization in GluR3 by the chimeric exchange, is exclusively a result of replacing the agonist binding domain S1.
The possibility that the observed lack of desensitization for chimeras R6TM1R3 and R3(R6S1) could be due to some other form of kinetic change were excluded for three reasons. First, desensitization of AMPA receptors can be blocked by cyclothiazide. Since both chimeras carry the 'GluR3-flip' region important for cyclothiazide binding (Partin, 1995 loc. cit.; Partin, 1996 loc. cit.), any occluded desensitization should be revealed by an increase of the peak response in the presence of this drug. However, addition of 100 µM cyclothiazide to the agonist solution (a concentration which increases peak responses of GluR3_{flip} up to three fold together with a complete block of desensitization; Partin, 1994 loc. cit.), resulted in an 14±4% and 12±3% inhibition of the peak response of R6TM1R3 (n=13) and R3(R6S1) (n=10) respectively. This inhibition is similar to that observed for AMPA receptors saturated with cyclothiazide, after rapid removal of the drug from the external solution (Partin, 1993 loc. cit.; Partin, 1994 loc. cit.). Second, the steady-state amplitude of a desensitized AMPA receptor is in the range of 2.5% of the peak response. Assuming similar channel densities, the responses from the chimeric receptor should be quite small. However, patch responses were 156±78 pA (n=30), ~13 fold larger than the average peak responses to GluR3, and ~3 fold larger than responses of GluR3 when treated with cyclothiazide. Third, a desensitized receptor state should be reflective in its single channel behavior by either smaller conductance states, shorter mean open times or longer shut times. On occasionally occurring patches that contained only a single chimeric channel, the channel opened to an apparent 23 pS state with a very high open probability (88.3±5%, at 10 mM glutamate); similar to the conductance behavior observed with GluR3 single channels treated with cyclothiazide.
Finally, since S1 is exclusively located on the extracellular site and is part of the ligand binding domain, mutagenesis may influence agonist binding but not ion permeation. Consistent with that, no obvious differences in the current voltage properties between GluR3_{flip} and the chimeras R6TM1R3 and R3(R6S1) were found (Figure 2E). Both chimeras responded to glutamate in a dose dependent manner which was similar to that observed for GluR3_{flip} (Figure 2F and see also Stern-Bach, 1994 loc. cit.). As agonist potency strongly depends on receptor desensitization (Trussell and Fischbach, Neuron 3 (1989), 209-218; Patneau and Mayer, J. Neurosci. 10 (1990), 2385-2399; Patneau, J. Neurosci. 13 (1993), 3496-3509; Yamada and Tang, J. Neurosci. 13 (1993), 3904-3915; Partin, 1994, loc. cit.), desensitization of the native receptor GluR3_{flip} was removed by coapplying the desensitization blocker cyclothiazide. Based on the lack of receptor desensitization, these measurements were carried out in whole-cell recordings that allowed a more accurate measurements of current amplitudes. Measurements were taken as described in Rosenmund, J. Neurosc. 15 (1995), 2788-2795. Potency values obtained from patches showed identical values and were thus pooled. Interestingly, cyclothiazide reduced glutamate potency from 155 µM to 398 µM for R6TM1R3 (n=5) and from 107 µM to 199 µM for R3(R6S1) (n=6). A similar reduction in affinity was observed for [³H]AMPA binding to rat brain membranes when treated with cyclothiazide (Kessler, Mol. Pharmacol. 49 (1996), 123-131).
Taken together, these results show that abolishing desensitization in R6TM1R3 and R3(R6S1) does not result in gross alteration of other receptor-channel functions. It also suggests that desensitization is an active gating process independent from the process of activation.

### Example 4: Three Distinct Regions in S1 Modify Desensitization Properties of GluR3 Receptors.

The S1 region of GluR6 consists of 162 amino acids, of which 79 are different from GluR3. In order to identify the residue(s) responsible for regulating desensitization, twelve new 'S1' chimeras, consisting of progressively smaller and complementary GluR6 substitutions (N1-N6 and C1-C6, Figure 3) were constructed. All of the functional C-terminal chimeras altered the desensitization properties of the GluR3 'parent'. Chimeras C6, C5, C3 and C2 did not desensitize, while C1 was partially desensitizing (Fig. 3). The kinetics of 'C1' was significantly different from both GluR3_{flip} (p<.001) and 'C2' (p<.001), suggesting that at least two sites within 'C2' modify desensitization.
The 34 amino acid region exchanged in 'C1' is proposed to include one of the hinge regions connecting the two agonist binding lobes (Stern-Bach, 1994, loc. cit.; Sutcliffe, Biophysical Journal 70 (1996), 1575-1589; Swanson, 1997, loc. cit.) and was recently found to be involved in glycine-independent NMDA receptor desensitization (pre-M1; Krupp, 1998, loc. cit.; Villarroel, 1998, loc. cit.). The role of the 12 residues in R3(R6S1C1) that are different from GluR3 were further examined, by grouping them into four different chimeras (C1a-d, Figure 3). In comparison to the 'C1' exchange, the desensitization of all 'C1a-d' chimeras were statistically different (p<0.05), suggesting that multiple combinations of mutations are required to produce the 'C1' phenotype.
In addition to the exchanges made at the C-terminus of S1, exchanges made at the N-terminus also modified desensitization properties of GluR3_{flip}. Chimeras 'N2', N3' and 'N4', but not 'N6' exhibited significant reductions in both desensitization and resensitization rates (Figure 3). Thus, residues located in the region between R417-Y474 may also be involved in desensitization.
Three distinct regions in S1 modify desensitization properties of GluR3 receptors. One is situated between R417-Y474 (cross of 'N4' and 'N6' exchanges), the second one between A501-D514 (cross of 'C2' and 'C1' exchanges) and the third one between F515-E548 ('C1').

### Example 5: A Single Exchange in the Vicinity of Residues that Bind Glutamate Removes Desensitization of GluR3 Receptors.

The region substituted in the 'C2' chimera, excluding 'C1' (i.e. A501-D514) contains only three amino acids that differ between GluR3 and GluR6. These are T504A, L507Y and E511K (Figure 4). A simultaneous exchange of all these three amino acids resulted in a barely desensitizing receptor (Figure 4A). Exchange of single amino acid residues within these positions reveal that L507Y accounted entirely for the removal of desensitization (Figure 4B middle). Its effect was slightly reduced when combined with E511 K (Figure 4B, right) but not with T504A (Figure 4B; left). In addition to glutamate, quisqualate (1 mM; P/S=1.03±0.06, n=34) or AMPA (1 mM; P/S=1.05±0.03, n=24) also elucidated non-desensitizing responses with an identical efficacy of opening as glutamate (glutamate/quisqualate=1.02±0.02 and glutamate/AMPA=0.97±0.03, n=7, respectively). Desensitization was also abolished by the L507Y mutation when introduced into the flop version of GluR3 (P/S=1.01±.04, n=12; not shown). In contrast to the L507Y exchange, T504A, E511K or their combined exchange had no effect on the desensitization rate (Figure 4C) nor on the resensitization properties of GluR3_{flip}. Moreover, desensitization of these three later mutants, was completely blocked by cyclothiazide (100 µM). In contrast, cyclothiazide reduced peak response of L507Y by 9.6±2.7% and reduced the affinity for glutamate from 48 to 262 µM (n=6), similar as what was observed for the nondesensitizing chimeras R6TM1R3 and R3(R6S1).
Interestingly, it was found that all mutants containing the T504A exchange, evoked a weak response to 10 mM glutamate; usually a saturating concentration (GluR3_{flip} receptors, Figure 2F). Responses evoked by quisqualate (1 mM) applied to the same patch usually about 3 fold larger compared to the response evoked by 10 mM glutamate (see Figure 4D). The difference between glutamate and quisqualate was observed on both desensitizing and nondesensitizing receptors. Since T504 is proposed to directly interact with glutamate (Stem-Bach, 1994, loc. cit.; Paas, Neuron 17 (1996), 979-990, Laube, Neuron 18 (1997), 493-503) and resides near R509, a residue shown by mutagenesis to be critical for agonist binding (Uchino, FEBS Lett. 308 (1992), 253-257) it was tested whether a change in glutamate efficacy or affinity had occurred. Dose-response analysis revealed that all mutants containing the T504A substitution exhibited more than 50 fold increase in the EC₅₀ for glutamate (Figure 4E). The responses at saturating concentrations equaled the response amplitude of quisqualata (Figure 4D), indicating that the efficacy of channel opening was not affected by the mutation T504A.

The effects on glutamate potency of both positions T504A and L507Y were independent to each other, as the introduction of the T504A mutation led to a parallel reduction of potency (Y>AY=44-fold, YK>AYK=73-fold; K>AK=90-fold; Figure 4E), suggesting that the mechanism of the affinity shift were independent. In summary, the mutations in the region T504-E511 reveal an intriguing convergence of agonist binding and receptor desensitization.

### Example 6: Specificity of Position L507 to AMPA Receptor Desensitization.

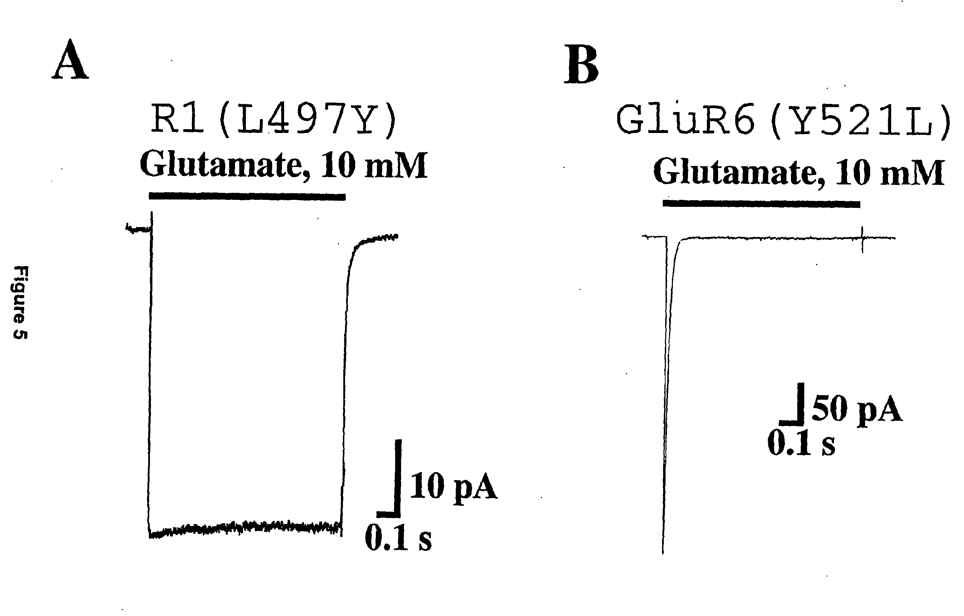

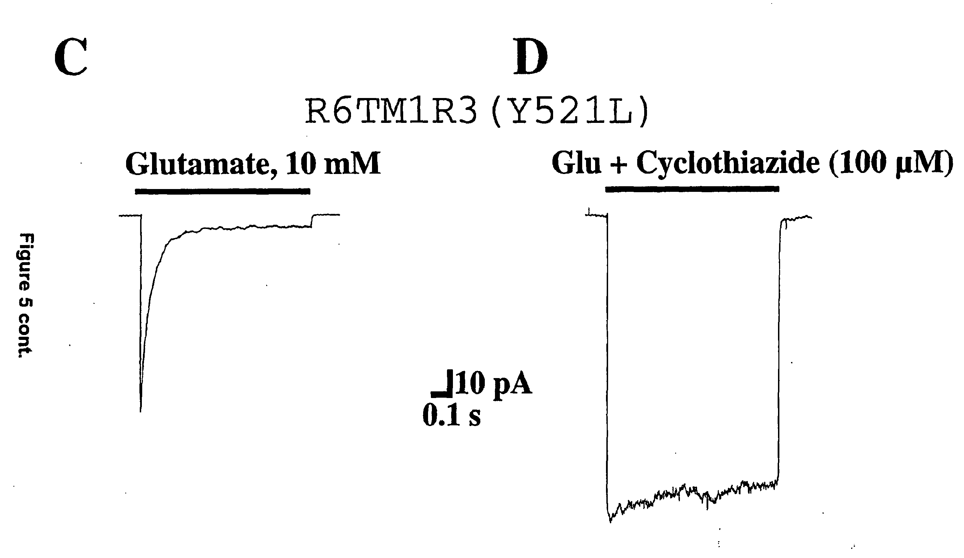

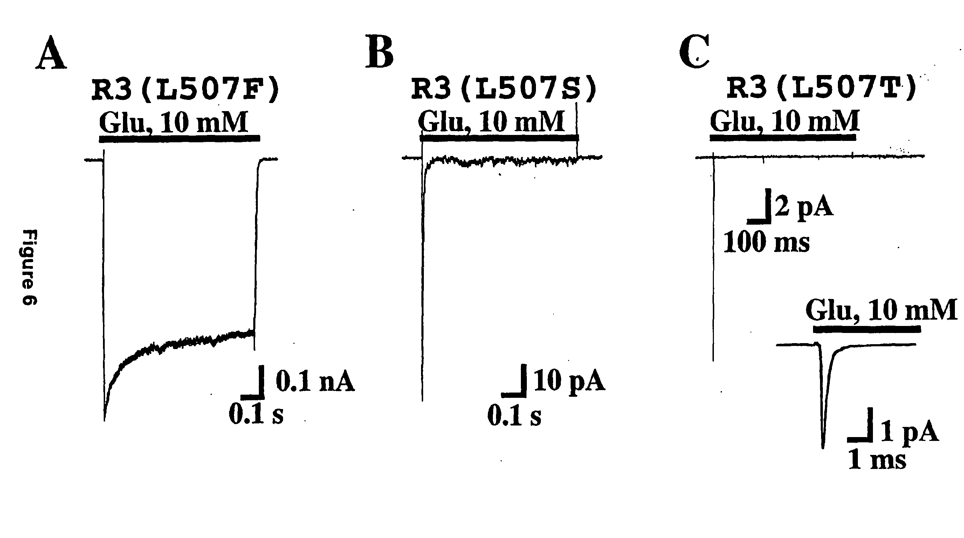

The AMPA receptor subunits GluR1-4 share high sequence homology in the S1 region (>85%), suggesting that a leucine to tyrosine exchange on other AMPA receptor subunits as well as in native AMPA receptors, would lead to the same phenotype. As shown in Fig. 5A, desensitization of the point mutant GluR1 L497Y is blocked with rapid application of 10 mM L-glutamate (see also Fig. 1C). This behavior is identical to the block of desensitization in the point mutant in AMPA-receptor GluR3 L507Y (Fig. 4B, middle trace). Thus, as shown hereinabove, block of desensitization is not limited to the AMPA-receptor subunit 1, but is also applicable to other AMPA-receptor subunits such as GluR3, GluR2 or GluR4. This is also not surprising, as the region in which the mutation is performed is to 100% identical between all AMPA-type Glutamate receptors (Fig. 9). The point mutation is very specific for AMPA receptors compared to other glutamate receptors, as shown in Fig. 5B, as the reverse substitution tyrosine to leucine at the kainate receptor subunit GluR6 (the corresponding position is 521) does not affects kainate receptor desensitization (see for comparison Fig. 2B). The uniqueness and specificity of the herein identified position is further demonstrated by the introduction of the reverse point mutation for the chimera R6TM1 R3 (which is nonesensitizing; Fig. 2C). The receptor R6TM1R3 (Y521L) does show desensitization properties similar to AMPA receptors. This result implies that Y521 is not involved in kainate receptor desensitization, although it was possible that a change to something other than leucine might have an effect. However, mutations of Y521 to glycine (n=7), valine (n=4) and glutamate (n=4) resulted in desensitization properties indistinguishable from GluR6 wild-type (not shown). Therefore, this particular site (R3-507/R6-521) appears to be specific for AMPA-but not kainate receptor desensitization. In summary, the point mutation is highly unique within the AMPA-receptor subunit, is specific for the AMPA-receptors in the glutamate receptor family, but is shared within all AMPA receptor subunits GIuR1-GluR4, based on the highly identical protein structure within the ligand binding domain. These results are thus identical to the observations made with GluR 1 point mutant L497Y.
As pointed herein above, to further test the specificity of site L507, a reverse mutation on the kainate receptor GluR6 was performed. Mutant R6(Y521 L) was almost identical in its kinetics when compared to the wild-type GluR6 receptor (Figure 5B).
The 'N1' exchange (Figure 3) - which includes the L507Y mutation - resulted in a partially desensitizing receptor, similar as found for the double mutation L507Y+E511K compared to L507Y alone (Figure 4B-right vs. middle). Thus, the control of desensitization by position 507 may either be modulated by other residues, or position 507 is necessary but not specific for the control of desensitization. To test this, the effect of a reversed Y to L mutation on the non-desensitizing R6TM1R3 chimera was first measured (see Figure 2C). The resulting R6TM1 R3(Y521 L) receptor, gained back almost complete desensitization, but with a 16-fold slower rate (R_{D}=15.4±1.1 s⁻¹; P/S=11.5±2.1, n=8; Figure 5C). Desensitization was blocked by cyclothiazide (Figure 5D) and resensitization was not different compared to GluR3_{flip} (R_{R}=14.2±4.2 s⁻¹, n=3), suggesting that the kinetic characteristics of mutant R6TM1 R3(Y521 L) resemble those of GluR3. Next, the role of position E511 was further examined. Recent molecular modeling of the glutamate binding domain predict that the region T506-V512 is α-helical. Both L507 and E511 are situated on the surface of lobe 1 with about the same orientation. The interaction observed between these two sites could be thus explained by either specific interactions between positions 511 and 507, or by the option that the entire α-helix nonspecifically controls desensitization. It was tested whether a tyrosine residue at position 511 will also result in a non-desensitizing receptor. However, mutant R3(E511Y) exhibit desensitization properties characteristic of the wild-type receptor (P/S=43.9±13; R_{D}=383±50 s⁻¹;

R_{R}=27.9±8.0 s⁻¹, n=4). Taken together it can be concluded that L507 is specifically required for AMPA-type receptor desensitization to occur, but with an additional modulatory effect of surrounding residues on this position.

### Example 7: Removal of Desensitization Requires the Exchange of L507 to an Aromatic Residue.

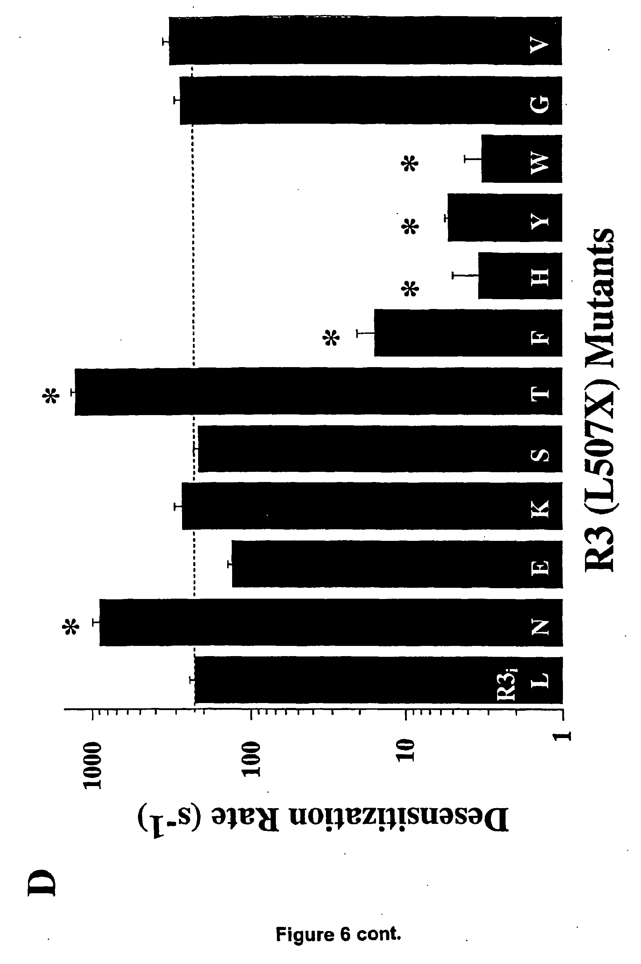

Placing tyrosine, a aromatic residue onto position 497 into the AMPA receptor GluR1 results in a nondesensitizing phenotype (Fig. 1B,C). Based on the identical structure of the ligand binding domain between the AMPA receptor GluR1 and another AMPA receptor GluR3 (Fig. 9), its desensitization properties and the nature of removal of desensitization can be further analyzed by introducing residues other than tyrosine at the corresponding position 507 of the AMPA-receptor GluR3 as shown in Fig. 6. Therefore, in order to understand the nature of the removal of desensitization by the L507Y mutation, residues other than tyrosine were introduced in this position (Figure 6). Of the 11 mutations tested, desensitization was blocked by three changes, to phenylalanine (F; P/S=1.08±0.11. n=7; Figure 6A&D), tryptophane (W; P/S=1.01±0.03, n=5; Figure 6D), and histidine (H; P/S=2.03±0.4, n=6; Figure 6D), all aromatic amino acids. The partial desensitization observed for mutation L507H may be due to the slightly smaller size of the imidazole ring rather than its protonation state since a similar behavior at different pH values was observed. Exchanges to the aliphatic alcohol side-chains serine (S; Figure 6B) and threonine (T; Figure 6C), resulted in fully desensitizing receptors, with a significant faster desensitization rate, R_{D}, for the L507T. A similar increase was also observed by the mutation to asparagine (N; Figure 6D). Finally, exchanges to the basic/positively charged lysine (K), acidic/negatively charged glutamate (E), or to the relative small side-chain valine (V) and glycine (G) had no apparent effect on desensitization when compared to GluR3_{flip} (Figure 6D).

### Example 8: Kainate Elicits Fast Desensitizing Currents at AMPA receptors.

A structural tie between agonist binding and desensitization could be the basis for the observation that AMPA receptor desensitization depends on the agonist used. Kainate applied to AMPA receptors, induces rapid, much weaker desensitizing responses, with considerably lower agonist efficacy than AMPA or glutamate (Patneau and Mayer, Neuron 6 (1991), 785-798; Patneau, 1993, loc. cit.). If kainate binding and activation induces conformations other than with glutamate (particular the one associated with desensitization), the degree of glutamate induced desensitization expressed by a receptor should not influence its kainate response. Responses evoked by saturating kainate concentrations (5-10 mM) from the GluR3-S1 chimeras (see Figures 2 and 3) were all essentially non-desensitizing. The efficacy of kainate was maximal for non-desensitizing receptors and was positively correlated (r=0.91) to the degree of inhibition of glutamate induced desensitization (Figure 7A). The fully desensitizing receptors exhibited a peak glutamate/kainate response ratio (G/K) of 53.2±5.3 (n=55), while for all non-desensitizing mutants G/K was 6.21±0.7 (n=66). The lack of apparent receptor desensitization, in contrast to native AMPA receptors (Patneau, 1993, loc. cit.), may result from desensitization kinetics that are considerably faster than those of activation, thus being either not measurable or overlooked. To test this idea advantage was taken of the 16-fold slower desensitizing receptor R6TM1 R3(Y521 L). Desensitizing responses to kainate were now apparent (Figure 7B, P/S=2.71±0.2; R_{D}=155±28 s⁻¹; n=6). Similar results were obtained from chimeras N1-N3 (Figure 3, n=21), indicating the validity of this aforementioned hypothesis. Kainate responses evoked on AMPA receptors appear rapidly desensitizing. Kainate as well as glutamate evoke the same kinetic changes in the different chimeric and/or mutant receptors of this invention. Therefore, the conformational changes that the receptor undergoes upon agonist binding occur regardless which agonist is employed. Differences in respect to the agonist may be the speed of the desensitization process, however.

### SEQUENCE LISTING

<110> Rosenmund, Christian Russo, Sebastian
<120> Non-desensitizing AMPA-Receptors
<130> D2234PCT
<140>
   <141>
<150> DE 198 47 064.9
   <151> 1998-10-13
<160> 22
<170> PatentIn Ver. 2.1
<210> 1
   <211> 907
   <212> PRT
   <213> Rattus norvegicus
<400> 1
   Met Pro Tyr Ile Phe Ala Phe Phe Cys Thr Gly Phe Leu Gly Ala Val
    1               5                  10                  15
   Val Gly Ala Asn Phe Pro Asn Asn Ile Gln Ile Gly Gly Leu Phe Pro
               20                  25                  30
   Asn Gln Gln Ser Gln Glu His Ala Ala Phe Arg Phe Ala Leu Ser Gln
           35                  40                  45
   Leu Thr Glu Pro Pro Lys Leu Leu Pro Gln Ile Asp Ile Val Asn Ile
       50                  55                  60
   Ser Asp Ser Phe Glu Met Thr Tyr Arg Phe Cys Ser Gln Phe Ser Lys
   65                  70                  75                  80
   Gly Val Tyr Ala Ile Phe Gly Phe Tyr Glu Arg Arg Thr Val Asn Met
                   85                  90                  95
   Leu Thr Ser Phe Cys Gly Ala Leu His Val Cys Phe Ile Thr Pro Ser
               100                 105                 110
   Phe Pro Val Asp Thr Ser Asn Gln Phe Val Leu Gln Leu Arg Pro Glu
           115                 120                 125
   Leu Gln Glu Ala Leu Ile Ser Ile Ile Asp His Tyr Lys Trp Gln Thr
       130                 135                 140
   Phe Val Tyr Ile Tyr Asp Ala Asp Arg Gly Leu Ser Val Leu Gln Arg
   145                 150                 155                 160
   Val Leu Asp Thr Ala Ala Glu Lys Asn Trp Gln Val Thr Ala Val Asn
                   165                 170                 175
   Ile Leu Thr Thr Thr Glu Glu Gly Tyr Arg Met Leu Phe Gln Asp Leu
               180                 185                 190
   Glu Lys Lys Lys Glu Arg Leu Val Val Val Asp Cys Glu Ser Glu Arg
           195                 200                 205
   Leu Asn Ala Ile Leu Gly Gln Ile Val Lys Leu Glu Lys Asn Gly Ile
       210                 215                 220
   Gly Tyr His Tyr Ile Leu Ala Asn Leu Gly Phe Met Asp Ile Asp Leu
   225                 230                 235                 240
   Asn Lys Phe Lys Glu Ser Gly Ala Asn Val Thr Gly Phe Gln Leu Val
                   245                 250                 255
   Asn Tyr Thr Asp Thr Ile Pro Ala Arg Ile Met Gln Gln Trp Arg Thr
               260                 265                 270
   Ser Asp Ser Arg Asp His Thr Arg Val Asp Trp Lys Arg Pro Lys Tyr
           275                 280                 285
   Thr Ser Ala Leu Thr Tyr Asp Gly Val Lys Val Met Ala Glu Ala Phe
       290                 295                 300
   Gln Ser Leu Arg Arg Gln Arg Ile Asp Ile Ser Arg Arg Gly Asn Ala
   305                 310                 315                 320
   Gly Asp Cys Leu Ala Asn Pro Ala Val Pro Trp Gly Gln Gly Ile Asp
                   325                 330                 335
   Ile Gln Arg Ala Leu Gln Gln Val Arg Phe Glu Gly Leu Thr Gly Asn
               340                 345                 350
   Val Gln Phe Asn Glu Lys Gly Arg Arg Thr Asn Tyr Thr Leu His Val
           355                 360                 365
   Ile Glu Met Lys His Asp Gly Ile Arg Lys Ile Gly Tyr Trp Asn Glu
       370                 375                 380
   Asp Asp Lys Phe Val Pro Ala Ala Thr Asp Ala Gln Ala Gly Gly Asp
   385                 390                 395                 400
   Asn Ser Ser Val Gln Asn Arg Thr Tyr Ile Val Thr Thr Ile Leu Glu
                   405                 410                 415
   Asp Pro Tyr Val Met Leu Lys Lys Asn Ala Asn Gln Phe Glu Gly Asn
               420                 425                 430
   Asp Arg Tyr Glu Gly Tyr Cys Val Glu Leu Ala Ala Glu Ile Ala Lys
           435                 440                 445
   His Val Gly Tyr Ser Tyr Arg Leu Glu Ile Val Ser Asp Gly Lys Tyr
       450                 455                 460
   Gly Ala Arg Asp Pro Asp Thr Lys Ala Trp Asn Gly Met Val Gly Glu
   465                 470                 475                 480
   Leu Val Tyr Gly Arg Ala Asp Val Ala Val Ala Pro Leu Thr Ile Thr
                   485                 490                 495
   Leu Val Arg Glu Glu Val Ile Asp Phe Ser Lys Pro Phe Met Ser Leu
               500                 505                 510
   Gly Ile Ser Ile Met Ile Lys Lys Pro Gln Lys Ser Lys Pro Gly Val
           515                 520                 525
   Phe Ser Phe Leu Asp Pro Leu Ala Tyr Glu Ile Trp Met Cys Ile Val
       530                 535                 540
   Phe Ala Tyr Ile Gly Val Ser Val Val Leu Phe Leu Val Ser Arg Phe
   545                 550                 555                 560
   Ser Pro Tyr Glu Trp His Ser Glu Glu Phe Glu Glu Gly Arg Asp Gln
                   565                 570                 575
   Thr Thr Ser Asp Gln Ser Asn Glu Phe Gly Ile Phe Asn Ser Leu Trp
               580                 585                 590
   Phe Ser Leu Gly Ala Phe Met Gln Gln Gly Cys Asp Ile Ser Pro Arg
           595                 600                 605
   Ser Leu Ser Gly Arg Ile Val Gly Gly Val Trp Trp Phe Phe Thr Leu
       610                 615                 620
   Ile Ile Ile Ser Ser Tyr Thr Ala Asn Leu Ala Ala Phe Leu Thr Val
   625                 630                 635                 640
   Glu Arg Met Val Ser Pro Ile Glu Ser Ala Glu Asp Leu Ala Lys Gln
                   645                 650                 655
   Thr Glu Ile Ala Tyr Gly Thr Leu Glu Ala Gly Ser Thr Lys Glu Phe
               660                 665                 670
   Phe Arg Arg Ser Lys Ile Ala Val Phe Glu Lys Met Trp Thr Tyr Met
           675                 680                 685
   Lys Ser Ala Glu Pro Ser Val Phe Val Arg Thr Thr Glu Glu Gly Met
       690                 695                 700
   Ile Arg Val Arg Lys Ser Lys Gly Lys Tyr Ala Tyr Leu Leu Glu Ser
   705                 710                 715                 720
   Thr Met Asn Glu Tyr Ile Glu Gln Arg Lys Pro Cys Asp Thr Met Lys
                   725                 730                 735
   Val Gly Gly Asn Leu Asp Ser Lys Gly Tyr Gly Ile Ala Thr Pro Lys
               740                 745                 750
   Gly Ser Ala Leu Arg Asn Pro Val Asn Leu Ala Val Leu Lys Leu Asn
           755                 760                 765
   Glu Gln Gly Leu Leu Asp Lys Leu Lys Asn Lys Trp Trp Tyr Asp Lys
       770                 775                 780
   Gly Glu Cys Gly Ser Gly Gly Gly Asp Ser Lys Asp Lys Thr Ser Ala
   785                 790                 795                 800
   Leu Ser Leu Ser Asn Val Ala Gly Val Phe Tyr Ile Leu Ile Gly Gly
                   805                 810                 815
   Leu Gly Leu Ala Met Leu Val Ala Leu Ile Glu Phe Cys Tyr Lys Ser
               820                 825                 830
   Arg Ser Glu Ser Lys Arg Met Lys Gly Phe Cys Leu Ile Pro Gln Gln
           835                     840                     845
   Ser Ile Asn Glu Ala Ile Arg Thr Ser Thr Leu Pro Arg Asn Ser Gly
       850                     855                     860
   Ala Gly Ala Ser Gly Gly Gly Gly Ser Gly Glu Asn Gly Arg Val Val
   865                     870                     875                     880
   Ser Gln Asp Phe Pro Lys Ser Met Gln Ser Ile Pro Cys Met Ser His
                   885                     890                     895
   Ser Ser Gly Met Pro Leu Gly Ala Thr Gly Leu
               900                     905
<210> 2
   <211> 883
   <212> PRT
   <213> Rattus norvegicus
<400> 2
   Met Gln Lys Ile Met His Ile Ser Val Leu Leu Ser Pro Val Leu Trp
    1               5                  10                  15
   Gly Leu Ile Phe Gly Val Ser Ser Asn Ser Ile Gln Ile Gly Gly Leu
               20                  25                  30
   Phe Pro Arg Gly Ala Asp Gln Glu Tyr Ser Ala Phe Arg Val Gly Met
           35                  40                  45
   Val Gln Phe Ser Thr Ser Glu Phe Arg Leu Thr Pro His Ile Asp Asn
       50                  55                  60
   Leu Glu Val Ala Asn Ser Phe Ala Val Thr Asn Ala Phe Cys Ser Gln
   65                  70                  75                  80
   Phe Ser Arg Gly Val Tyr Ala Ile Phe Gly Phe Tyr Asp Lys Lys Ser
                   85                  90                  95
   Val Asn Thr Ile Thr Ser Phe Cys Gly Thr Leu His Val Ser Phe Ile
               100                 105                 110
   Thr Pro Ser Phe Pro Thr Asp Gly Thr His Pro Phe Val Ile Gln Met
           115                 120                 125
   Arg Pro Asp Leu Lys Gly Ala Leu Leu Ser Leu Ile Glu Tyr Tyr Gln
       130                 135                 140
   Trp Asp Lys Phe Ala Tyr Leu Tyr Asp Ser Asp Arg Gly Leu Ser Thr
   145                 150                 155                 160
   Leu Gln Ala Val Leu Asp Ser Ala Ala Glu Lys Lys Trp Gln Val Thr
                   165                 170                 175
   Ala Ile Asn Val Gly Asn Ile Asn Asn Asp Lys Lys Asp Glu Thr Tyr
               180                 185                 190
   Arg Ser Leu Phe Gln Asp Leu Glu Leu Lys Lys Glu Arg Arg Val Ile
           195                 200                 205
   Leu Asp Cys Glu Arg Asp Lys Val Asn Asp Ile Val Asp Gln Val Ile
       210                 215                 220
   Thr Ile Gly Lys His Val Lys Gly Tyr His Tyr Ile Ile Ala Asn Leu
   225                 230                 235                 240
   Gly Phe Thr Asp Gly Asp Leu Leu Lys Ile Gln Phe Gly Gly Ala Asn
                   245                 250                 255
   Val Ser Gly Phe Gln Ile Val Asp Tyr Asp Asp Ser Leu Val Ser Lys
               260                 265                 270
   Phe Ile Glu Arg Trp Ser Thr Leu Glu Glu Lys Glu Tyr Pro Gly Ala
           275                 280                 285
   His Thr Ala Thr Ile Lys Tyr Thr Ser Ala Leu Thr Tyr Asp Ala Val
       290                 295                 300
   Gln Val Met Thr Glu Ala Phe Arg Asn Leu Arg Lys Gln Arg Ile Glu
   305                 310                 315                 320
   Ile Ser Arg Arg Gly Asn Ala Gly Asp Cys Leu Ala Asn Pro Ala Val
                   325                 330                 335
   Pro Trp Gly Gln Gly Val Glu Ile Glu Arg Ala Leu Lys Gln Val Gln
               340                 345                 350
   Val Glu Gly Leu Ser Gly Asn Ile Lys Phe Asp Gln Asn Gly Lys Arg
           355                 360                 365
   Ile Asn Tyr Thr Ile Asn Ile Met Glu Leu Lys Thr Asn Gly Pro Arg
       370                 375                 380
   Lys Ile Gly Tyr Trp Ser Glu Val Asp Lys Met Val Val Thr Leu Thr
   385                 390                 395                 400
   Glu Leu Pro Ser Gly Asn Asp Thr Ser Gly Leu Glu Asn Lys Thr Val
                   405                 410                 415
   Val Val Thr Thr Ile Leu Glu Ser Pro Tyr Val Met Met Lys Lys Asn
               420                 425                 430
   His Glu Met Leu Glu Gly Asn Glu Arg Tyr Glu Gly Tyr Cys Val Asp
           435                 440                 445
   Leu Ala Ala Glu Ile Ala Lys His Cys Gly Phe Lys Tyr Lys Leu Thr
       450                 455                 460
   Ile Val Gly Asp Gly Lys Tyr Gly Ala Arg Asp Ala Asp Thr Lys Ile
   465                 470                 475                 480
   Trp Asn Gly Met Val Gly Glu Leu Val Tyr Gly Lys Ala Asp Ile Ala
                   485                 490                 495
   Ile Ala Pro Leu Thr Ile Thr Leu Val Arg Glu Glu Val Ile Asp Phe
               500                 505                 510
   Ser Lys Pro Phe Met Ser Leu Gly Ile Ser Ile Met Ile Lys Lys Pro
   515 520 525
   Gln Lys Ser Lys Pro Gly Val Phe Ser Phe Leu Asp Pro Leu Ala Tyr
   530 535 540
   Glu Ile Trp Met Cys Ile Val Phe Ala Tyr Ile Gly Val Ser Val Val
   545 550 555 560
   Leu Phe Leu Val Ser Arg Phe Ser Pro Tyr Glu Trp His Thr Glu Glu
   565 570 575
   Phe Glu Asp Gly Arg Glu Thr Gln Ser Ser Glu Ser Thr Asn Glu Phe
   580 585 590
   Gly Ile Phe Asn Ser Leu Trp Phe Ser Leu Gly Ala Phe Met Arg Gln
   595 600 605
   Gly Cys Asp Ile Ser Pro Arg Ser Leu Ser Gly Arg Ile Val Gly Gly
   610 615 620
   Val Trp Trp Phe Phe Thr Leu Ile Ile Ile Ser Ser Tyr Thr Ala Asn
   625 630 635 640
   Leu Ala Ala Phe Leu Thr Val Glu Arg Met Val Ser Pro Ile Glu Ser
   645 650 655
   Ala Glu Asp Leu Ser Lys Gln Thr Glu Ile Ala Tyr Gly Thr Leu Asp
   660 665 670
   Ser Gly Ser Thr Lys Glu Phe Phe Arg Arg Ser Lys Ile Ala Val Phe
   675 680 685
   Asp Lys Met Trp Thr Tyr Met Arg Ser Ala Glu Pro Ser Val Phe Val
   690 695 700
   Arg Thr Thr Ala Glu Gly Val Ala Arg Val Arg Lys Ser Lys Gly Lys
   705 710 715 720
   Tyr Ala Tyr Leu Leu Glu Ser Thr Met Asn Glu Tyr Ile Glu Gln Arg
   725 730 735
   Lys Pro Cys Asp Thr Met Lys Val Gly Gly Asn Leu Asp Ser Lys Gly
   740 745 750
   Tyr Gly Ile Ala Thr Pro Lys Gly Ser Ser Leu Gly Asn Ala Val Asn
   755 760 765
   Leu Ala Val Leu Lys Leu Asn Glu Gln Gly Leu Leu Asp Lys Leu Lys
   770 775 780
   Asn Lys Trp Trp Tyr Asp Lys Gly Glu Cys Gly Ser Gly Gly Gly Asp
   785 790 795 800
   Ser Lys Glu Lys Thr Ser Ala Leu Ser Leu Ser Asn Val Ala Gly Val
   805 810 815
   Phe Tyr Ile Leu Val Gly Gly Leu Gly Leu Ala Met Leu Val Ala Leu
   820 825 830
   Ile Glu Phe Cys Tyr Lys Ser Arg Ala Glu Ala Lys Arg Met Lys Val
           835                 840                 845
   Ala Lys Asn Pro Gln Asn Ile Asn Pro Ser Ser Ser Gln Asn Ser Gln
       850                 855                 860
   Asn Phe Ala Thr Tyr Lys Glu Gly Tyr Asn Val Tyr Gly Ile Glu Ser
   865                 870                 875                 880
   Val Lys Ile
<210> 3
   <211> 888
   <212> PRT
   <213> Rattus norvegicus
<400> 3
   Met Gly Gln Ser Val Leu Arg Ala Val Phe Phe Leu Val Leu Gly Leu
    1               5                  10                  15
   Leu Gly His Ser His Gly Gly Phe Pro Asn Thr Ile Ser Ile Gly Gly
               20                  25                  30
   Leu Phe Met Arg Asn Thr Val Gln Glu His Ser Ala Phe Arg Phe Ala
           35                  40                  45
   Val Gln Leu Tyr Asn Thr Asn Gln Asn Thr Thr Glu Lys Pro Phe His
       50                  55                  60
   Leu Asn Tyr His Val Asp His Leu Asp Ser Ser Asn Ser Phe Ser Val
   65                  70                  75                  80
   Thr Asn Ala Phe Cys Ser Gln Phe Ser Arg Gly Val Tyr Ala Ile Phe
                   85                  90                  95
   Gly Phe Tyr Asp Gln Met Ser Met Asn Thr Leu Thr Ser Phe Cys Gly
               100                 105                 110
   Ala Leu His Thr Ser Phe Val Thr Pro Ser Phe Pro Thr Asp Ala Asp
           115                 120                 125
   Val Gln Phe Val Ile Gln Met Arg Pro Ala Leu Lys Gly Ala Ile Leu
       130                 135                 140
   Ser Leu Leu Ser Tyr Tyr Lys Trp Glu Lys Phe Val Tyr Leu Tyr Asp
   145                 150                 155                 160
   Thr Glu Arg Gly Phe Ser Val Leu Gln Ala Ile Met Glu Ala Ala Val
                   165                 170                 175
   Gln Asn Asn Trp Gln Val Thr Ala Arg Ser Val Gly Asn Ile Lys Asp
               180                 185                 190
   Val Gln Glu Phe Arg Arg Ile Ile Glu Glu Met Asp Arg Arg Gln Glu
           195                 200                 205
   Lys Arg Tyr Leu Ile Asp Cys Glu Val Glu Arg Ile Asn Thr Ile Leu
   210 215 220
   Glu Gln Val Val Ile Leu Gly Lys His Ser Arg Gly Tyr His Tyr Met
   225 230 235 240
   Leu Ala Asn Leu Gly Phe Thr Asp Ile Leu Leu Glu Arg Val Met His
   245 250 255
   Gly Gly Ala Asn Ile Thr Gly Phe Gln Ile Val Asn Asn Glu Asn Pro
   260 265 270
   Met Val Gln Gln Phe Ile Gln Arg Trp Val Arg Leu Asp Glu Arg Glu
   275 280 285
   Phe Pro Glu Ala Lys Asn Ala Pro Leu Lys Tyr Thr Ser Ala Leu Thr
   290 295 300
   His Asp Ala Ile Leu Val Ile Ala Glu Ala Phe Arg Tyr Leu Arg Arg
   305 310 315 320
   Gln Arg Val Asp Val Ser Arg Arg Gly Ser Ala Gly Asp Cys Leu Ala
   325 330 335
   Asn Pro Ala Val Pro Trp Ser Gln Gly Ile Asp Ile Glu Arg Ala Leu
   340 345 350
   Lys Met Val Gln Val Gln Gly Met Thr Gly Asn Ile Gln Phe Asp Thr
   355 360 365
   Tyr Gly Arg Arg Thr Asn Tyr Thr Ile Asp Val Tyr Glu Met Lys Val
   370 375 380
   Ser Gly Ser Arg Lys Ala Gly Tyr Trp Asn Glu Tyr Glu Arg Phe Val
   385 390 395 400
   Pro Phe Ser Asp Gln Gln Ile Ser Asn Asp Ser Ser Ser Ser Glu Asn
   405 410 415
   Arg Thr Ile Val Val Thr Thr Ile Leu Glu Ser Pro Tyr Val Met Tyr
   420 425 430
   Lys Lys Asn His Glu Gln Leu Glu Gly Asn Glu Arg Tyr Glu Gly Tyr
   435 440 445
   Cys Val Asp Leu Ala Tyr Glu Ile Ala Lys His Val Arg Ile Lys Tyr
   450 455 460
   Lys Leu Ser Ile Val Gly Asp Gly Lys Tyr Gly Ala Arg Asp Pro Glu
   465 470 475 480
   Thr Lys Ile Trp Asn Gly Met Val Gly Glu Leu Val Tyr Gly Arg Ala
   485 490 495
   Asp Ile Ala Val Ala Pro Leu Thr Ile Thr Leu Val Arg Glu Glu Val
   500 505 510
   Ile Asp Phe Ser Lys Pro Phe Met Ser Leu Gly Ile Ser Ile Met Ile
   515 520 525
   Lys Lys Pro Gln Lys Ser Lys Pro Gly Val Phe Ser Phe Leu Asp Pro
   530 535 540
   Leu Ala Tyr Glu Ile Trp Met Cys Ile Val Phe Ala Tyr Ile Gly Val
   545 550 555 560
   Ser Val Val Leu Phe Leu Val Ser Arg Phe Ser Pro Tyr Glu Trp His
   565 570 575
   Leu Glu Asp Asn Asn Glu Glu Pro Arg Asp Pro Gln Ser Pro Pro Asp
   580 585 590
   Pro Pro Asn Glu Phe Gly Ile Phe Asn Ser Leu Trp Phe Ser Leu Gly
   595 600 605
   Ala Phe Met Gln Gln Gly Cys Asp Ile Ser Pro Arg Ser Leu Ser Gly
   610 615 620
   Arg Ile Val Gly Gly Val Trp Trp Phe Phe Thr Leu Ile Ile Ile Ser
   625 630 635 640
   Ser Tyr Thr Ala Asn Leu Ala Ala Phe Leu Thr Val Glu Arg Met Val
   645 650 655
   Ser Pro Ile Glu Ser Ala Glu Asp Leu Ala Lys Gln Thr Glu Ile Ala
   660 665 670
   Tyr Gly Thr Leu Asp Ser Gly Ser Thr Lys Glu Phe Phe Arg Arg Ser
   675 680 685
   Lys Ile Ala Val Tyr Glu Lys Met Trp Ser Tyr Met Lys Ser Ala Glu
   690 695 700
   Pro Ser Val Phe Thr Lys Thr Thr Ala Asp Gly Val Ala Arg Val Arg
   705 710 715 720
   Lys Ser Lys Gly Lys Phe Ala Phe Leu Leu Glu Ser Thr Met Asn Glu
   725 730 735
   Tyr Ile Glu Gln Arg Lys Pro Cys Asp Thr Met Lys Val Gly Gly Asn
   740 745 750
   Leu Asp Ser Lys Gly Tyr Gly Val Ala Thr Pro Lys Gly Ser Ala Leu
   755 760 765
   Gly Asn Ala Val Asn Leu Ala Val Leu Lys Leu Asn Glu Gln Gly Leu
   770 775 780
   Leu Asp Lys Leu Lys Asn Lys Trp Trp Tyr Asp Lys Gly Glu Cys Gly
   785 790 795 800
   Ser Gly Gly Gly Asp Ser Lys Asp Lys Thr Ser Ala Leu Ser Leu Ser
   805 810 815
   Asn Val Ala Gly Val Phe Tyr Ile Leu Val Gly Gly Leu Gly Leu Ala
   820 825 830
   Met Met Val Ala Leu Ile Glu Phe Cys Tyr Lys Ser Arg Ala Glu Ser
   835 840 845
   Lys Arg Met Lys Leu Thr Lys Asn Thr Gln Asn Phe Lys Pro Ala Pro
   850 855 860
   Ala Thr Asn Thr Gln Asn Tyr Ala Thr Tyr Arg Glu Gly Tyr Asn Val
   865 870 875 880
   Tyr Gly Thr Glu Ser Val Lys Ile
   885
<210> 4
   <211> 902
   <212> PRT
   <213> Rattus norvegicus
<400> 4
   Met Arg Ile Ile Cys Arg Gln Ile Val Leu Leu Phe Ser Gly Phe Trp
    1               5                  10                  15
   Gly Leu Ala Met Gly Ala Phe Pro Ser Ser Val Gln Ile Gly Gly Leu
               20                  25                  30
   Phe Ile Arg Asn Thr Asp Gln Glu Tyr Thr Ala Phe Arg Leu Ala Ile
           35                  40                  45
   Phe Leu His Asn Thr Ser Pro Asn Ala Ser Glu Ala Pro Phe Asn Leu
       50                  55                  60
   Val Pro His Val Asp Asn Ile Glu Thr Ala Asn Ser Phe Ala Val Thr
   65                  70                  75                  80
   Asn Ala Phe Cys Ser Gln Tyr Ser Arg Gly Val Phe Ala Ile Phe Gly
                   85                  90                  95
   Leu Tyr Asp Lys Arg Ser Val His Thr Leu Thr Ser Phe Cys Arg Arg
               100                 105                 110
   Leu His Ile Ser Leu Ile Thr Pro Ser Phe Pro Thr Glu Gly Glu Ser
           115                 120                 125
   Gln Phe Val Leu Gln Leu Arg Pro Ser Leu Arg Gly Ala Leu Leu Ser
       130                 135                 140
   Leu Leu Asp His Tyr Glu Trp Asn Cys Phe Val Phe Leu Tyr Asp Thr
   145                 150                 155                 160
   Asp Arg Gly Tyr Ser Ile Leu Gln Ala Ile Met Glu Lys Ala Gly Gln
                   165                 170                 175
   Asn Gly Trp His Val Ser Ala Ile Cys Val Glu Asn Phe Asn Asp Val
               180                 185                 190
   Ser Tyr Arg Gln Leu Leu Glu Glu Leu Asp Arg Arg Gln Glu Lys Lys
           195                 200                 205
   Phe Val Ile Asp Cys Glu Ile Glu Arg Leu Gln Asn Ile Leu Glu Gln
       210                 215                 220
   Ile Val Ser Val Gly Lys His Val Lys Gly Tyr His Tyr Ile Ile Ala
   225                 230                 235                 240
   Asn Leu Gly Phe Lys Asp Ile Ser Leu Glu Arg Phe Ile His Gly Gly
                   245                 250                 255
   Ala Asn Val Thr Gly Phe Gln Leu Val Asp Phe Asn Thr Pro Met Val
               260                 265                 270
   Thr Lys Leu Met Asp Arg Trp Lys Lys Leu Asp Gln Arg Glu Tyr Pro
           275                 280                 285
   Gly Ser Glu Thr Pro Pro Lys Tyr Thr Ser Ala Leu Thr Tyr Asp Gly
       290                 295                 300
   Val Leu Val Met Ala Glu Thr Phe Arg Ser Leu Arg Arg Gln Lys Ile
   305                 310                 315                 320
   Asp Ile Ser Arg Arg Gly Asn Ala Gly Asp Cys Leu Ala Asn Pro Ala
                   325                 330                 335
   Ala Pro Trp Gly Gln Gly Ile Asp Met Glu Arg Thr Leu Lys Gln Val
               340                 345                 350
   Arg Ile Gln Gly Leu Thr Gly Asn Val Gln Phe Asp His Tyr Gly Arg
           355                 360                 365
   Arg Val Asn Tyr Thr Met Asp Val Phe Glu Leu Lys Ser Thr Gly Pro
       370                 375                 380
   Arg Lys Val Gly Tyr Trp Asn Asp Met Asp Lys Leu Val Leu Ile Gln
   385                 390                 395                 400
   Asp Met Pro Thr Leu Gly Asn Asp Thr Ala Ala Ile Glu Asn Arg Thr
                   405                 410                 415
   Val Val Val Thr Thr Ile Met Glu Ser Pro Tyr Val Met Tyr Lys Lys
               420                 425                 430
   Asn His Glu Met Phe Glu Gly Asn Asp Lys Tyr Glu Gly Tyr Cys Val
           435                 440                 445
   Asp Leu Ala Ser Glu Ser Ala Lys His Ile Gly Ile Lys Tyr Lys Ile
       450                 455                 460
   Ala Ile Val Pro Asp Gly Lys Tyr Gly Ala Arg Asp Ala Asp Thr Lys
   465                 470                 475                 480
   Ile Trp Asn Gly Met Val Gly Glu Leu Val Tyr Gly Lys Ala Glu Ile
                   485                 490                 495
   Ala Ile Ala Pro Leu Thr Ile Thr Leu Val Arg Glu Glu Val Ile Asp
               500                 505                 510
   Phe Ser Lys Pro Phe Met Ser Leu Gly Ile Ser Ile Met Ile Lys Lys
           515                 520                 525
   Pro Gln Lys Ser Lys Pro Gly Val Phe Ser Phe Leu Asp Pro Leu Ala
       530                 535                 540
   Tyr Glu Ile Trp Met Cys Ile Val Phe Ala Tyr Ile Gly Val Ser Val
   545                 550                 555                 560
   Val Leu Phe Leu Val Ser Arg Phe Ser Pro Tyr Glu Trp His Thr Glu
                   565                 570                 575
   Glu Pro Glu Asp Gly Lys Glu Gly Pro Ser Asp Gln Pro Pro Asn Glu
               580                 585                 590
   Phe Gly Ile Phe Asn Ser Leu Trp Phe Ser Leu Gly Ala Phe Met Gln
           595                 600                 605
   Gln Gly Cys Asp Ile Ser Pro Arg Ser Leu Ser Gly Arg Ile Val Gly
       610                 615                 620
   Gly Val Trp Trp Phe Phe Thr Leu Ile Ile Ile Ser Ser Tyr Thr Ala
   625                 630                 635                 640
   Asn Leu Ala Ala Phe Leu Thr Val Glu Arg Met Val Ser Pro Ile Glu
                   645                 650                 655
   Ser Ala Glu Asp Leu Ala Lys Gln Thr Glu Ile Ala Tyr Gly Thr Leu
               660                 665                 670
   Asp Ser Gly Ser Thr Lys Glu Phe Phe Arg Arg Ser Lys Ile Ala Val
           675                 680                 685
   Tyr Glu Lys Met Trp Thr Tyr Met Arg Ser Ala Glu Pro Ser Val Phe
       690                 695                 700
   Thr Arg Thr Thr Ala Glu Gly Val Ala Arg Val Arg Lys Ser Lys Gly
   705                 710                 715                 720
   Lys Phe Ala Phe Leu Leu Glu Ser Thr Met Asn Glu Tyr Ile Glu Gln
                   725                 730                 735
   Arg Lys Pro Cys Asp Thr Met Lys Val Gly Gly Asn Leu Asp Ser Lys
               740                 745                 750
   Gly Tyr Gly Val Ala Thr Pro Lys Gly Ser Ser Leu Arg Thr Pro Val
           755                 760                 765
   Asn Leu Ala Val Leu Lys Leu Ser Glu Ala Gly Val Leu Asp Lys Leu
       770                 775                 780
   Lys Asn Lys Trp Trp Tyr Asp Lys Gly Glu Cys Gly Pro Lys Asp Ser
   785                 790                 795                 800
   Gly Ser Lys Asp Lys Thr Ser Ala Leu Ser Leu Ser Asn Val Ala Gly
                   805                 810                 815
   Val Phe Tyr Ile Leu Val Gly Gly Leu Gly Leu Ala Met Leu Val Ala
               820                 825                 830
   Leu Ile Glu Phe Cys Tyr Lys Ser Arg Ala Glu Ala Lys Arg Met Lys
           835                 840                 845
   Leu Thr Phe Ser Glu Ala Ile Arg Asn Lys Ala Arg Leu Ser Ile Thr
       850                 855                 860
   Gly Ser Val Gly Glu Asn Gly Arg Val Leu Thr Pro Asp Cys Pro Lys
   865                 870                 875                 880
   Ala Val His Thr Gly Thr Ala Ile Arg Gln Ser Ser Gly Leu Ala Val
   885 890 895
   Ile Ala Ser Asp Leu Pro
   900
<210> 5
   <211> 1043
   <212> PRT
   <213> Homo sapiens
<400> 5
<210> 6
   <211> 883
   <212> PRT
   <213> Homo sapiens
<400> 6
   Met Gln Lys Ile Met His Val Ser Val Leu Leu Ser Pro Val Leu Trp
    1               5                  10                  15
   Gly Leu Ile Phe Gly Val Ser Ser Asn Ser Ile Gln Ile Gly Gly Leu
               20                  25                  30
   Phe Pro Arg Gly Ala Asp Gln Glu Tyr Ser Ala Phe Arg Val Gly Met
           35                  40                  45
   Val Gln Phe Ser Thr Ser Glu Phe Arg Leu Thr Pro His Ile Asp Asn
       50                  55                  60
   Leu Glu Val Ala Asn Ser Phe Ala Val Thr Asn Ala Phe Cys Ser Gln
   65                  70                  75                  80
   Phe Ser Arg Gly Val Tyr Ala Ile Phe Gly Phe Tyr Asp Lys Lys Ser
                   85                  90                  95
   Val Asn Thr Ile Thr Ser Phe Cys Gly Thr Leu His Val Ser Phe Ile
   100 105 110
   Thr Pro Ser Phe Pro Thr Asp Gly Thr His Pro Phe Val Ile Gln Met
   115 120 125
   Arg Pro Asp Leu Lys Gly Ala Leu Leu Ser Leu Ile Glu Tyr Tyr Gln
   130 135 140
   Trp Asp Lys Phe Ala Tyr Leu Tyr Asp Ser Asp Arg Gly Leu Ser Thr
   145 150 155 160
   Leu Gln Ala Val Leu Asp Ser Ala Ala Glu Lys Lys Trp Gln Val Thr
   165 170 175
   Ala Ile Asn Val Gly Asn Ile Asn Asn Asp Lys Lys Asp Glu Met Tyr
   180 185 190
   Arg Ser Leu Phe Gln Asp Leu Glu Leu Lys Lys Glu Arg Arg Val Ile
   195 200 205
   Leu Asp Cys Glu Arg Asp Lys Val Asn Asp Ile Val Asp Gln Val Ile
   210 215 220
   Thr Ile Gly Lys His Val Lys Gly Tyr His Tyr Ile Ile Ala Asn Leu
   225 230 235 240
   Gly Phe Thr Asp Gly Asp Leu Leu Lys Ile Gln Phe Gly Gly Ala Asn
   245 250 255
   Val Ser Gly Phe Gln Ile Val Asp Tyr Asp Asp Ser Leu Val Ser Lys
   260 265 270
   Phe Ile Glu Arg Trp Ser Thr Leu Glu Glu Lys Glu Tyr Pro Gly Ala
   275 280 285
   His Thr Thr Thr Ile Lys Tyr Thr Ser Ala Leu Thr Tyr Asp Ala Val
   290 295 300
   Gln Val Met Thr Glu Ala Phe Arg Asn Leu Arg Lys Gln Arg Ile Glu
   305 310 315 320
   Ile Ser Arg Arg Gly Asn Ala Gly Asp Cys Leu Ala Asn Pro Ala Val
   325 330 335
   Pro Trp Gly Gln Gly Val Glu Ile Glu Arg Ala Leu Lys Gln Val Gln
   340 345 350
   Val Glu Gly Leu Ser Gly Asn Ile Lys Phe Asp Gln Asn Gly Lys Arg
   355 360 365
   Ile Asn Tyr Thr Ile Asn Ile Met Glu Leu Lys Thr Asn Gly Pro Arg
   370 375 380
   Lys Ile Gly Tyr Trp Ser Glu Val Asp Lys Met Val Val Thr Leu Thr
   385 390 395 400
   Glu Leu Pro Ser Gly Asn Asp Thr Ser Gly Leu Glu Asn Lys Thr Val
   405 410 415
   Val Val Thr Thr Ile Leu Glu Ser Pro Tyr Val Met Met Lys Lys Asn
               420                 425                 430
   His Glu Met Leu Glu Gly Asn Glu Arg Tyr Glu Gly Tyr Cys Val Asp
           435                 440                 445
   Leu Ala Ala Glu Ile Ala Lys His Cys Gly Phe Lys Tyr Lys Leu Thr
       450                 455                 460
   Ile Val Gly Asp Gly Lys Tyr Gly Ala Arg Asp Ala Asp Thr Lys Ile
   465                 470                 475                 480
   Trp Asn Gly Met Val Gly Glu Leu Val Tyr Gly Lys Ala Asp Ile Ala
                   485                 490                 495
   Ile Ala Pro Leu Thr Ile Thr Leu Val Arg Glu Glu Val Ile Asp Phe
               500                 505                 510
   Ser Lys Pro Phe Met Ser Leu Gly Ile Ser Ile Met Ile Lys Lys Pro
           515                 520                 525
   Gln Lys Ser Lys Pro Gly Val Phe Ser Phe Leu Asp Pro Leu Ala Tyr
       530                 535                 540
   Glu Ile Trp Met Cys Ile Val Phe Ala Tyr Ile Gly Val Ser Val Val
   545                 550                 555                 560
   Leu Phe Leu Val Ser Arg Phe Ser Pro Tyr Glu Trp His Thr Glu Glu
                   565                 570                 575
   Phe Glu Asp Gly Arg Glu Thr Gln Ser Ser Glu Ser Thr Asn Glu Phe
               580                 585                 590
   Gly Ile Phe Asn Ser Leu Trp Phe Ser Leu Gly Ala Phe Met Arg Gln
           595                 600                 605
   Gly Cys Asp Ile Ser Pro Arg Ser Leu Ser Gly Arg Ile Val Gly Gly
       610                 615                 620
   Val Trp Trp Phe Phe Thr Leu Ile Ile Ile Ser Ser Tyr Thr Ala Asn
   625                 630                 635                 640
   Leu Ala Ala Phe Leu Thr Val Glu Arg Met Val Ser Pro Ile Glu Ser
                   645                 650                 655
   Ala Glu Asp Leu Ser Lys Gln Thr Glu Ile Ala Tyr Gly Thr Leu Asp
               660                 665                 670
   Ser Gly Ser Thr Lys Glu Phe Phe Arg Arg Ser Lys Ile Ala Val Phe
           675                 680                 685
   Asp Lys Met Trp Thr Tyr Met Arg Ser Ala Glu Pro Ser Val Phe Val
       690                 695                 700
   Arg Thr Thr Ala Glu Gly Val Ala Arg Val Arg Lys Ser Lys Gly Lys
   705                 710                 715                 720
   Tyr Ala Tyr Leu Leu Glu Ser Thr Met Asn Glu Tyr Ile Glu Gln Arg
                   725                 730                 735
   Lys Pro Cys Asp Thr Met Lys Val Gly Gly Asn Leu Asp Ser Lys Gly
   740 745 750
   Tyr Gly Ile Ala Thr Pro Lys Gly Ser Ser Leu Arg Asn Ala Val Asn
   755 760 765
   Leu Ala Val Leu Lys Leu Asn Glu Gln Gly Leu Leu Asp Lys Leu Lys
   770 775 780
   Asn Lys Trp Trp Tyr Asp Lys Gly Glu Cys Gly Ser Gly Gly Gly Asp
   785 790 795 800
   Ser Lys Glu Lys Thr Ser Ala Leu Ser Leu Ser Asn Val Ala Gly Val
   805 810 815
   Phe Tyr Ile Leu Val Gly Gly Leu Gly Leu Ala Met Leu Val Ala Leu
   820 825 830
   Ile Glu Phe Cys Tyr Lys Ser Arg Ala Glu Ala Lys Arg Met Lys Val
   835 840 845
   Ala Lys Asn Ala Gln Asn Ile Asn Pro Ser Ser Ser Gln Asn Ser Gln
   850 855 860
   Asn Phe Ala Thr Tyr Lys Glu Gly Tyr Asn Val Tyr Gly Ile Glu Ser
   865 870 875 880
   Val Lys Ile
<210> 7
   <211> 894
   <212> PRT
   <213> Homo sapiens
<400> 7
   Met Ala Arg Gln Lys Lys Met Gly Gln Ser Val Leu Arg Ala Val Phe
    1               5                  10                  15
   Phe Leu Val Leu Gly Leu Leu Gly His Ser His Gly Gly Phe Pro Asn
               20                  25                  30
   Thr Ile Ser Ile Gly Gly Leu Phe Met Arg Asn Thr Val Gln Glu His
           35                  40                  45
   Ser Ala Phe Arg Phe Ala Val Gln Leu Tyr Asn Thr Asn Gln Asn Thr
       50                  55                  60
   Thr Glu Lys Pro Phe His Leu Asn Tyr His Val Asp His Leu Asp Ser
   65                  70                  75                  80
   Ser Asn Ser Phe Ser Val Thr Asn Ala Phe Cys Ser Gln Phe Ser Arg
                   85                  90                  95
   Gly Val Tyr Ala Ile Phe Gly Phe Tyr Asp Gln Met Ser Met Asn Thr
               100                 105                 110
   Leu Thr Ser Phe Cys Gly Ala Leu His Thr Ser Phe Val Thr Pro Ser
           115                 120                 125
   Phe Pro Thr Asp Ala Asp Val Gln Phe Val Ile Gln Met Arg Pro Ala
       130                 135                 140
   Leu Lys Gly Ala Ile Leu Ser Leu Leu Gly His Tyr Lys Trp Glu Lys
   145                 150                 155                 160
   Phe Val Tyr Leu Tyr Asp Thr Glu Arg Gly Phe Ser Ile Leu Gln Ala
                   165                 170                 175
   Ile Met Glu Ala Ala Val Gln Asn Asn Trp Gln Val Thr Ala Arg Ser
               180                 185                 190
   Val Gly Asn Ile Lys Asp Val Gln Glu Phe Arg Arg Ile Ile Glu Glu
           195                 200                 205
   Met Asp Arg Arg Gln Glu Lys Arg Tyr Leu Ile Asp Cys Glu Val Glu
       210                 215                 220
   Arg Ile Asn Thr Ile Leu Glu Gln Val Val Ile Leu Gly Lys His Ser
   225                 230                 235                 240
   Arg Gly Tyr His Tyr Met Leu Ala Asn Leu Gly Phe Thr Asp Ile Leu
                   245                 250                 255
   Leu Glu Arg Val Met His Gly Gly Ala Asn Ile Thr Gly Phe Gln Ile
               260                 265                 270
   Val Asn Asn Glu Asn Pro Met Val Gln Gln Phe Ile Gln Arg Trp Val
           275                 280                 285
   Arg Leu Asp Glu Arg Glu Phe Pro Glu Ala Lys Asn Ala Pro Leu Lys
       290                 295                 300
   Tyr Thr Ser Ala Leu Thr His Asp Ala Ile Leu Val Ile Ala Glu Ala
   305                 310                 315                 320
   Phe Arg Tyr Leu Arg Arg Gln Arg Val Asp Val Ser Arg Arg Gly Ser
                   325                 330                 335
   Ala Gly Asp Cys Leu Ala Asn Pro Ala Val Pro Trp Ser Gln Gly Ile
               340                 345                 350
   Asp Ile Glu Arg Ala Leu Lys Met Val Gln Val Gln Gly Met Thr Gly
           355                 360                 365
   Asn Ile Gln Phe Asp Thr Tyr Gly Arg Arg Thr Asn Tyr Thr Ile Asp
       370                 375                 380
   Val Tyr Glu Met Lys Val Ser Gly Ser Arg Lys Ala Gly Tyr Trp Asn
   385                 390                 395                 400
   Glu Tyr Glu Arg Phe Val Pro Phe Ser Asp Gln Gln Ile Ser Asn Asp
                   405                 410                 415
   Ser Ala Ser Ser Glu Asn Arg Thr Ile Val Val Thr Thr Ile Leu Glu
               420                 425                 430
   Ser Pro Tyr Val Met Tyr Lys Lys Asn His Glu Gln Leu Glu Gly Asn
           435                 440                 445
   Glu Arg Tyr Glu Gly Tyr Cys Val Asp Leu Ala Tyr Glu Ile Ala Lys
   450 455 460
   His Val Arg Ile Lys Tyr Lys Leu Ser Ile Val Gly Asp Gly Lys Tyr
   465 470 475 480
   Gly Ala Arg Asp Pro Glu Thr Lys Ile Trp Asn Gly Met Val Gly Glu
   485 490 495
   Leu Val Tyr Gly Arg Ala Asp Ile Ala Val Ala Pro Leu Thr Ile Thr
   500 505 510
   Leu Val Arg Glu Glu Val Ile Asp Phe Ser Lys Pro Phe Met Ser Leu
   515 520 525
   Gly Ile Ser Ile Met Ile Lys Lys Pro Gln Lys Ser Lys Pro Gly Val
   530 535 540
   Phe Ser Phe Leu Asp Pro Leu Ala Tyr Glu Ile Trp Met Cys Ile Val
   545 550 555 560
   Phe Ala Tyr Ile Gly Val Ser Val Val Leu Phe Leu Val Ser Arg Phe
   565 570 575
   Ser Pro Tyr Glu Trp His Leu Glu Asp Asn Asn Glu Glu Pro Arg Asp
   580 585 590
   Pro Gln Ser Pro Pro Asp Pro Pro Asn Glu Phe Gly Ile Phe Asn Ser
   595 600 605
   Leu Trp Phe Ser Leu Gly Ala Phe Met Gln Gln Gly Cys Asp Ile Ser
   610 615 620
   Pro Arg Ser Leu Ser Gly Arg Ile Val Gly Gly Val Trp Trp Phe Phe
   625 630 635 640
   Thr Leu Ile Ile Ile Ser Ser Tyr Thr Ala Asn Leu Ala Ala Phe Leu
   645 650 655
   Thr Val Glu Arg Met Val Ser Pro Ile Glu Ser Ala Glu Asp Leu Ala
   660 665 670
   Lys Gln Thr Glu Ile Ala Tyr Gly Thr Leu Asp Ser Gly Ser Thr Lys
   675 680 685
   Glu Phe Phe Arg Arg Ser Lys Ile Ala Val Tyr Glu Lys Met Trp Ser
   690 695 700
   Tyr Met Lys Ser Ala Glu Pro Ser Val Phe Thr Lys Thr Thr Ala Asp
   705 710 715 720
   Gly Val Ala Arg Val Arg Lys Ser Lys Gly Lys Phe Ala Phe Leu Leu
   725 730 735
   Glu Ser Thr Met Asn Glu Tyr Ile Glu Gln Arg Lys Pro Cys Asp Thr
   740 745 750
   Met Lys Val Gly Gly Asn Leu Asp Ser Lys Gly Tyr Gly Val Ala Thr
   755 760 765
   Pro Lys Gly Ser Ala Leu Gly Asn Ala Val Asn Leu Ala Val Leu Lys
       770                 775                 780
   Leu Asn Glu Gln Gly Leu Leu Asp Lys Leu Lys Asn Lys Trp Trp Tyr
   785                 790                 795                 800
   Asp Lys Gly Glu Cys Gly Ser Gly Gly Gly Asp Ser Lys Asp Lys Thr
                   805                 810                 815
   Ser Ala Leu Ser Leu Ser Asn Val Ala Gly Val Phe Tyr Ile Leu Val
               820                 825                 830
   Gly Gly Leu Gly Leu Ala Met Met Val Ala Leu Ile Glu Phe Cys Tyr
           835                 840                 845
   Lys Ser Arg Ala Glu Ser Lys Arg Met Lys Leu Thr Lys Asn Thr Gln
       850                 855                 860
   Asn Phe Lys Pro Ala Pro Ala Thr Asn Thr Gln Asn Tyr Ala Thr Tyr
   865                 870                 875                 880
   Arg Glu Gly Tyr Asn Val Tyr Gly Thr Glu Ser Val Lys Ile
                   885                 890
<210> 8
   <211> 902
   <212> PRT
   <213> Homo sapiens
<400> 8
   Met Arg Ile Ile Ser Arg Gln Ile Val Leu Leu Phe Ser Gly Phe Trp
    1               5                  10                  15
   Gly Leu Ala Met Gly Ala Phe Pro Ser Ser Val Gln Ile Gly Gly Leu
               20                  25                  30
   Phe Ile Arg Asn Thr Asp Gln Glu Tyr Thr Ala Phe Arg Leu Ala Ile
           35                  40                  45
   Phe Leu His Asn Thr Ala Pro Asn Ala Ser Glu Ala Pro Phe Asn Leu
       50                  55                  60
   Val Pro His Val Asp Asn Ile Glu Thr Ala Asn Ser Phe Ala Val Thr
   65                  70                  75                  80
   Asn Ala Phe Cys Ser Gln Tyr Ser Arg Gly Val Phe Ala Ile Phe Gly
                   85                  90                  95
   Leu Tyr Asp Lys Arg Ser Val His Thr Leu Thr Ser Phe Cys Ser Ala
               100                 105                 110
   Leu His Ile Ser Leu Ile Thr Pro Ser Phe Pro Thr Glu Gly Glu Ser
           115                 120                 125
   Gln Phe Val Leu Gln Leu Arg Pro Ser Leu Arg Gly Ala Leu Leu Ser
       130                 135                 140
   Leu Leu Asp His Tyr Glu Trp Asn Cys Phe Val Phe Leu Tyr Asp Thr
   145 150 155 160
   Asp Arg Gly Tyr Ser Ile Leu Gln Ala Ile Met Glu Lys Ala Gly Gln
   165 170 175
   Asn Gly Trp His Val Ser Ala Ile Cys Val Glu Asn Phe Asn Asp Val
   180 185 190
   Ser Tyr Arg Gln Leu Leu Glu Glu Leu Asp Arg Arg Gln Glu Lys Lys
   195 200 205
   Phe Val Ile Asp Cys Glu Ile Glu Arg Leu Gln Asn Ile Leu Glu Gln
   210 215 220
   Ile Val Ser Val Gly Lys His Val Lys Gly Tyr His Tyr Ile Ile Ala
   225 230 235 240
   Asn Leu Gly Phe Lys Asp Ile Ser Leu Glu Arg Phe Ile His Gly Gly
   245 250 255
   Ala Asn Val Thr Gly Phe Gln Leu Val Asp Phe Asn Thr Pro Met Val
   260 265 270
   Thr Lys Leu Met Asp Arg Trp Lys Lys Leu Asp Gln Arg Glu Tyr Pro
   275 280 285
   Gly Ser Glu Thr Pro Pro Lys Tyr Thr Ser Ala Leu Thr Tyr Asp Gly
   290 295 300
   Val Leu Val Met Ala Glu Thr Phe Arg Ser Leu Arg Arg Gln Lys Ile
   305 310 315 320
   Asp Ile Ser Arg Arg Gly Lys Ser Gly Asp Cys Leu Ala Asn Pro Ala
   325 330 335
   Ala Pro Trp Gly Gln Gly Ile Asp Met Glu Arg Thr Leu Lys Gln Val
   340 345 350
   Arg Ile Gln Gly Leu Thr Gly Asn Val Gln Phe Asp His Tyr Gly Arg
   355 360 365
   Arg Val Asn Tyr Thr Met Asp Val Phe Glu Leu Lys Ser Thr Gly Pro
   370 375 380
   Arg Lys Val Gly Tyr Trp Asn Asp Met Asp Lys Leu Val Leu Ile Gln
   385 390 395 400
   Asp Val Pro Thr Leu Gly Asn Asp Thr Ala Ala Ile Glu Asn Arg Thr
   405 410 415
   Val Val Val Thr Thr Ile Met Glu Ser Pro Tyr Val Met Tyr Lys Lys
   420 425 430
   Asn His Glu Met Phe Glu Gly Asn Asp Lys Tyr Glu Gly Tyr Cys Val
   435 440 445
   Asp Leu Ala Ser Glu Ile Ala Lys His Ile Gly Ile Lys Tyr Lys Ile
   450 455 460
   Ala Ile Val Pro Asp Gly Lys Tyr Gly Ala Arg Asp Ala Asp Thr Lys
   465                 470                 475                 480
   Ile Trp Asn Gly Met Val Gly Glu Leu Val Tyr Gly Lys Ala Glu Ile
                   485                 490                 495
   Ala Ile Ala Pro Leu Thr Ile Thr Leu Val Arg Glu Glu Val Ile Asp
               500                 505                 510
   Phe Ser Lys Pro Phe Met Ser Leu Gly Ile Ser Ile Met Ile Lys Lys
           515                 520                 525
   Pro Gln Lys Ser Lys Pro Gly Val Phe Ser Phe Leu Asp Pro Leu Ala
       530                 535                 540
   Tyr Glu Ile Trp Met Cys Ile Val Phe Ala Tyr Ile Gly Val Ser Val
   545                 550                 555                 560
   Val Leu Phe Leu Val Ser Arg Phe Ser Pro Tyr Glu Trp His Thr Glu
                   565                 570                 575
   Glu Pro Glu Asp Gly Lys Glu Gly Pro Ser Asp Gln Pro Pro Asn Glu
               580                 585                 590
   Phe Gly Ile Phe Asn Ser Leu Trp Phe Ser Leu Gly Ala Phe Met Gln
           595                 600                 605
   Gln Gly Cys Asp Ile Ser Pro Arg Ser Leu Ser Gly Arg Ile Val Gly
       610                 615                 620
   Gly Val Trp Trp Phe Phe Thr Leu Ile Ile Ile Ser Ser Tyr Thr Ala
   625                 630                 635                 640
   Asn Leu Ala Ala Phe Leu Thr Val Glu Arg Met Val Ser Pro Ile Glu
                   645                 650                 655
   Ser Ala Glu Asp Leu Ala Lys Gln Thr Glu Ile Ala Tyr Gly Thr Leu
               660                 665                 670
   Asp Ser Gly Ser Thr Lys Glu Phe Phe Arg Arg Ser Lys Ile Ala Val
           675                 680                 685
   Tyr Glu Lys Met Trp Thr Tyr Met Arg Ser Ala Glu Pro Ser Val Phe
       690                 695                 700
   Thr Arg Thr Thr Ala Glu Gly Val Ala Arg Val Arg Lys Ser Lys Gly
   705                 710                 715                 720
   Lys Phe Ala Phe Leu Leu Glu Ser Thr Met Asn Asp Asn Ile Glu Gln
                   725                 730                 735
   Arg Lys Pro Cys Asp Thr Met Lys Val Gly Gly Asn Leu Asp Ser Lys
               740                 745                 750
   Gly Tyr Gly Val Ala Thr Pro Lys Gly Ser Ser Leu Arg Thr Pro Val
           755                 760                 765
   Asn Leu Ala Val Leu Lys Leu Ser Glu Ala Gly Val Leu Asp Lys Leu
       770                 775                 780
   Lys Asn Lys Trp Trp Tyr Asp Lys Gly Glu Cys Gly Pro Lys Asp Ser
   785                 790                 795                 800
   Gly Ser Lys Asp Lys Thr Ser Ala Leu Ser Leu Ser Asn Val Ala Gly
                   805                 810                 815
   Val Phe Tyr Ile Leu Val Gly Gly Leu Gly Leu Ala Met Leu Val Ala
               820                 825                 830
   Leu Ile Glu Phe Cys Tyr Lys Ser Arg Ala Glu Ala Lys Arg Met Lys
           835                 840                 845
   Leu Thr Phe Ser Glu Ala Ile Arg Asn Lys Ala Arg Leu Ser Ile Thr
       850                 855                 860
   Gly Ser Val Gly Glu Asn Gly Arg Val Leu Thr Pro Asp Cys Pro Lys
   865                 870                 875                 880
   Ala Val His Thr Gly Thr Ala Ile Arg Gln Ser Ser Gly Leu Ala Val
                   885                 890                 895
   Ile Ala Ser Asp Leu Pro
               900
<210> 9
   <211> 907
   <212> PRT
   <213> Mus musculus
<400> 9
   Met Pro Tyr Ile Phe Ala Phe Phe Cys Thr Gly Phe Leu Gly Ala Val
    1               5                  10                  15
   Val Gly Ala Asn Phe Pro Asn Asn Ile Gln Ile Gly Gly Leu Phe Pro
               20                  25                  30
   Asn Gln Gln Ser Gln Glu His Ala Ala Phe Arg Phe Ala Leu Ser Gln
           35                  40                  45
   Leu Thr Glu Pro Pro Lys Leu Leu Pro Gln Ile Asp Ile Val Asn Ile
       50                  55                  60
   Ser Asp Ser Phe Glu Met Thr Tyr Arg Phe Cys Ser Gln Phe Ser Lys
   65                  70                  75                  80
   Gly Val Tyr Ala Ile Phe Gly Phe Tyr Glu Arg Arg Thr Val Asn Met
                   85                  90                  95
   Leu Thr Ser Phe Cys Gly Ala Leu His Val Cys Phe Ile Thr Pro Ser
               100                 105                 110
   Phe Pro Val Asp Thr Ser Asn Gln Phe Val Leu Gln Leu Arg Pro Glu
           115                 120                 125
   Leu Gln Glu Ala Leu Ile Ser Ile Ile Asp His Tyr Lys Trp Gln Thr
       130                 135                 140
   Phe Val Tyr Ile Tyr Asp Ala Asp Arg Gly Leu Ser Val Leu Gln Arg
   145                 150                 155                 160
   Val Leu Asp Thr Ala Ala Glu Lys Asn Trp Gln Val Thr Ala Val Asn
                   165                 170                 175
   Ile Leu Thr Thr Thr Glu Glu Gly Tyr Arg Met Leu Phe Gln Asp Leu
               180                 185                 190
   Glu Lys Lys Lys Glu Arg Leu Val Val Val Asp Cys Glu Ser Glu Arg
           195                 200                 205
   Leu Asn Ala Ile Leu Gly Gln Ile Val Lys Leu Glu Lys Asn Gly Ile
       210                 215                 220
   Gly Tyr His Tyr Ile Leu Ala Asn Leu Gly Phe Met Asp Ile Asp Leu
   225                 230                 235                 240
   Asn Lys Phe Lys Glu Ser Gly Ala Asn Val Thr Gly Phe Gln Leu Val
                   245                 250                 255
   Asn Tyr Thr Asp Thr Ile Pro Ala Arg Ile Met Gln Gln Trp Arg Thr
               260                 265                 270
   Ser Asp Ala Arg Asp His Thr Arg Val Asp Trp Lys Arg Pro Lys Tyr
           275                 280                 285
   Thr Ser Ala Leu Thr Tyr Asp Gly Val Lys Val Met Ala Glu Ala Phe
       290                 295                 300
   Gln Ser Leu Arg Arg Gln Arg Ile Asp Ile Ser Arg Arg Gly Asn Ala
   305                 310                 315                 320
   Gly Asp Cys Leu Ala Asn Pro Ala Val Pro Trp Gly Gln Gly Ile Asp
                   325                 330                 335
   Ile Gln Arg Ala Leu Gln Gln Val Arg Phe Glu Gly Leu Thr Gly Asn
               340                 345                 350
   Val Gln Phe Asn Glu Lys Gly Arg Arg Thr Asn Tyr Thr Leu His Val
           355                 360                 365
   Ile Glu Met Lys His Asp Gly Ile Arg Lys Ile Gly Tyr Trp Asn Glu
       370                 375                 380
   Asp Asp Lys Phe Val Pro Ala Ala Thr Asp Ala Gln Ala Gly Gly Asp
   385                 390                 395                 400
   Asn Ser Ser Val Gln Asn Arg Thr Tyr Ile Val Thr Thr Ile Leu Glu
                   405                 410                 415
   Asp Pro Tyr Val Met Leu Lys Lys Asn Ala Asn Gln Phe Glu Gly Asn
               420                 425                 430
   Asp Arg Tyr Glu Gly Tyr Cys Val Glu Leu Ala Ala Glu Ile Ala Lys
           435                 440                 445
   His Val Gly Tyr Ser Tyr Arg Leu Glu Ile Val Ser Asp Gly Lys Tyr
       450                 455                 460
   Gly Ala Arg Asp Pro Asp Thr Lys Ala Trp Asn Gly Met Val Gly Glu
   465 470 475 480
   Leu Val Tyr Gly Arg Ala Asp Val Ala Val Ala Pro Leu Thr Ile Thr
   485 490 495
   Leu Val Arg Glu Glu Val Ile Asp Phe Ser Lys Pro Phe Met Ser Leu
   500 505 510
   Gly Ile Ser Ile Met Ile Lys Lys Pro Gln Lys Ser Lys Pro Gly Val
   515 520 525
   Phe Ser Phe Leu Asp Pro Leu Ala Tyr Glu Ile Trp Met Cys Ile Val
   530 535 540
   Phe Ala Tyr Ile Gly Val Ser Val Val Leu Phe Leu Val Ser Arg Phe
   545 550 555 560
   Ser Pro Tyr Glu Trp His Ser Glu Glu Phe Glu Glu Gly Arg Asp Gln
   565 570 575
   Thr Thr Ser Asp Gln Ser Asn Glu Phe Gly Ile Phe Asn Ser Leu Trp
   580 585 590
   Phe Ser Leu Gly Ala Phe Met Gln Gln Gly Cys Asp Ile Ser Pro Arg
   595 600 605
   Ser Leu Ser Gly Arg Ile Val Gly Gly Val Trp Trp Phe Phe Thr Leu
   610 615 620
   Ile Ile Ile Ser Ser Tyr Thr Ala Asn Leu Ala Ala Phe Leu Thr Val
   625 630 635 640
   Glu Arg Met Val Ser Pro Ile Glu Ser Ala Glu Asp Leu Ala Lys Gln
   645 650 655
   Thr Glu Ile Ala Tyr Gly Thr Leu Glu Ala Gly Ser Thr Lys Glu Phe
   660 665 670
   Phe Arg Arg Ser Lys Ile Ala Val Phe Glu Lys Met Trp Thr Tyr Met
   675 680 685
   Lys Ser Ala Glu Pro Ser Val Phe Val Arg Thr Thr Glu Glu Gly Met
   690 695 700
   Ile Arg Val Arg Lys Ser Lys Gly Lys Tyr Ala Tyr Leu Leu Glu Ser
   705 710 715 720
   Thr Met Asn Glu Tyr Ile Glu Gln Arg Lys Pro Cys Asp Thr Met Lys
   725 730 735
   Val Gly Gly Asn Leu Asp Ser Lys Gly Tyr Gly Ile Ala Thr Pro Lys
   740 745 750
   Gly Ser Ala Leu Arg Gly Pro Val Asn Leu Ala Val Leu Lys Leu Ser
   755 760 765
   Glu Gln Gly Val Leu Asp Lys Leu Lys Ser Lys Trp Trp Tyr Asp Lys
   770 775 780
   Gly Glu Cys Gly Ser Lys Asp Ser Gly Ser Lys Asp Lys Thr Ser Ala
   785                 790                 795                 800
   Leu Ser Leu Ser Asn Val Ala Gly Val Phe Tyr Ile Leu Ile Gly Gly
                   805                 810                 815
   Leu Gly Leu Ala Met Leu Val Ala Leu Ile Glu Phe Cys Tyr Lys Ser
               820                 825                 830
   Arg Ser Glu Ser Lys Arg Met Lys Gly Phe Cys Leu Ile Pro Gln Gln
           835                 840                 845
   Ser Ile Asn Glu Ala Ile Arg Thr Ser Thr Leu Pro Arg Asn Ser Gly
       850                 855                 860
   Ala Gly Ala Ser Gly Gly Ser Gly Ser Gly Glu Asn Gly Arg Val Val
   865                 870                 875                 880
   Ser Gln Asp Phe Pro Lys Ser Met Gln Ser Ile Pro Cys Met Ser His
                   885                 890                 895
   Ser Ser Gly Met Pro Leu Gly Ala Thr Gly Leu
               900                 905
<210> 10
   <211> 883
   <212> PRT
   <213> Mus musculus
<400> 10
   Met Gln Lys Ile Met His Ile Ser Val Leu Leu Ser Pro Val Leu Trp
   1 5 10 15
   Gly Leu Ile Phe Gly Val Ser Ser Asn Ser Ile Gln Ile Gly Gly Leu
   20 25 30
   Phe Pro Arg Gly Ala Asp Gln Glu Tyr Ser Ala Phe Arg Val Gly Met
   35 40 45
   Val Gln Phe Ser Thr Ser Glu Phe Arg Leu Thr Pro His Ile Asp Asn
   50 55 60
   Leu Glu Val Ala Asn Ser Phe Ala Val Thr Asn Ala Phe Cys Ser Gln
   65 70 75 80
   Phe Ser Arg Gly Val Tyr Ala Ile Phe Gly Phe Tyr Asp Lys Lys Ser
   85 90 95
   Val Asn Thr Ile Thr Ser Phe Cys Gly Thr Leu His Val Ser Phe Ile
   100 105 110
   Thr Pro Ser Phe Pro Thr Asp Gly Thr His Pro Phe Val Ile Gln Met
   115 120 125
   Arg Pro Asp Leu Lys Gly Ala Leu Leu Ser Leu Ile Glu Tyr Tyr Gln
   130 135 140
   Trp Asp Lys Phe Ala Tyr Leu Tyr Asp Ser Asp Arg Gly Leu Ser Thr
   145 150 155 160
   Leu Gln Ala Val Leu Asp Ser Ala Ala Glu Lys Lys Trp Gln Val Thr
                   165                 170                 175
   Ala Ile Asn Val Gly Asn Ile Asn Asn Asp Lys Lys Asp Glu Thr Tyr
               180                 185                 190
   Arg Ser Leu Phe Gln Asp Leu Glu Leu Lys Lys Glu Arg Arg Val Ile
           195                 200                 205
   Leu Asp Cys Glu Arg Asp Lys Val Asn Asp Ile Val Asp Gln Val Ile
       210                 215                 220
   Thr Ile Gly Lys His Val Lys Gly Tyr His Tyr Ile Ile Ala Asn Leu
   225                 230                 235                 240
   Gly Phe Thr Asp Gly Asp Leu Leu Lys Ile Gln Phe Gly Gly Ala Asn
                   245                 250                 255
   Val Ser Gly Phe Gln Ile Val Val Tyr Asp Asp Ser Leu Ala Ser Lys
               260                 265                 270
   Phe Ile Glu Arg Trp Ser Thr Leu Glu Gly Lys Glu Tyr Pro Gly Ala
           275                 280                 285
   His Thr Ala Thr Ile Lys Tyr Thr Ser Ala Leu Thr Tyr Asp Ala Val
       290                 295                 300
   Gln Val Met Thr Glu Ala Phe Arg Asn Leu Arg Lys Gln Arg Ile Glu
   305                 310                 315                 320
   Ile Ser Arg Arg Gly Asn Ala Gly Asp Cys Leu Ala Asn Pro Ala Val
                   325                 330                 335
   Pro Trp Gly Gln Gly Val Glu Ile Glu Arg Ala Leu Lys Gln Val Gln
               340                 345                 350
   Val Glu Gly Leu Ser Gly Asn Ile Lys Phe Asp Gln Asn Gly Lys Arg
           355                 360                 365
   Ile Asn Tyr Thr Ile Asn Ile Met Glu Leu Lys Thr Asn Gly Pro Arg
       370                 375                 380
   Lys Ile Gly Tyr Trp Ser Glu Val Asp Lys Met Val Val Thr Leu Thr
   385                 390                 395                 400
   Glu Leu Pro Ser Gly Asn Asp Thr Ser Gly Leu Glu Asn Lys Thr Val
                   405                 410                 415
   Val Val Thr Thr Ile Leu Glu Ser Pro Tyr Val Met Met Lys Lys Asn
               420                 425                 430
   His Glu Met Leu Glu Gly Asn Glu Arg Tyr Glu Gly Tyr Cys Val Asp
           435                 440                 445
   Leu Ala Ala Glu Ile Ala Lys His Cys Gly Phe Lys Tyr Lys Leu Thr
       450                 455                 460
   Ile Val Gly Asp Gly Lys Tyr Gly Ala Arg Asp Ala Asp Thr Lys Ile
   465                 470                 475                 480
   Trp Asn Gly Met Val Gly Glu Leu Val Tyr Gly Lys Ala Asp Ile Ala
                   485                 490                 495
   Ile Ala Pro Leu Thr Ile Thr Leu Val Arg Glu Glu Val Ile Asp Phe
               500                 505                 510
   Ser Lys Pro Phe Met Ser Leu Gly Ile Ser Ile Met Ile Lys Lys Pro
           515                 520                 525
   Gln Lys Ser Lys Pro Gly Val Phe Ser Phe Leu Asp Pro Leu Ala Tyr
       530                 535                 540
   Glu Ile Trp Met Cys Ile Val Phe Ala Tyr Ile Gly Val Ser Val Val
   545                 550                 555                 560
   Leu Phe Leu Val Ser Arg Phe Ser Pro Tyr Glu Trp His Thr Glu Glu
                   565                 570                 575
   Phe Glu Asp Gly Arg Glu Thr Gln Ser Ser Glu Ser Thr Asn Glu Phe
               580                 585                 590
   Gly Ile Phe Asn Ser Leu Trp Phe Ser Leu Gly Ala Phe Met Arg Gln
           595                 600                 605
   Gly Cys Asp Ile Ser Pro Arg Ser Leu Ser Gly Arg Ile Val Gly Gly
       610                 615                 620
   Val Trp Trp Phe Phe Thr Leu Ile Ile Ile Ser Ser Tyr Thr Ala Asn
   625                 630                 635                 640
   Leu Ala Ala Phe Leu Thr Val Glu Arg Met Val Ser Pro Ile Glu Ser
                   645                 650                 655
   Ala Glu Asp Leu Ser Lys Gln Thr Glu Ile Ala Tyr Gly Thr Leu Asp
               660                 665                 670
   Ser Gly Ser Thr Lys Glu Phe Phe Arg Arg Ser Lys Ile Ala Val Phe
           675                 680                 685
   Asp Lys Met Trp Thr Tyr Met Arg Ser Ala Glu Pro Ser Val Phe Val
       690                 695                 700
   Arg Thr Thr Ala Glu Gly Val Ala Arg Val Arg Lys Ser Lys Gly Lys
   705                 710                 715                 720
   Tyr Ala Tyr Leu Leu Glu Ser Thr Met Asn Glu Tyr Ile Glu Gln Arg
                   725                 730                 735
   Lys Pro Cys Asp Thr Met Lys Val Gly Gly Asn Leu Asp Ser Lys Gly
               740                 745                 750
   Tyr Gly Ile Ala Thr Pro Lys Gly Ser Ser Leu Gly Asn Ala Val Asn
           755                 760                 765
   Leu Ala Val Leu Lys Leu Asn Glu Gln Gly Leu Leu Asp Lys Leu Lys
       770                 775                 780
   Asn Lys Trp Trp Tyr Asp Lys Gly Glu Cys Gly Ser Gly Gly Gly Asp
   785                 790                 795                 800
   Ser Lys Glu Lys Thr Ser Ala Leu Ser Leu Ser Asn Val Ala Gly Val
                   805                 810                 815
   Phe Tyr Ile Leu Val Gly Gly Leu Gly Leu Ala Met Leu Val Ala Leu
               820                 825                 830
   Ile Glu Phe Cys Tyr Lys Ser Arg Ala Glu Ala Lys Arg Met Lys Val
           835                 840                 845
   Ala Lys Asn Ala Gln Asn Ile Asn Pro Ser Ser Ser Gln Asn Ser Gln
       850                 855                 860
   Asn Phe Ala Thr Tyr Lys Glu Gly Tyr Asn Val Tyr Gly Ile Glu Ser
   865                 870                 875                 880
   Val Lys Ile
<210> 11
   <211> 2724
   <212> DNA
   <213> Rattus norvegicus
<400> 11 wherein nnn codes for any aromatic amino acid
<210> 12
   <211> 2652
   <212> DNA
   <213> Rattus norvegicus
<400> 12 wherein nnn codes for any aromatic amino acid
<210> 13
   <211> 2667
   <212> DNA
   <213> Rattus norvegicus
<400> 13 wherein nnn codes for any aromatic amino acid
<210> 14
   <211> 2709
   <212> DNA
   <213> Rattus norvegicus
<400> 14 wherein nnn codes for any aromatic amino acid
<210> 15
   <211> 2721
   <212> DNA
   <213> Homo sapiens
<400> 15 wherein nnn codes for any aromatic amino acid
<210> 16
   <211> 2652
   <212> DNA
   <213> Homo sapiens
<400> 16 wherein nnn codes for any aromatic amino acid
<210> 17
   <211> 2685
   <212> DNA
   <213> Homo sapiens
<400> 17 wherein nnn codes for any aromatic amino acid
<210> 18
   <211> 2709
   <212> DNA
   <213> Homo sapiens
<400> 18 wherein nnn codes for any aromatic amino acid
<210> 19
   <211> 2724
   <212> DNA
   <213> Mus musculus
<400> 19 wherein nnn codes for any aromatic amino acid
<210> 20
   <211> 2652
   <212> DNA
   <213> Mus musculus
<400> 20 wherein nnn codes for any aromatic amino acid
<210> 21
   <211> 45
   <212> DNA
   <213> Rattus norvegicus
<400> 21
   gctcccttga ccataaccta tgttcgcgag gaagtcatcg acttc 45
<210> 22
   <211> 45
   <212> DNA
   <213> Rattus norvegicus
<400> 22
   gaagtcgatg acttcctcgc gaacataggt tatggtcaag ggagc 45

## Claims

1. A nucleic acid molecule encoding a (poly)peptide which has an amino acid sequence of a glutamate receptor of the AMPA-type and/or of a subunit of said receptor and functions as a non-desensitizing AMPA-receptor or as a non-desensitizing subunit thereof, wherein the leucine corresponding to position 497 of the wildtype rat AMPA-receptor GluR1_{flip} or the leucine at the position which corresponds in other glutamate receptors of the AMPA-type by comparison of homology to position 497 of the wildtype rat AMPA-receptor GluR1_{flip} is replaced by an aromatic amino acid;
wherein said nucleic acid molecule is
(a) a nucleic acid molecule encoding the (poly)peptide having the amino acid sequence of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO:8, SEQ ID NO: 9, or SEQ ID NO: 10, wherein the leucine residue corresponding to position 497 of SEQ ID NO: 1, 5 or 9, corresponding to position 504 of SEQ ID NO: 2, 6 or 10, corresponding to position 507 of SEQ ID NO: 3, to position 505 of SEQ ID NO: 4 or 8, or corresponding to position 513 of SEQ ID NO: 7 is replaced by an aromatic amino acid;
(b) a nucleic acid molecule having the DNA sequence of SEQ ID NO: 11, SEQ ID NO. 12, SEQ ID NO: 13, SEQ ID NO: 14, SEQ ID NO: 15, SEQ ID NO: 16, SEQ ID NO: 17, SEQ ID NO: 18, SEQ ID NO: 19 or SEQ ID NO: 20, wherein the codon represented by nnn corresponds to a codon coding for an aromatic amino acid;
(c) a nucleic acid molecule hybridizing to the complementary strand of a nucleic acid molecule of (a) or (b); or
(d) a nucleic acid molecule being degenerate as a result of the genetic code to the nucleotide sequence of a nucleic acid molecule as defined in (c).

2. The nucleic acid molecule of claim 1 wherein the (poly)peptide comprises an aromatic amino acid at position 497 of SEQ ID NO: 1, 5 or 9, at position 504 of SEQ ID NO: 2, 6 or 10, at position 507 of SEQ ID NO: 3, at position 505 of SEQ ID NO: 4 or 8 or at position 513 of SEQ ID NO: 7, but differs therefrom by at least one mutation selected from the group consisting of amino acid substitutions, addition(s) insertions, deletions, inversions and/or duplications.

3. The nucleic acid molecule of claims 1 or 3 derived from a rat, a mouse or a human.

4. The nucleic acid molecule of any one of claims 1 to 3, wherein said aromatic amino acid residue is tyrosine, phenylalanine, tryptophan or histidine.

5. The nucleic acid molecule of any one of claims 1 to 4 which is DNA, RNA or PNA.

6. The nucleic acid molecule of any one of claims 1 to 5 encoding a fusion protein.

7. A vector comprising the nucleic acid molecule of any one of claims 1 to 6.

8. A vector of claim 7 which is an expression vector, a gene targeting vector and/or a gene transfer vector.

9. A host transformed with a vector of claim 7 or 8 or comprising the nucleic acid molecule of claim 1 to 6, wherein the host is not a human organism.

10. The host of claim 9 which is a mammalian cell, an amphibian cell, an insect cell, a fungal cell, a plant cell or a bacterial cell.

11. The host of claim 10, wherein said mammalian cell is a HEK cell.

12. The host of claim 10, wherein said amphibian cell is an oocyte.

13. The host of claim 12, wherein said oocyte is a frog oocyte.

14. The host of claim 9 which is a non-human transgenic organism.

15. The host of claim 14, wherein said non-human organism is a mammal, amphibian, an insect, a fungus or a plant.

16. A method for producing the (poly)peptide encoded by a nucleic acid molecule of any one of claims 1 to 6 comprising culturing/raising the host of any one of claims 9 to 15 and isolating the produced (poly)peptide.

17. A (poly)peptide encoded by the nucleic acid molecule of any one of claims 1 to 6 or produced by the method of claim 16.

18. An antibody specifically directed to the (poly)peptide of claim 17, wherein said antibody specifically reacts with an epitope comprising the aromatic amino acid which replaces the leucine at position 497 of the wildtype rat AMPA-receptor GtuR1_{flip} or the leucine at the position which corresponds in other glutamate receptors of the AMPA-type by comparison of homology to position 497 of said wildtype rat AMPA receptor GluR1_{flip}.

19. The antibody of claim 18 which is a monoclonal antibody.

20. A composition comprising the nucleic acid molecule of any one of claims 1 to 6, the vector of claim 7 or 8, the (poly)peptide of claim 17 and/or the antibody of claim 18 or 19.

21. The composition of claim 20 which is a pharmaceutical composition, optionally further comprising a pharmaceutically acceptable carrier and/or diluent and/or excipient.

22. The composition of claim 20 which is a diagnostic composition, optionally further comprising suitable means for detection.

23. A method for the blocking of desensitization of a glutamate receptor of the AMPA-type, comprising the step of replacing a leucine corresponding to position 497 of the wildtype rat AMPA-receptor GluR1_{flip} or the leucine at the position which corresponds in other glutamate receptors of the AMPA-type by comparison of homology to position 497 of the wildtype rat AMPA-receptor GluR1_{flip} by an aromatic amino acid.

24. A method for identifying molecules which are capable of interacting with glutamate receptors of the AMPA-type, comprising the steps of
(a) contacting a non-desensitizing AMPA-receptor as encoded by a nucleic acid molecule of any one of claims 1 to 6, a vector of claims 7 or 8, a host of any one of claims 9 to 15, or an antibody of claim 18 or 19 with said molecule; and
(b) identifying among these molecules the molecules which are capable of interacting with said glutamate receptors of the AMPA-type.

25. A method for the characterization of molecules which are capable of interaction with glutamate receptors of the AMPA-type, comprising the steps of
(a) contacting a non-desensitizing AMPA-receptor as defined in any one of claims 1 to 6, a vector of claims 7 or 8, a host of any one of claims 9 to 15, or an antibody of claim 18 or 19 with said molecules; and
(b) measuring and/or detecting the characteristic effect said molecules evoke.

26. A method of screening for molecules which are capable of interacting with glutamate receptors of the AMPA-type, comprising the steps of
(a) contacting a non-desensitizing AMPA-receptor as encoded by a nucleic acid molecule of any one of claims 1 to 6, a vector of claim 7 or 8 or a host of any one of claims 9 to 15 with a candidate molecule; and
(b) measuring and/or detecting a response; and
(c) comparing said response to a standard response as measured in the absence of said candidate molecule.

27. Use of a non-desensitizing AMPA-receptor as encoded by the nucleic acid molecule of any one of claims 1 to 6 or use of a host as defined in any one of claims 9 to 15 as a biosensor for glutamate concentrations

28. Use of a non-desensitizing AMPA-receptor as encoded by the nucleic acid molecule of any one of claims 1 to 6 or use of a host as defined in any one of claims 9 to 15 for the characterization of glutamate receptor channel properties.

29. Use of a nucleic acid molecule of any one of claims 1 to 6, of a vector of claims 7 or 8, of a host of claims 9 or 10, of a (poly)peptide of claim 17, and/or of the antibody of claim 18 or 19 for the preparation of a pharmaceutical composition for preventing and/or treating neurological and/or neurodegenerative disorders.

30. The use of claim 29, wherein said neurological and/or neurodegenerative disorders are selected from the group consisting of Alzheimer's disease, Parkinson's disease, Huntington's disease, amyotrophic lateral sclerosis (FALS/SALS), ischemia, stroke, epilepsy, AIDS dementia and learning disorders.

31. Use of the nucleic acid molecule of any one of claims 1 to 6, the vector of claim 7 or 8, the host cell of claim 9 or 10 for the preparation of a pharmaceutical composition for gene therapy.

## Patentansprüche

1. Nucleinsäuremolekül, das ein (Poly)peptid codiert, welches eine Aminosäuresequenz eines Glutamatrezeptors des AMPA-Typs und/oder einer Untereinheit des Rezeptors aufweist und welches als ein nicht-desensibilisierender AMPA-Rezeptor oder als eine nicht-desensibilisierende Untereinheit davon fungiert, wobei das Leucin entsprechend der Position 497 des Wildtyp-AMPA-Rezeptors der Ratte GluR1_{flip} oder das Leucin an der Position, welche gemäß Homologievergleich in anderen Glutamatrezeptoren des AMPA-Typs Position 497 des Wildtyp-AMPA-Rezeptors der Ratte GluR1_{flip} entspricht, durch eine aromatische Aminosäure ersetzt ist, wobei das Nucleinsäuremolekül
(a) ein Nucleinsäuremolekül ist, das das (Poly)peptid mit der Aminosäuresequenz nach SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9 oder SEQ ID NO: 10 codiert, wobei der Leucinrest entsprechend der Position 497 von SEQ ID NO: 1, 5 oder 9, entsprechend der Position 504 von SEQ ID NO: 2, 6, oder 10, entsprechend der Position 507 von SEQ ID NO: 3, der Position 505 von SEQ ID NO: 4 oder 8 oder entsprechend der Position 513 von SEQ ID NO: 7 ersetzt ist durch eine aromatische Aminosäure;
(b) ein Nucleinsäuremolekül ist, mit der DNA-Sequenz nach SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19 oder SEQ ID NO: 20, wobei das Codon, das durch nnn dargestellt wird, einem Codon entspricht, das eine aromatische Aminosäure codiert;
(c) ein Nucleinsäuremolekül ist, das mit dem Komplementärstrang eines Nucleinsäuremoleküls nach (a) oder (b) hybridisiert; oder
(d) ein Nucleinsäuremolekül ist, das aufgrund des genetischen Codes degeneriert ist zu der Nucleotidsequenz eines Nucleinsäuremoleküls wie in (c) definiert.

2. Nucleinsäuremolekül nach Anspruch 1, wobei das (Poly)peptid eine aromatische Aminosäure an Position 497 von SEQ ID NO: 1, 5 oder 9, an Position 504 von SEQ ID NO: 2, 6 oder 10, an Position 507 von SEQ ID NO: 3, an Position 505 von SEQ ID NO: 4 oder 8 oder an Position 513 von SEQ ID NO: 7 umfasst, jedoch sich davon durch mindestens eine Mutation unterscheidet, ausgewählt aus der Gruppe bestehend aus Aminosäureaustauschen, -addition(en), -insertionen, -deletionen, -inversionen und/oder -duplikationen.

3. Nucleinsäuremolekül nach Anspruch 1 oder 3, das von einer Ratte, einer Maus oder einem Menschen stammt.

4. Aminosäuremolekül nach einem der Ansprüche 1 bis 3, wobei der aromatische Aminosäurerest Tyrosin, Phenylalanin, Tryptophan oder Histidin ist.

5. Nucleinsäuremolekül nach einem der Ansprüche 1 bis 4, welches DNA, RNA oder PNA ist.

6. Nucleinsäuremolekül nach einem der Ansprüche 1 bis 5, das ein Fusionsprotein codiert.

7. Vektor umfassend das Nucleinsäuremolekül nach einem der Ansprüche 1 bis 6.

8. Vektor nach Anspruch 7, welcher ein Expressionsvektor, ein Gen-Targeting-Vektor und/oder ein Gentransfervektor ist.

9. Wirt, transformiert mit einem Vektor nach Anspruch 7 oder 8 oder umfassend das Nucleinsäuremolekül nach Anspruch 1 bis 6, wobei der Wirt kein menschlicher Organismus ist.

10. Wirt nach Anspruch 9, welcher eine Säugerzelle, eine Amphibienzelle, eine Insektenzelle, eine Pilzzelle, eine Pflanzenzelle oder eine bakterielle Zelle ist.

11. Wirt nach Anspruch 10, wobei die Säugerzelle eine HEK-Zelle ist.

12. Wirt nach Anspruch 10, wobei die Amphibienzelle ein Oocyt ist.

13. Wirt nach Anspruch 12, wobei der Oocyt ein Frosch-Oocyt ist.

14. Wirt nach Anspruch 9, welcher ein nicht-menschlicher transgener Organismus ist.

15. Wirt nach Anspruch 14, wobei der nicht-menschliche Organismus ein Säuger, eine Amphibie, ein Insekt, ein Pilz oder eine Pflanze ist.

16. Verfahren zur Herstellung des (Poly)peptids, das durch ein Nucleinsäuremolekül nach einem der Ansprüche 1 bis 6 codiert wird, umfassend das Züchten/Aufziehen des Wirts nach einem der Ansprüche 9 bis 15 und das Isolieren des hergestellten (Poly)peptids.

17. (Poly)peptid, codiert durch das Nucleinsäuremolekül nach einem der Ansprüche 1 bis 6 oder hergestellt durch das Verfahren nach Anspruch 16.

18. Antikörper, der spezifisch gegen das (Poly)peptid nach Anspruch 17 gerichtet ist, wobei der Antikörper spezifisch mit einem Epitop reagiert, das die aromatische Aminosäure umfasst, welche das Leucin an Position 497 des Wildtyp-AMPA-Rezeptors der Ratte GluR1_{flip} oder das Leucin an der Position ersetzt, welche gemäß Homologievergleich Position 497 des Wildtyp-AMPA-Rezeptors der Ratte GluR1_{flip} in anderen Glutamatrezeptoren des AMPA-Typs entspricht.

19. Antikörper nach Anspruch 18, welcher ein monoclonaler Antikörper ist.

20. Zusammensetzung, umfassend das Nucleinsäuremolekül nach einem der Ansprüche 1 bis 6, den Vektor nach Anspruch 7 oder 8, das (Poly)peptid nach Anspruch 17 und/oder den Antikörper nach Anspruch 18 oder 19.

21. Zusammensetzung nach Anspruch 20, welche ein Arzneimittel ist, das gegebenenfalls ferner einen pharmazeutisch verträglichen Träger und/oder Verdünnungsmittel und/oder Exzipient umfasst.

22. Zusammensetzung nach Anspruch 20, welche eine diagnostische Zusammensetzung ist, die gegebenenfalls ferner geeignete Mittel zum Nachweis umfasst.

23. Verfahren zur Blockierung oder Desensibilisierung eines Glutamatrezeptors des AMPA-Typs, umfassend den Schritt des Ersetzens eines Leucins entsprechend der Position 497 des Wildtyp-AMPA-Rezeptors der Ratte GluR1_{flip} oder des Leucins an der Position, welche gemäß Homologievergleich Position 497 des Wildtyp-AMPA-Rezeptors der Ratte GluR1_{flip} in anderen Glutamatrezeptoren des AMPA-Typs entspricht, durch eine aromatische Aminosäure.

24. Verfahren zur Identifizierung von Molekülen, welche in der Lage sind, mit Glutamatrezeptoren des AMPA-Typs zu interagieren, umfassend die Schritte des
(a) Kontaktierens eines nicht-desensibilisierenden AMPA-Rezeptors, der von einem Nucleinsäuremolekül nach einem der Ansprüche 1 bis 6 codiert wird, eines Vektors nach Anspruch 7 oder 8, eines Wirts nach einem der Ansprüche 9 bis 15 oder eines Antikörpers nach Anspruch 18 oder 19 mit dem Molekül; und
(b) Identifizierens aus diesen Molekülen der Moleküle, welche in der Lage sind, mit den Glutamatrezeptoren des AMPA-Typs zu interagieren.

25. Verfahren zur Charakterisierung von Molekülen, welche in der Lage sind, mit Glutamatrezeptoren des AMPA-Typs zu interagieren, umfassend die Schritte des
(a) Kontaktierens eines nicht-desensibilisierenden AMPA-Rezeptors wie definiert in einem der Ansprüche 1 bis 6, eines Vektors nach Anspruch 7 oder 8, eines Wirts nach einem der Ansprüche 9 bis 15 oder eines Antikörpers nach Anspruch 18 oder 19 mit den Molekülen; und
(b) Messens und/oder Nachweisens des charakteristischen Effekts, der von den Molekülen hervorgerufen wird.

26. Verfahren zum Screenen von Molekülen, welche in der Lage sind, mit Glutamatrezeptoren des AMPA-Typs zu interagieren, umfassend die Schritte des
(a) Kontaktierens eines nicht-desensibilisierenden AMPA-Rezeptors, der durch ein Nucleinsäuremolekül nach einem der Ansprüche 1 bis 6 codiert wird, eines Vektors nach Anspruch 7 oder 8 oder eines Wirts nach einem der Ansprüche 9 bis 15 mit einem Kandidatenmolekül; und
(b) Messens und/oder Nachweisens einer Antwort; und
(c) Vergleichens der Antwort mit einer Standardantwort, die in der Abwesenheit des Kandidatenmoleküls gemessen wird .

27. Verwendung eines nicht-desensibilisierenden AMPA-Rezeptors, der durch das Nucleinsäuremolekül nach einem der Ansprüche 1 bis 6 codiert wird, oder Verwendung eines Wirts wie in einem der Ansprüche 9 bis 15 definiert als einen Biosensor für Glutamatkonzentrationen.

28. Verwendung eines nicht-desensibilisierenden AMPA-Rezeptors, der durch das Nucleinsäuremolekül nach einem der Ansprüche 1 bis 6 codiert wird, oder die Verwendung eines Wirts wie in einem der Ansprüche 9 bis 15 definiert für die Charakterisierung von Glutamatrezeptorkanaleigenschaften.

29. Verwendung eines Nucleinsäuremoleküls nach einem der Ansprüche 1 bis 6, eines Vektors nach Anspruch 7 oder 8, eines Wirts nach Ansprüchen 9 oder 10, eines (Poly)peptids nach Anspruch 17 und/oder des Antikörpers nach Anspruch 18 oder 19 für die Herstellung eines Arzneimittels zur Prävention und/oder Behandlung von neurologischen und/oder neurodegenerativen Erkrankungen.

30. Verwendung nach Anspruch 29, wobei die neurologischen und/oder neurodegenerativen Erkrankungen ausgewählt sind aus der Gruppe bestehend aus Alzheimersche Krankheit, Parkinson-Krankheit, Huntington-Krankheit, amyotrophische Lateralsklerose (FALS/SALS), Ischämie, Schlaganfall, Epilepsie, AIDS-Demenz und Lernstörungen.

31. Verwendung des Nucleinsäuremoleküls nach einem der Ansprüche 1 bis 6, des Vektors nach Anspruch 7 oder 8, des Wirts nach Anspruch 9 oder 10 zur Herstellung eines Arzneimittels für Gentherapie.

## Revendications

1. Molécule d'acide nucléique codant pour un (poly)peptide qui a une séquence d'acides aminés d'un récepteur au glutamate du type AMPA et/ou d'une sous-unité dudit récepteur et fonctionne comme un récepteur AMPA non désensi-bilisant ou comme une sous-unité non désensibilisante de celui-ci, dans laquelle la leucine correspondant à la position 497 du récepteur AMPA de rat de type sauvage GluR1_{flip} ou la leucine à la position qui correspond dans d'autres récepteurs au glutamate du type AMPA par comparaison d'homologie à la position 497 du récepteur AMPA de rat de type sauvage GluR1_{flip} est remplacée par un acide aminé aromatique ;
dans laquelle ladite molécule d'acide nucléique est
(a) une molécule d'acide nucléique codant pour le (poly)peptide ayant la séquence d'acides aminés de SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, ou SEQ ID NO : 10, dans laquelle le résidu leucine correspondant à la position 497 de SEQ ID NO : 1, 5 ou 9, correspondant à la position 504 de SEQ ID NO : 2, 6 ou 10, correspondant à la position 507 de SEQ ID NO : 3, à la position 505 de SEQ ID NO : 4 ou 8, ou correspondant à la position 513 de SEQ ID NO : 7, est remplacé par un acide aminé aromatique ;
(b) une molécule d'acide nucléique ayant la séquence d'ADN de SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19 ou SEQ ID NO : 20, dans laquelle le codon représenté par nnn correspond à un codon codant pour un acide aminé aromatique ;
(c) une molécule d'acide nucléique s'hybridant au brin complémentaire d'une molécule d'acide nucléique de (a) ou (b) ; ou
(d) une molécule d'acide nucléique étant dégénérée en conséquence du code génétique conduisant à la séquence nucléotidique d'une molécule d'acide nucléique telle que définie en (c).

2. Molécule d'acide nucléique selon la revendication 1, dans laquelle le (poly)peptide comprend un acide aminé aromatique en position 497 de SEQ ID NO : 1, 5 ou 9, en position 504 de SEQ ID NO : 2, 6 ou 10, en position 507 de SEQ ID NO : 3, en position 505 de SEQ ID NO : 4 ou 8 ou en position 513 de SEQ ID NO : 7, mais diffère de celui-ci d'au moins une mutation choisie parmi le groupe constitué de substitutions, addition(s), insertions, délétions, inversions et/ou duplications d'acide aminé.

3. Molécule d'acide nucléique selon la revendication 1 ou 3, dérivée d'un rat, d'une souris ou d'un humain.

4. Molécule d'acide nucléique selon l'une quelconque des revendications 1 à 3, dans laquelle ledit résidu d'acide aminé aromatique est une tyrosine, une phénylalanine, un tryptophane ou une histidine.

5. Molécule d'acide nucléique selon l'une quelconque des revendications 1 à 4, qui est un ADN, un ARN ou un PNA ("Peptide Nucleic Acid").

6. Molécule d'acide nucléique selon l'une quelconque des revendications 1 à 5, codant pour une protéine de fusion.

7. Vecteur comprenant la molécule d'acide nucléique selon l'une quelconque des revendications 1 à 6.

8. Vecteur selon la revendication 7, qui est un vecteur d'expression, un vecteur de ciblage de gène et/ou un vecteur de transfert de gène.

9. Hôte transformé avec un vecteur selon la revendication 7 ou 8 ou comprenant la molécule d'acide nucléique selon la revendication 1 à 6, dans lequel l'hôte n'est pas un organisme humain.

10. Hôte selon la revendication 9, qui est une cellule de mammifère, une cellule d'amphibien, une cellule d'insecte, une cellule de champignon, une cellule de plante ou une cellule de bactérie.

11. Hôte selon la revendication 10, dans lequel ladite cellule de mammifère est une cellule HEK.

12. Hôte selon la revendication 10, dans lequel ladite cellule d'amphibien est un oocyte.

13. Hôte selon la revendication 12, dans lequel ledit oocyte est un oocyte de grenouille.

14. Hôte selon la revendication 9, qui est un organisme transgénique non humain.

15. Hôte selon la revendication 14, dans lequel ledit organisme non humain est un mammifère, un amphibien, un insecte, un champignon ou une plante.

16. Procédé pour la production du (poly)peptide codé par une molécule d'acide nucléique selon l'une quelconque des revendications 1 à 6, comprenant la culture/l'élevage de l'hôte selon l'une quelconque des revendications 9 à 15 et l'isolement du (poly)peptide produit.

17. (Poly)peptide codé par la molécule d'acide nucléique selon l'une quelconque des revendications 1 à 6 ou produit par le procédé selon la revendication 16.

18. Anticorps dirigé spécifiquement contre le (poly)peptide selon la revendication 17, dans lequel ledit anticorps réagit spécifiquement avec un épitope comprenant l'acide aminé aromatique qui remplace la leucine en position 497 du récepteur AMPA de rat de type sauvage GluR1_{flip} ou la leucine à la position qui correspond dans d'autres récepteurs au glutamate du type AMPA par comparaison d'homologie à la position 497 dudit récepteur AMPA de rat de type sauvage GluR1_{flip}.

19. Anticorps selon la revendication 18, qui est un anticorps monoclonal.

20. Composition comprenant la molécule d'acide nucléique selon l'une quelconque des revendications 1 à 6, le vecteur selon la revendication 7 ou 8, le (poly)peptide selon la revendication 17 et/ou l'anticorps selon la revendication 18 ou 19.

21. Composition selon la revendication 20, qui est une composition pharmaceutique, comprenant éventuellement en outre un véhicule et/ou un diluant et/ou un excipient pharmaceutiquement acceptables.

22. Composition selon la revendication 20, qui est une composition diagnostique, comprenant éventuellement en outre des moyens de détection appropriés.

23. Procédé destiné à bloquer la désensibilisation d'un récepteur au glutamate du type AMPA, comprenant l'étape de remplacement d'une leucine correspondant à la position 497 du récepteur AMPA de rat de type sauvage GluR1_{flip} ou de la leucine à la position qui correspond dans d'autres récepteurs au glutamate du type AMPA par comparaison d'homologie à la position 497 du récepteur AMPA de rat de type sauvage GluR1_{flip} par un acide aminé aromatique.

24. Procédé d'identification de molécules qui sont capables d'interagir avec des récepteurs au glutamate de type AMPA, comprenant les étapes de
(a) mise en contact d'un récepteur AMPA non désensibilisant tel que codé par une molécule d'acide nucléique selon l'une quelconque des revendications 1 à 6, d'un vecteur selon la revendication 7 ou 8, d'un hôte selon l'une quelconque des revendications 9 à 15, ou d'un anticorps selon la revendication 18 ou 19 avec ladite molécule ; et
(b) identification parmi ces molécules des molécules qui sont capables d'interagir avec lesdits récepteurs au glutamate du type AMPA.

25. Procédé de caractérisation de molécules qui sont capables d'interagir avec des récepteurs au glutamate du type AMPA, comprenant les étapes de
(a) mise en contact d'un récepteur AMPA non désensibilisant tel que défini dans l'une quelconque des revendications 1 à 6, d'un vecteur selon la revendication 7 ou 8, d'un hôte selon l'une quelconque des revendications 9 à 15, ou d'un anticorps selon la revendication 18 ou 19 avec lesdites molécules ; et
(b) mesure et/ou détection de l'effet caractéristique que lesdites molécules provoquent.

26. Procédé de criblage de molécules qui sont capables d'interagir avec des récepteurs au glutamate du type AMPA, comprenant les étapes de
(a) mise en contact d'un récepteur AMPA non désensibilisant tel que codé par une molécule d'acide nucléique selon l'une quelconque des revendications 1 à 6, d'un vecteur selon la revendication 7 ou 8 ou d'un hôte selon l'une quelconque des revendications 9 à 15 avec une molécule candidate ; et
(b) mesure et/ou détection d'une réponse ; et
(c) comparaison de ladite réponse à une réponse de référence telle que mesurée en l'absence de ladite molécule candidate.

27. Utilisation d'un récepteur AMPA non désensibilisant tel que codé par la molécule d'acide nucléique selon l'une quelconque des revendications 1 à 6 ou utilisation d'un hôte tel que défini dans l'une quelconque des revendications 9 à 15, en tant que biodétecteur de concentrations en glutamate.

28. Utilisation d'un récepteur AMPA non désensibilisant tel que codé par la molécule d'acide nucléique selon l'une quelconque des revendications 1 à 6 ou utilisation d'un hôte tel que défini dans l'une quelconque des revendications 9 à 15, pour la caractérisation de propriétés du canal du récepteur au glutamate.

29. Utilisation d'une molécule d'acide nucléique selon l'une quelconque des revendications 1 à 6, d'un vecteur selon la revendication 7 ou 8, d'un hôte selon la revendication 9 ou 10, d'un (poly)peptide selon la revendication 17, et/ou de l'anticorps selon la revendication 18 ou 19, pour la préparation d'une composition pharmaceutique destinée à prévenir et/ou à traiter des troubles neurologiques et/ou neurodégénératifs.

30. Utilisation selon la revendication 29, dans laquelle lesdits troubles neurologiques et/ou neurodégénératifs sont choisis parmi le groupe constitué de la maladie d'Alzheimer, la maladie de Parkinson, la maladie d'Huntington, la sclérose latérale amyotrophique (FALS/SALS), l'ischémie, l'attaque, l'épilepsie, la démence liée au SIDA (AIDS) et les troubles de l'apprentissage.

31. Utilisation de la molécule d'acide nucléique selon l'une quelconque des revendications 1 à 6, du vecteur selon la revendication 7 ou 8, de la cellule hôte selon la revendication 9 ou 10, pour la préparation d'une composition pharmaceutique destinée à la thérapie génique.
